# EUROPEAN PATENT APPLICATION

(11) **EP 2 998 322 A1**
(43) Date of publication of application: **23.03.2016**
(21) Application number: 14797306.9
(22) Date of filing: 14.05.2014
(51) Int. Cl.: C07K 19/00, C12N 15/62, C12N 15/63, C12N 1/00, A61K 39/385, A61P 37/02

(54) **EPITOPE VACCINE FOR LOW IMMUNOGENIC PROTEIN AND PREPARING METHOD AND USAGE THEREOF**

(30) Priority: 14.05.2013 CN 201310178135
(71) Applicant: Shanghai Hycharm Inc, Shanghai 200131 (CN)
(72) Inventor: LI, Rongxiu, Shanghai 200131 (CN); ZHANG, Li, Shanghai 200131 (CN); ZHONG, Conghao, Shanghai 200131 (CN); CHENG, Chao, Shanghai 200131 (CN); ZHANG, Li, Shanghai 200131 (CN); XU, Aizhang, Shanghai 200131 (CN); LU, Wuguang, Shanghai 200131 (CN); LIN, Zhibing, Shangai 200131 (CN)
(74) Representative: Predazzi, Valentina
(86) International application number: PCT/CN2014/077522
(87) International publication number: WO 2014/183649

(57) **Abstract**

The present invention provides recombinant protein carrying an epitope. The recombinant protein has a skeleton structure from carrier protein, and a low immunogenic protein epitope is imported through splicing, replacement, and/or insertion to at least one molecular surface amino acid residue area of the carrier protein. The carrier protein has at least one T cell epitope, and the recombinant protein can effectively excite immunoreaction of an organism to the low immunogenic protein epitope.

## Description

### Technical field

The present invention relates to field of biology and medical, and particularly relates to a vaccine for epitope antigens of low immunogenic protein and preparing method and usage thereof. The present invention can effectively stimulate an organism to produce an immune response against a specific epitope of low immunogenic protein.

### Background Art

Chronic diseases include cardiovascular diseases and cancer. Along with sustained economic development and improvement of living standard of nutrition, morbidity and mortality have increased rapidly and these diseases have become main causes for human illness and death. The advantages of using monoclonal antibody therapy include a known target and an affirmative effect, but it requires a large dose and treatment is repeated and expensive.

The prophylactic vaccine as an important means for controlling infectious diseases and complications thereof has saved hundreds of million of human lives and made a significant contribution to human health. The antigen of vaccine traditionally derived from pathogens is a naturally exogenous substance relative to human body and can stimulate body's immune protective response. The etiology and therapeutic intervention targets of chronic diseases are substances of a body itself. It is a very attractive therapeutic approach if it is possible to design a vaccine to stimulate an immune response of the body through active immunization to produce an autoantibody which has a similar therapeutic effect of a monoclonal antibody drug, because it has a long duration, a low-cost treatment, convenient and economical medication, and potential to prevent diseases.

However, under normal healthy state, the body does not produce an immune response against its own materials due to presence of protective mechanism of immune tolerance to the body's own substances. Up to present, Le Buanec et al. and French NeoVacs company (http://neovacs.fr/) have conjugated a keyhole limpet hemocyanin (KLH) with dozens of human protein (including various cytokines and growth factors) using glutaraldehyde and inactived bioactivity of human protein molecules by long time treatment of formaldehyde, thereby forming a kinoid antigen vaccine. The TNFK antigen prepared by conjugating KLH with TNF-α had a therapeutic effect in a mouse inflammation model, and clinical trials showed that it successfully induced an immune response in human body [Le Buanec H, et al. TNF alpha kinoid vaccination-induced neutralizing antibodies to TNF alpha protect mice from autologous TNF alpha-driven chronic and acute inflammation. Proc Natl Acad Sci USA 2006; 103 (51):19442-7]. Although effectiveness of this vaccine was partly verified, because dozens of human protein molecules were conjugated to KLH molecule and long time treatment of formaldehyde formed a complicated coagulation substance, it was difficult to meet clinical requirements on clear components, clear structure, and controllable quality and it might cause unknown risks.

Therefore, there is an urgent need in the art to develop a vaccine antigen technology which can effectively stimulates an organism to produce an immune response against a low immunogenic protein, and can meet clinical requirements on clear components, clear structure, and controllable quality.

### Summary of the invention

The object of the present invention is to provide an effective method to stimulate an organism to produce an immune response to a low immunogenic protein and to provide a related recombinant protein and a composition such as a vaccine composition.

In the first aspect of the invention, it provides a recombinant protein with an antigenic epitope, wherein the recombinant protein has a skeleton structure of a carrier protein which has at least one T cell epitope, and the antigenic epitope is imported into at least one molecular surface amino acid residue area of the carrier protein by splicing, replacement, and/or insertion.

In one preferred embodiment, the "molecular surface amino acid residue area" comprises a loop region, a beta-turn region, N-terminal or C-terminal.

In one preferred embodiment, the recombinant protein can induce an immune response to the antigenic epitope.

In one preferred embodiment, the antigenic epitope is a B-cell epitope or T cell epitope.

In one preferred embodiment, the carrier protein is a pathogen protein, including a viral protein, a bacterial protein, a chlamydia protein, a mycoplasma protein, or combination thereof.

In one preferred embodiment, the antigenic epitope is not from the carrier protein.

In one preferred embodiment, the antigenic epitope is derived from a mammalian protein.

In one preferred embodiment, the antigenic epitope is selected from the group consisting of:
(a) an antigenic epitope with single epitope; preferably, the length of the antigenic epitope (peptide) with single epitope is 5-30 amino acids, and preferably 6-15 amino acids; or
(b) an antigenic epitope with multi-epitopes; preferably, the length of the antigenic epitope (peptide) with multi-epitopes is 15-500 amino acids, preferably 20-300 amino acids, and more preferably 30-200 amino acids.

In one preferred embodiment, the antigenic epitope (peptide) with multi-epitopes was introduced to the C or N-terminal of the epitope carrier protein.

In one preferred embodiment, when the recombinant protein is administered to a human, an immune response to the antigenic epitope is induced in the human.

In one preferred embodiment, the replacement comprises partial or entire replacement of amino acid sequence of the molecular surface amino acid residue area of the carrier protein.

In one preferred embodiment, the partial replacement comprises a replacement of 1-15 amino acid residues and preferably 2-10 amino acid residues of the surface amino acid residue area of carrier protein.

In one preferred embodiment, the antigenic epitope is an epitope from a low immunogenic protein. Preferably, the low immunogenic protein comprises human and non-human mammalian protein.

In one preferred embodiment, the low immunogenic protein comprises an autoimmune disease-related protein, a tumor-associated protein, or a cardiovascular disease-related protein.

In one preferred embodiment, the low immunogenic protein comprises:
(a) VEGF, TNF-α, Her2 protein, clotting factor, interleukin, fibroblast activation protein (FAP), EGFR, PDL1 or combinations thereof;
(b) a protein formed by replacing, deleting, or adding one or more amino acid residues of the protein of (a) and capable of eliciting an immune response against the low immunogenic protein after it is recombinant with the carrier protein.

In one preferred embodiment, the low immunogenic protein is TNF-α or a protein formed by replacing, deleting, or adding one or more amino acid residues of TNF-α, and capable of eliciting an immune response against TNF-α after it is recombinant with the carrier protein.

In one preferred embodiment, the antigenic epitope is derived form TNF-α, and has two point mutations A145R and Y87H in TNF-α.

In one preferred embodiment, the antigenic epitope is derived form TNF-α, and is selected from the group consisting of:
(1) an amino acid sequence as shown in SEQ ID NO.: 10 or SEQ ID NO.: 12;
(2) a derivative polypeptide, which is derived from the amino acid sequence as shown in SEQ ID NO.: 10 or SEQ ID NO.: 12 by replacement, deletion, or addition of one or more amino acid residues, and capable of eliciting an immune response after it is recombinant with the carrier protein.

In one preferred embodiment, the antigenic epitope is one or more members selected from the group consisting of:
(1) VSRFAISYQEKVNLLSA;
(2) LDFAESGQV;
(3) WLNRRANA;
(4) GMDLKDNQLVV;
(5) VSRFAISYQEKVNLLSAV;
(6) ISRIAVSYQTKVNLLSA;
(7) ISRIAVSYQTKVNLLSAI; and
(8) a peptide fragment in other TNF-α protein and corresponding to any of the epitope sequences of (1) to (7).

In one preferred embodiment, the low immunogenic protein is a clotting factor.

In one preferred embodiment, the clotting factor is FXI.

In one preferred embodiment, the antigenic epitope is one or more members selected from the group consisting of:
(1) FYGVESPK;
(2) QYKMAESGYDI;
(3) WGYRKLRDKIQ;
(4) TNEECQKRYRGHKITH;
(5) ACIRDIF;
(6) TTKIKPRIVGGTASVRGE; and
(7) a peptide fragment in other FXI protein and corresponding to any of the epitope sequences of (1) to (6).

In one preferred embodiment, the antigenic epitope comprises a catalytic structural domain of clotting factor FXI.

In one preferred embodiment, the catalytic structural domain of FXI is selected from the group consisting of:
(a) a polypeptide having an amino acid sequence as shown in SEQ ID NO.: 45;
(b) a derivative polypeptide, which is derived from (a) by replacement, deletion, or addition of one or more amino acid residues in the amino acid sequence as shown in SEQ ID NO.: 45, and is capable of eliciting an immune response after it is recombinant with the carrier protein.

In one preferred embodiment, the clotting factor is FXII.

In one preferred embodiment, the antigenic epitope is one or more members selected from the group consisting of:
(1) EAFSPVSYQHDLA;
(2) EGFSSITYQHDLA; and
(3) a peptide fragment in other FXII protein and corresponding to any of the epitope sequences of (1) and (2).

In one preferred embodiment, the antigenic epitope comprises a catalytic structural domain of clotting factor FXII.

In one preferred embodiment, the catalytic structural domain of FXII is selected from the group consisting of:
(a) a polypeptide having an amino acid sequence as shown in SEQ ID NO.: 129 or SEQ ID NO.:130;
(b) a derivative polypeptide, which is derived from (a) by replacement, deletion, or addition of one or more amino acid residues in the amino acid sequence as shown in SEQ ID NO.: 129 or SEQ ID NO.:130, and is capable of eliciting an immune response after it is recombinant with the carrier protein.

In one preferred embodiment, the antigenic epitope peptide is derived from FAP, and the antigenic epitope peptide comprises YGDEQYPR.

In one preferred embodiment, the low immunogenic protein is FAP.

In one preferred embodiment, the antigenic epitope derived from FAP, and the antigenic epitope comprises YGDEQYPR.

In one preferred embodiment, the antigenic epitope is derived from a catalytic structural domain of FAP.

In one preferred embodiment, the catalytic structural domain of FAP is selected from the group consisting of:
(a) a polypeptide having an amino acid sequence as shown in SEQ ID NO.: 108;
(b) a derivative polypeptide, which is derived from (a) by replacement, deletion, or addition of one or more amino acid residues in the amino acid sequence as shown in SEQ ID NO.: 108 and is capable of eliciting an immune response after it is recombinant with the carrier protein.

In one preferred embodiment, the amino acid sequence of the recombinant protein is selected from the group consisting of:
(a) a polypeptide having an amino acid sequence as shown in SEQ ID NO.: 50 or SEQ ID NO.: 51;
(b) a derivative polypeptide, which is derived from (a) by replacement, deletion, or addition of one or more amino acid residues and is capable of eliciting an immune response after it is recombinant with the carrier protein.

In one preferred embodiment, the antigenic epitope is derived from VEGF.

In one preferred embodiment, the antigenic epitope is selected from the group consisting of:
(a) a polypeptide having an amino acid sequence as shown in SEQ ID NO.: 63;
(b) a derivative polypeptide, which is derived from (a) by replacement, deletion, or addition of one or more amino acid residues in the amino acid sequence as shown in SEQ ID NO.: 63 and is capable of eliciting an immune response after it is recombinant with the carrier protein.

In one preferred embodiment, the amino acid sequence of the recombinant protein is selected from the group consisting of:
(a) a polypeptide having an amino acid sequence of SEQ ID NO.: 62;
(b) a derivative polypeptide, which is derived from (a) by replacement, deletion, or addition of one or more amino acid residues in the amino acid sequence as shown in SEQ ID NO.: 62 and is capable of eliciting an immune response after it is recombinant with the carrier protein.

In one preferred embodiment, the antigenic epitope is derived from PDL1.

In one preferred embodiment, the antigenic epitope is derived from PDL1E.

In one preferred embodiment, the antigenic epitope is selected from the group consisting of:
(a) a polypeptide having an amino acid sequence as shown in SEQ ID NO.:67, SEQ ID NO.:68, SEQ ID NO.:122, SEQ ID NO.:123, SEQ ID NO.:124 or SEQ ID NO.:125;
(b) a derivative polypeptide, which is derived from (a) by replacement, deletion, or addition of one or more amino acid residues and is capable of eliciting an immune response after it is recombinant with the carrier protein.

In one preferred embodiment, the recombinant protein is selected from the group consisting of:
(a) a polypeptide having an amino acid sequence as shown in SEQ ID NO.:69, SEQ ID NO.:70, SEQ ID NO.:126 or SEQ ID NO.:127;
(b) a derivative polypeptide, which is derived from (a) by replacement, deletion, or addition of one or more amino acid residues and is capable of eliciting an immune response after it is recombinant with the carrier protein.

In one preferred embodiment, the antigenic epitope is derived from EGFR.

In one preferred embodiment, the antigenic epitope is derived from sub-region III of EGFR.

In one preferred embodiment, the antigenic epitope is derived from EGFR and is selected from the group consisting of:
(a) a polypeptide having an amino acid sequence as shown in SEQ ID NO.:83, SEQ ID NO.:109, SEQ ID NO.:110, or SEQ ID NO.:111;
(b) a derivative polypeptide, which is derived from (a) by replacement, deletion, or addition of one or more amino acid residues and capable of eliciting an immune response after it is recombinant with the carrier protein.

In one preferred embodiment, the antigenic epitope of the recombinant protein is derived from epidermal growth factor receptor (EGFR) and comprises at least one amino acid sequence as shown in SEQ ID NO.:83 or SEQ ID NO.:109, and at least one amino acid sequence as shown in SEQ ID NO.:110 or SEQ ID NO.:111.

In one preferred embodiment, the recombinant protein is selected from the group consisting of:
(a) a polypeptide having an amino acid sequence as shown in SEQ ID NO.:84, SEQ ID NO.:85, SEQ ID NO.:86, SEQ ID NO.:87, SEQ ID NO.:119 or SEQ ID NO.:120;
(b) a derivative polypeptide, which is derived from (a) by replacement, deletion, or addition of one or more amino acid residues and is capable of eliciting an immune response after it is recombinant with the carrier protein.

In one preferred embodiment, the length of the antigenic epitope is 5-40 amino acids, and preferably 8-30 amino acids.

In one preferred embodiment, the carrier protein comprises Diphtheria toxin or DT, a toxin transmembrane domain of Diphtheria or DTT, Rotavirus VP7, a heat shock protein of *Leishmania,* a flagellin of *Campylobacter jejuni,* a major outer membrane protein of *Chlamydia trachomatis,* a hemocyanin (Keyhole Limpet Hemocyanin, KLH), a Bovine serum albumin (BSA), a chicken ovalbumin (Ovalbumin, OVA), a fibrinogen, or a poly-lysine (PLL).

In one preferred embodiment, the surface amino acid residue area is within amino acid position 287 to 299 of DTT or the surface amino acid residue area is C-terminal or N-terminal of DTT. Preferably, the surface amino acid residue area is selected from the group consisting of: amino acid position 290-297 of DTT, amino acid position 291-297 of DTT, amino acid position 292-297 of DTT, amino acid position 293-297 of DTT, amino acid position 294-297 of DTT, and amino acid position 295-297 of DTT and amino acid position 296-297 of DTT.

In one preferred embodiment, the surface amino acid residue area is selected from the group consisting of: amino acid position 305-310 of DTT and amino acid position 295-310 of DTT

In one preferred embodiment, the antigenic epitope is introduced into 1 to 5 (preferably 1 to 3) surface amino acid residue areas.

In one preferred embodiment, the surface amino acid residue area comprises C-terminal of the carrier protein.

In one preferred embodiment, the surface amino acid residue area comprises N-terminal of the carrier protein.

In one preferred embodiment, the antigenic epitope is linked to the C-terminal and/or N-terminal of the carrier protein.

In one preferred embodiment, there is a linker peptide between the antigenic epitope and the carrier protein. Preferably the linker peptide has 3 to 30 amino acids. More preferably, the linker peptide has 4 to 20 amino acids. Most preferably, the linker peptide has 5 to 10 amino acids.

In one preferred embodiment, there is not a linker peptide between the antigenic epitope and the carrier protein.

In one preferred embodiment, the antigenic epitope replaces 1 to 50 amino acids of the C-terminal or N-terminal of the carrier protein. Preferably, the antigenic epitope replaces 5 to 30 amino acids of the C-terminal or N-terminal of the carrier protein. More preferably, the antigenic epitope replaces 10 to 20 amino acids of the C-terminal or N-terminal of the carrier protein.

In one preferred embodiment, the carrier protein is a toxin transmembrane domain of Diphtheria (DTT), and the surface amino acid residue area suitable for inserting an antigenic epitope comprises those shown in the following table:

The surface amino acid residue area in toxin transmembrane domain of Diphtheria (DTT) and suitable for inserting an antigenic epitope

| NO. | Amino acid position of 1F0L.pdb | Amino acid sequence of 1F0L.pdb |
|---|---|---|
| DTT-1 | 221-225 | KEHGP |
| DTT-2 | 230-241 | MSESPNKTVSEE |
| DTT-3 | 255-261 | LEHPELS |
| DTT-4 | 267-277 | TGTNPVFAGAN |
| DTT-5 | 287-299 | QVIDSETADNLEK |
| DTT-6 | 305-311 | SILPGIG |
| DTT-7 | 317-325 | ADGAVHHNT |

In the second aspect of the invention, it provides a polynucleotide encoding the recombinant protein according to the first aspect of the invention.

In the third aspect of the invention, it provides a vector containing the polynucleotide according to the fourth aspect of the invention.

In the fourth aspect of the invention, it provides a host cell which contains the vector according to the third aspect of the invention, or has the polynucleotide according to the second aspect of the invention integrated in its genome.

In one preferred embodiment, the host cell comprises a prokaryotic and eukaryotic cell.

In one preferred embodiment, the host cell comprises an *Escherichia coli,* a Yeast, a CHO cell, a DC cell and so on.

In the fifth aspect of the invention, it provides a pharmaceutical composition which comprises the recombinant protein according to the first aspect of the invention, the polynucleotide according to the second aspect of the invention, or the vector according to the third aspect of the invention, or the host cell according to the fourth aspect of the invention, and a pharmaceutically acceptable carrier and/or excipient.

In one preferred embodiment, the composition is vaccine.

In the sixth aspect of the invention, it provides a vaccine composition which comprises the recombinant protein according to the first aspect of the invention, the polynucleotide according to the second aspect of the invention, or the vector according to the third aspect of the invention, or the host cell according to the fourth aspect of the invention, and an immunologically acceptable carrier and/or excipient.

In one preferred embodiment, the vaccine composition further comprises an adjuvant.

In one preferred embodiment, the vaccine composition is a nucleic acid vaccine composition which comprises the polynucleotide according to the second aspect of the invention, or the vector according to the third aspect of the invention.

In one preferred embodiment, the adjuvant comprises alumina, saponin, quil A, muramyl dipeptide, mineral oil or vegetable oil, vesicle-based adjuvant, non-ionic block copolymer or DEAE dextran, cytokine (including IL-1, IL-2, IFN-γ, GM-CSF, IL-6, IL-12, and CpG).

In the seventh aspect of the invention, it provides a use of the recombinant protein with an antigenic epitope according to the first aspect of the invention, wherein,
(a) the recombinant protein is used for preparing an antibody against the antigenic epitope; and/or
(b) the recombinant protein is used for preparing a medicine for treatment of an antigenic epitope-related disease.

In one preferred embodiment, the disease comprises autoimmune disease (such as rheumatoid arthritis), cancer, cardiovascular disease, etc.

In the eighth aspect of the invention, it provides a method of treatment which comprises a step of administering the recombinant protein of the first aspect of the invention, the pharmaceutical composition of the fourth aspect of the invention or the vaccine composition of the third aspect of the invention to a subject in need of.

In the ninth aspect of the invention, it provides an antigenic epitope peptide, wherein the antigenic epitope peptide is derived from a low immunogenic protein of mammal (such as human), and contains one or more antigenic epitopes,
and the length of amino acid sequence of the antigenic epitope peptide is 5-100% of the full-length of corresponding low immunogenic protein (preferably, 5-70% of the full-length of corresponding low immunogenic protein; more preferably, 5-50% of the full-length of corresponding low immunogenic protein; and most preferably, 5-30% of the full-length of corresponding low immunogenic protein, such as 10%, 15%, 20%, 25%), and the length of the antigenic epitope peptide is 5-500 amino acids;
and a recombinant protein formed by the antigenic epitope peptide with a carrier protein of the present invention can induce an immune response against the low immunogenic protein in a mammal of the same species.

Preferably, the length of the antigenic epitope peptide is 3-57 amino acids; and more preferably, the length of the antigenic epitope peptide is 5-17 amino acids.

In one preferred embodiment, the low immunogenic protein comprises VEGF, TNF-α, HER2 protein, clotting factor, interleukin, FAP, PDL1, or EGFR.

In one preferred embodiment, the antigenic epitope peptide is derived from TNF-α, and is selected from the group consisting of:
(1) an amino acid sequence as shown in SEQ ID NO.: 10 or SEQ ID NO.: 12;
(2) a derivative polypeptide, which is derived from the amino acid sequence as shown in SEQ ID NO.: 10 or SEQ ID NO.: 12 by replacement, deletion, or addition of one or more amino acid residues and is capable of eliciting an immune response after it is recombinant with the carrier protein.

In one preferred embodiment, the antigenic epitope peptide is derived from TNF-α, and is selected from the group consisting of:
(a) a polypeptide as shown in any of SEQ ID NO.: 5 to 8, SEQ ID NO.:34, SEQ ID NO.:117 or SEQ ID NO.:118;
(b) a derivative polypeptide, which is derived from (a) by replacement, deletion, or addition of one or more amino acid residues in the amino acid sequence as shown in any of SEQ ID NO.: 5 to 8, or SEQ ID NO.:34, and is capable of eliciting an immune response against TNF-αafter it is recombinant with the carrier protein.

In one preferred embodiment, the antigenic epitope peptide is derived from clotting factor FXI, and is selected from the group consisting of:
(a) a polypeptide as shown in SEQ ID NO.: 40 or SEQ ID NO.:41;
(b) a derivative polypeptide, which is derived from (a) by replacement, deletion, or addition of one or more amino acid residues in the amino acid sequence as shown in SEQ ID NO.: 40 or SEQ ID NO.:41 and is capable of eliciting an immune response after it is recombinant with the carrier protein.

In one preferred embodiment, the antigenic epitope peptide comprises a catalytic structural domain of clotting factor FXI or a homologous sequence thereof.

In one preferred embodiment, the protein sequence of the catalytic structural domain of clotting factor FXI is shown in SEQ ID NO.:45.

In one preferred embodiment, the antigenic epitope peptide is derived from clotting factor FXI, and is selected from the group consisting of:
(a) a polypeptide as shown in SEQ ID NO.: 45;
(b) a derivative polypeptide, which is derived from (a) by replacement, deletion, or addition of one or more amino acid residues in the amino acid sequence as shown in SEQ ID NO.: 45 and is capable of eliciting an immune response after it is recombinant with the carrier protein.

In one preferred embodiment, the antigenic epitope peptide is derived from FXII.

In one preferred embodiment, the antigenic epitope is one or more members selected from the group consisting of:
(1) EAFSPVSYQHDLA;
(2) EGFSSITYQHDLA; and
(3) a peptide fragment in other FXII protein and corresponding to any of the epitope sequences of (1) and (2).

In one preferred embodiment, the antigenic epitope comprises a catalytic structural domain of clotting factor FXII.

In one preferred embodiment, the catalytic structural domain of FXII is selected from the group consisting of:
(a) a polypeptide having an amino acid sequence as shown in SEQ ID NO.: 129 or SEQ ID NO.:130;
(b) a derivative polypeptide, which is derived from (a) by replacement, deletion, or addition of one or more amino acid residues in the amino acid sequence as shown in SEQ ID NO.: 129 or SEQ ID NO.:130 and is capable of eliciting an immune response after it is recombinant with the carrier protein.

In one preferred embodiment, the antigenic epitope is derived from a catalytic structural domain of FAP.

In one preferred embodiment, the catalytic structural domain of FAP is selected from the group consisting of:
(a) a polypeptide having an amino acid sequence as shown in SEQ ID NO.: 108;
(b) a derivative polypeptide, which is derived from (a) by replacement, deletion, or addition of one or more amino acid residues in the amino acid sequence as shown in SEQ ID NO.: 108 and is capable of eliciting an immune response after it is recombinant with the carrier protein.

In one preferred embodiment, the antigenic epitope peptide is derived from FAP, and is selected from the group consisting of:
(a) a polypeptide having an amino acid sequence as shown in SEQ ID NO.: 49;
(b) a derivative polypeptide, which is derived from (a) by replacement, deletion, or addition of one or more amino acid residues in the amino acid sequence as shown in SEQ ID NO.: 49 and is capable of eliciting an immune response after it is recombinant with the carrier protein.

In one preferred embodiment, the antigenic epitope is derived from VEGF.

In one preferred embodiment, the antigenic epitope is selected from the group consisting of:
(a) a polypeptide having an amino acid sequence as shown in SEQ ID NO.: 63;
(b) a derivative polypeptide, which is derived from (a) by replacement, deletion, or addition of one or more amino acid residues in the amino acid sequence as shown in SEQ ID NO.: 63 and is capable of eliciting an immune response after it is recombinant with the carrier protein.

In one preferred embodiment, the antigenic epitope is derived from PDL1.

In one preferred embodiment, the antigenic epitope is derived from PDL1E.

In one preferred embodiment, the antigenic epitope is derived from PDL1 and is selected from the group consisting of:
(a) a polypeptide having an amino acid sequence as shown in SEQ ID NO.:67, SEQ ID NO.:68, SEQ ID NO.:122, SEQ ID NO.:123, SEQ ID NO.:124 or SEQ ID NO.:125;
(b) a derivative polypeptide, which is derived from (a) by replacement, deletion, or addition of one or more amino acid residues and is capable of eliciting an immune response after it is recombinant with the carrier protein.

In one preferred embodiment, the antigenic epitope is derived from EGFR.

In one preferred embodiment, the antigenic epitope is derived from sub-region III of EGFR.

In one preferred embodiment, the antigenic epitope is derived from EGFR and is selected from the group consisting of:
(a) a polypeptide having an amino acid sequence as shown in SEQ ID NO.:83, 110, or 111;
(b) a derivative polypeptide, which is derived from (a) by replacement, deletion, or addition of one or more amino acid residues and is capable of eliciting an immune response after it is recombinant with the carrier protein.

In one preferred embodiment, the antigenic epitope is derived from epidermal growth factor receptor (EGFR) and comprises an amino acid sequence as shown in SEQ ID NO.:83, and
at least one amino acid sequence as shown in SEQ ID NO.:110, and SEQ ID NO.:111.

In the tenth aspect of the present invention, it provides a fusion protein which is formed by fusing the antigenic epitope peptide of the ninth aspect of the present invention with the carrier protein.

In one preferred embodiment, the carrier protein and the antigenic peptide is not from the same protein, and the carrier protein comprises at least one T cell epitope, and the carrier protein can enhance the immunogenicity of the epitope peptide.

In one preferred embodiment, the carrier protein comprises Diphtheria toxin (DT), a toxin transmembrane domain of Diphtheria (DTT), Rotavirus VP7, a heat shock protein of *Leishmania,* a flagellin of *Campylobacter jejuni,* a major outer membrane protein of *Chlamydia trachomatis,* a hemocyanin (Keyhole Limpet Hemocyanin, KLH), a Bovine serum albumin (BSA), a Chicken ovalbumin (Ovalbumin, OVA), or a fibrinogen.

In one preferred embodiment, the antigenic epitope peptide is imported into at least one molecular surface amino acid residue area of the carrier protein by splicing, replacement, and/or insertion.

In one preferred embodiment, the "molecular surface amino acid residue area" comprises a loop region, a beta-turn region, N-terminal or C-terminal.

In one preferred embodiment, the antigenic epitope peptide is linked to the C-terminal and/or N-terminal of the carrier protein to form the fusion protein.

In one preferred embodiment, there is a linker peptide between the antigenic epitope peptide and the carrier protein. Preferably, the linker peptide has 3 to 30 amino acids. More preferably, the linker peptide has 4 to 20 amino acids. Most preferably, the linker peptide has 7 to 17 amino acids.

In one preferred embodiment, there is not a linker peptide between the antigenic epitope peptide and the carrier protein.

In one preferred embodiment, the fusion protein is selected from the group consisting of:
(a) a polypeptide as shown in SEQ ID NOs.: 9, 11, 13, 35, 37, 39, 42, 43, 44, 50, 51, 62, 69, 70, 84, 85, 86, 87, 112, 119, 120, 126 or 127;
(b) a derivative polypeptide, which is derived from (a) by replacement, deletion, or addition of one or more amino acid residues and is capable of eliciting an immune response after it is recombinant with the carrier protein.

It should be understood that in the present invention, the technical features specifically described above and below (such as the Examples) can be combined with each other, thereby constituting a new or preferred technical solution, which needs not be specified one by one.

### DESCRIPTION OF FIGURES

Figure 1 show an amplification schematic based on overlapping PCR in examples.

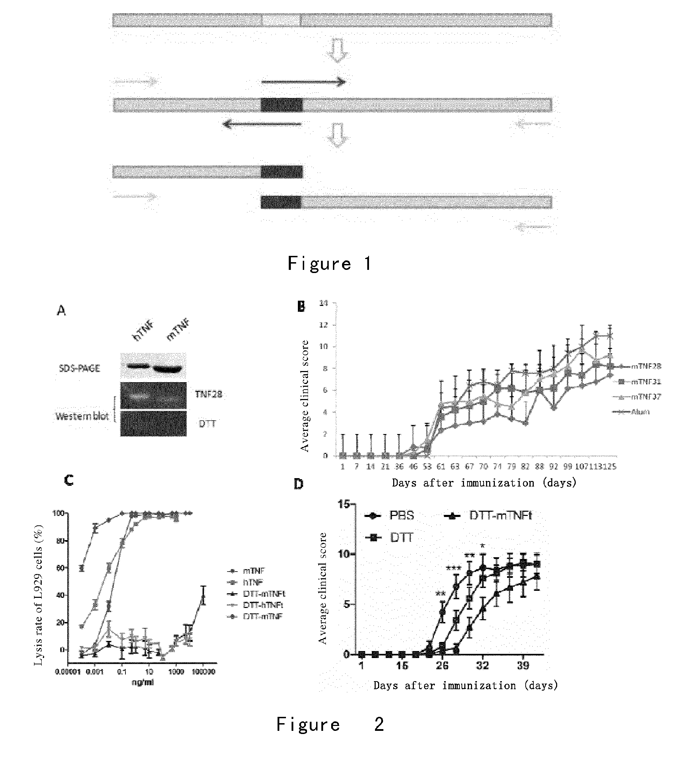

Figure 2 shows the effect of mTNF vaccine for inhibiting disease in Example 1. Figure 2A shows protein hybridization experiments of TNF28 antiserum against mouse or human TNF proteins and compared with DT antiserum. Figure 2B shows an average onset score of each group of mice in immunity and onset duration. It can be seen that significant therapeutical effects of retardation and reduction of inflammation are shown in the group of mTNF28 compared with the control group of Alum. Figure 2C shows biological activity of each fusion protein. Figure 2D shows an average onset score of each group of mice in immunity and onset duration, and it can be seen that significant inhibition effects of the onset score are shown in the group of DTT-mTNFt compared with the control group.
Figure 3 shows the effect of eliciting an immune response by mTNF28-1 antigen protein in Example 2. Figure 3A shows the titer against IgG1 is more than 10,000 obtained by an antibody isotyping analysis of blood taken 60 days after the fourth booster immunization. Figure 3B shows an average onset score of each group of mice in immunity and onset duration and it can be seen that, compared with the control group, the group mTNF28-1 shows significant inhibition effects of the onset score.
Figure 4 shows the effect of antithrombus of FXI vaccine in Example 3. Figure 4A shows a survival rate of mice after immunized with antigen of each group in lethal pulmonary embolism model. Figure 4B shows the time from injecting human placenta leachate into mice to emerge difficulty of breathing. Figures 4C and 4D show the form (C) and wet weight (D) of mice thrombus after antigen immunization.
Figure 5 shows that the antibody in mice immunized with FXI vaccine extends APTT value of normal human plasma (A) and APTT value of FXI specific of plasma (B) in Example 3.
Figure 6 shows the form (C) and wet weight (D) of mice thrombus after immunized with FXII vaccine in Example 4.
Figure 7 shows the inhibition of tumor growth by fusion vaccine DTT-FAP in Example 5. Figure 7A shows survival rate of mice after tumor inoculation. Figure 7B shows a change trend of tumor volume. Figure 7C shows the size of tumor four weeks after inoculation.
Figure 8 shows that the survival time of tumor-bearing mice is significantly extended after immunized with DTT-VEGF in Example 6.
Figure 9 shows blood vessels around tumors, internal pathological morphology of tumor, change trend of tumor weight and tumor volume in different experimental groups in Example 6.
Figure 10 shows inhibition of tumor growth by DTT-PDL1E and DTT-PDL1E recombinant protein vaccine in Example 7. Figure 10A shows change of tumor volume in mice subjected to immunotherapy.
Figure 10B, and Figure 10C and Figure 10D separately show tumor weight change (Figure 10B), weight of tumor-bearing mice (Figure 10C) and ratio of tumor weight to the tumor-bearing mice weight (Fig. 10D) in Example 7.

### DETAILED DESCRIPTION OF INVENTION

Through extensive and intensive researches, the inventors have unexpectedly and firstly discovered that a class of novel recombinant protein can be prepared based on a backbone structure of a suitable carrier protein by importing a peptide fragment from a low immunogenic protein into at least one molecular surface amino acid residue area of the carrier protein by splicing, replacement, and/or insertion. The recombinant protein can effectively stimulate an organism (such as a mammal) to produce an immune response against the recombinant protein, and efficiently produce an immune responses against the peptide fragment from the low immunogenic protein, including producing antibodies. Based on this discovery, the inventors have completed the present invention.

### Definition

As used herein, the term "carrier protein" refers to a protein which is served as a protein structural scaffold in the recombinant protein of the present invention. Typically, the carrier protein is a protein with strong immunogenicity, e.g., a pathogen protein, and the representative examples include (but not limited to): viral protein, bacterial protein, chlamydia protein, mycoplasma protein and so on.

As used herein, the term "antigenic epitope (peptide)" refers to a peptide of other protein for which an immune response is to be induced in an animal. For a carrier protein, such an antigenic epitope is not a peptide fragment from the carrier protein itself which can induce an immune response. Typically, an antigenic epitope refers to a peptide fragment to be targeted in an immune response. Preferably, the antigenic epitope is from a mammal (e.g. human) protein but is not from a carrier protein.

As used herein, the term "pdb" specifically refers to a data file of protein tertiary structure, which is available from Protein Data Bank (www.pdb.org).

As used herein, the term "DTT" refers to the transmembrane domain of diphtheria toxin.

As used herein, the term "T cell epitope" refers to a T cell epitope (also known as a T cell antigenic epitope) which is a peptide fragment produced by antigen-presenting cells after digestion processing of the antigen molecules. The peptide fragment can bind with the major histocompatibility complex (MHC) molecules and then is presented on the cell surface and binded by T cell receptor (TCR), so that T cells are activated. This term also includes a T helper cell epitope and the like.

As used herein, the term "low immunogenic protein" refers to a protein which can not induce a sufficient immune response when an animal is immunized with said protein alone.

As used herein, the term "molecular surface amino acid residue area" or "surface amino acid residue area" refers to an area formed by amino acid residues located at the molecule surface of a protein. Preferably, the "molecular surface amino acid residue area" comprises a loop region, a beta-turn region, N-terminal or C-terminal.

### Representative Carrier Proteins

### 1. Diphtheria toxin and the transmembrane domain thereof

Diphtheria toxin (DT) is an exotoxin produced by Corynebacterium diphtheriae which is infected by beta phages, and is present in the DPT vaccine used in clinical. Security has been verified in clinical uses for many years, and serious adverse effects are extremely rare. There are no reports of allergic reactions caused by the diphtheria ingredients.

Diphtheria toxin molecule consists of 535 amino acid residues, and comprises a catalytic domain (1-193aa), a transmembrane domain (205-378aa) and a receptor-binding domain (386-535aa) which is relatively independent from each other in space. The transmembrane domain and receptor-binding domain itself are nontoxic, and the function thereof is to transduce the catalytic domain into a cell by binding onto a cell surface receptor.

The amino acid sequence of Diphtheria toxin (P00588, DTX_CORBE) is as follows:

GADDVVDSSK SFVMENFSSY HGTKPGYVDS IQKGIQKPKS GTQGNYDDDW KGFYSTDNKY
DAAGYSVDNE NPLSGKAGGV VKVTYPGLTK VLALKVDNAE TIKKELGLSL TEPLMEQVGT EEFIKRFGDG
ASRVVLSLPF AEGSSSVEYI NNWEQAKALS VELEINFETR GKRGQDAMYE YMAQACAGNR VRRSVGSSLS
CINLDWDVIR DKTKTKIESL KEHGPIKNKM SESPNKTVSE EKAKQYLEEF HQTALEHPEL SELKTVTGTN
PVFAGANYAA WAVNVAQVID SETADNLEKT TAALSILPGI GSVMGIADGA VHHNTEEIVA QSIALSSLMV
AQAIPLVGEL VDIGFAAYNF VESIINLFQV VHNSYNRPAY SPGHKTQPFL HDGYAVSWNT VEDSIIRTGF
QGESGHDIKI TAENTPLPIA GVLLPTIPGK LDVNKSKTHI SVNGRKIRMR CRAIDGDVTF CRPKSPVYVG
NGVHANLHVA FHRSSSEKIH SNEISSDSIG VLGYQKTVDH TKVNSKLSLF FEIKS
(SEQ ID No.: 1)

Five T-helper cell epitopes in Diphtheria toxin molecule can be recognized by up to 80% human MHC class II. Four T-helper cell epitopes are located in the transmembrane domain of diphtheria toxin (T region, DTT) which are DTT-Th epitope 271-290 (271-PVFAGANYAAWAVNVAQVID-290), DTT-Th epitope 321 -340 (321-VHHNTEEIVAQSIALSSLMV-340), DTT-Th epitope 331-350 (QSIALSSLMVAQAIPLVGEL-350), and DTT-Th epitope 351-370 (351-VDIGFAAYNFVESIINLFQV-370), respectively, and mainly distribute in three long α helix structure (276-ANYAAWAVNVA-286; 327-EIVAQSIALSSLMVAQAIPLV-347; 353-IGFAAYNFVESIINLFQVVHNSYN-376).

A simulation study of diphtheria toxin protein was done by the present inventors and α helices and P folding elements required for structural stability and T cell epitopes were reserved and the positions of surface amino acid residue areas capable of being replaced to implant an antigenic epitope were shown in the following table.

The summary of implantable position of surface amino acid residue area of Diphtheria toxin (DT)

| NO. | Implantable position for epitope (amino acid residues) | Number of residues replaced |
|---|---|---|
| DT-1 | 6-10 | 5 |
| DT-2 | 26-34 | 9 |
| DT-3 | 26-43 | 18 |
| DT-4 | 26-48 | 23 |
| DT-5 | 68-76 | 9 |
| DT-6 | 86-88 | 3 |
| DT-7 | 107-112 | 6 |
| DT-8 | 129-133 | 5 |
| DT-9 | 169-177 | 9 |
| DT-10 | 222-224 | 3 |
| DT-11 | 232-239 | 8 |
| DT-12 | 256-258 | 3 |
| DT-13 | 268-271 | 4 |
| DT-14 | 289-296 | 8 |
| DT-15 | 317-320 | 4 |
| DT-16 | 348-354 | 7 |
| DT-17 | 438-440 | 3 |
| DT-18 | 463-467 | 5 |
| DT-19 | 495-502 | 8 |
| DT-15 | 516-523 | 8 |

The transmembrane domain of diphtheria toxin (DTT) itself is non-toxic, and the core frame mainly comprises α helical elements which are connected by flexible loop regions among them.

The amino acid sequence of DTT (1F0L.pdb: 202- 378) is as follows:

### 202 INLDWDVIRD KTKTKIESLK EHGPIKNKMS ESPNKTVSEE KAKQYLEEFH QTALEHPELS

### 262 ELKTVTGTNP VFAGANYAAW AVNVAQVIDS ETADNLEKTT AALSILPGIG SVMGIADGAV

### 322 HHNTEEIVAQ SIALSSLMVA QAIPLVGELV DIGFAAYNFV ESTINLFQVV HNSYNRP (SEQ ID No.: 2)

A simulation study of DTT protein was done by the present inventors and α helices and β folding elements required for structural stability and T cell epitopes were reserved and the positions of surface amino acid residue areas capable to be replaced to implant an antigenic epitope were shown in the following table.

The summary of surface amino acid residue area suitable for implantting an antigenic epitope in DTT

| NO. | General implantable position (amino acid residues) | Preferable implantable position (amino acid residues) | Preferable number of residues replaced |
|---|---|---|---|
| DTT-1 | 220-226 | 222-224 | 3 |
| DTT-2 | 228-241 | 230-239 | 10 |
| DTT-3 | 253-262 | 255-260 | 6 |
| DTT-4 | 265-276 | 267-274 | 8 |
| DTT-5 | 285-298 | 287-296 | 10 |
| DTT-6 | 303-312 | 305-310 | 6 |
| DTT-7 | 313-327 | 315-325 | 11 |
| DTT-8 | 315-322 | 317-320 | 4 |

In a preferred embodiment of the present invention, a peptide fragment from a target protein which is proposed for drug interventions is implanted into DTT to replace a surface amino acid residue area (including amino acid residues of loop region between α helix elements) of DTT.

Study of the present invention have showed that after integrating a diphtheria toxin transmembrane and a peptide fragment of the target protein, their respective foldings are not or not essentially affected. In the recombinant protein, DTT is the protein scaffold, and after implanting the target protein peptide fragment into the DTT, an immune response against the target protein can be induced in animals. Therefore, DTT is a very suitable protein scaffold.

### Composition and Methods of Administration

The present invention further provides a pharmaceutical composition which comprises (i) the recombinant protein of the present invention or the polynucleotide encoding the recombinant protein of the present invention, and (ii) a pharmaceutically or immunologically acceptable excipient or adjuvant.

In the present invention, the term "comprising" refers to various components can be used or present together in the composition of the present invention. Thus, term "comprising" includes term "essentially consist of" and "consist of".

The composition of the present invention comprises a pharmaceutical composition and a vaccine composition.

The composition of the present invention can be monovalent (comprising only one kind of recombinant protein or polynucleotide) or multivalent (comprising several kinds of recombinant proteins or polynucleotides).

The pharmaceutical composition or vaccine composition of the present invention can be prepared into various conventional formulations including (but not limited to): injections, granules, tablets, pills, suppositories, capsules, suspensions, sprays and the like.

### (1) Pharmaceutical Composition

The pharmaceutical composition comprises a therapeutically effective amount of recombinant protein or polynucleotide of the invention.

The term "therapeutically effective amount" as used herein refers to an amount of a therapeutic agent to treat, ameliorate, or prevent a desired disease or condition, or to exhibit a detectable therapeutic or preventative effect. The effect can be detected by, for example, antigen levels. Therapeutic effects also include reduction in physical symptoms. The precise effective amount for a subject will depend upon the subject's size and health, the nature and extent of the condition, and the therapeutics or combination of therapeutics selected for administration. Thus, it is not useful to specify an exact effective amount in advance. However, the effective amount for a given situation can be determined by routine experimentation and is within the judgment of the clinician.

For purposes of the present invention, an effective dose will be from about 0.001 mg/kg to 1000 mg/kg, and preferably about 0.01 mg/kg to 100 mg/kg in an individual to be administered.

A pharmaceutical composition can also contain a pharmaceutically acceptable carrier. The term "pharmaceutically acceptable carrier" refers to a carrier for administration of a therapeutic agent, such as a recombinant protein of the invention. The term refers to any pharmaceutical carrier that does not itself induce the production of antibodies harmful to the individual receiving the composition, and which may be administered without undue toxicity. Suitable carriers may be large, slowly metabolized macromolecules such as proteins, polysaccharides, polylactic acids, polyglycolic acids and so on. Such carriers are well known to those of ordinary skill in the art. A thorough discussion of pharmaceutically acceptable excipients is available in Remington's Pharmaceutical Sciences (Mack Pub. Co., N.J. 1991).

Pharmaceutically acceptable carriers in therapeutic compositions may contain liquids such as water, saline, glycerol and ethanol. Additionally, auxiliary substances, such as wetting or emulsifying agents, pH buffering substances, and the like, may be present in such vehicles. Typically, the therapeutic compositions are prepared as injectables, either as liquid solutions or suspensions; solid forms suitable for solution in, or suspension in, liquid vehicles prior to injection may also be prepared. Liposomes are included within the definition of a pharmaceutically acceptable carrier.

### (ii) Vaccine Composition

A vaccine (composition) according to the invention may either be prophylactic (i.e., to prevent disease) or therapeutic (i.e., to treat disease after sickness).

Such vaccines comprise an immunizing antigen (including recombinant protein of the invention), usually in combination with "pharmaceutically acceptable carriers," which include any carrier that does not itself induce the production of antibodies harmful to the individual receiving the composition. Suitable carriers are typically large, slowly metabolized macromolecules such as proteins, polysaccharides, polylactic acids, polyglycolic acids, polymeric amino acids, amino acid copolymers, lipid aggregates (such as oil droplets or liposomes), and so on. Such carriers are well known to those of ordinary skill in the art. Additionally, these carriers may function as immunostimulating agents ("adjuvants"). Furthermore, the immunogen or antigen may be conjugated to a bacterial toxoid, such as a toxoid from diphtheria, tetanus, cholera, H. pylori, or other pathogens.

Preferred adjuvants to enhance effectiveness of the composition include, but are not limited to: (1) aluminum salts (alum), such as aluminum hydroxide, aluminum phosphate, aluminum sulfate, etc; (2) oil-in-water emulsion formulations, such as (a) MF59 (WO 90/14837), (b) SAF, and (c) RIBI™ adjuvant system (RAS), (Ribi Immunochem, Hamilton, Mont.); (3) saponin adjuvants; (4) Complete Freund's Adjuvant (CFA) and Incomplete Freund's Adjuvant (IFA); (5) cytokines, such as interleukins (e.g., IL-1, IL-2, IL-4, IL-5, IL-6, IL-7, IL-12, etc.), interferons (e.g., gamma interferon), macrophage colony stimulating factor (M-CSF), tumor necrosis factor (TNF), etc.; (6) detoxification variants of ADP-ribosylating bacterial toxins (e.g., *Escherichia coli* heat-labile toxin LT) and (7) other substances that act as immunostimulating agents to enhance the effectiveness of the composition.

The vaccine compositions including immunogenic compositions (e.g., which may include the antigen, pharmaceutically acceptable carrier, and adjuvant) typically will contain diluents, such as water, saline, glycerol, ethanol, etc. Additionally, auxiliary substances, such as wetting or emulsifying agents, pH buffering substances, and the like, may be present in such vehicles.

More specifically, vaccines comprising immunogenic compositions comprise an immunologically effective amount of the immunogenic polypeptides, as well as any other of the above-mentioned components, as needed. By "immunologically effective amount", it is meant that the administration of that amount to an individual, either in a single dose or as part of a series, is effective for treatment or prevention. This amount varies depending upon the health and physical condition of the individual to be treated, the taxonomic group of individual to be treated (e.g., human), the capacity of the individual's immune system to synthesize antibodies, the degree of protection desired, the formulation of the vaccine, the treating doctor's assessment of the medical situation, and other relevant factors. It is expected that the amount will fall in a relatively broad range that can be determined through routine trials.

Typically, the vaccine compositions or immunogenic compositions are prepared as injectables, either as liquid solutions or suspensions; solid forms suitable for solution in, or suspension in, liquid vehicles prior to injection may also be prepared. The preparation also may be emulsified or encapsulated in liposomes for enhanced adjuvant effect.

In addition, the vaccine compositions of the present invention can be monovalent or multivalent vaccine.

### (iii) Route and dosage of administration

Once formulated, the compositions of the invention can be administered directly to a subject. The subjects to be treated can be mammals; and in particular, human subjects can be treated.

When used as a vaccine, the recombinant protein of the invention can be applied directly to individuals using known methods. Usually, these vaccines are administrated by the same administration route of conventional vaccines and/or simulating pathogen infection routes.

Routes for administering a pharmaceutical composition or vaccine composition of the present invention include (but are not limited to): intramuscular, subcutaneous, transdermal, pulmonary, intravenous, nasal, oral or other parenteral routes of administration. If desired, the route of administration may be combined, or adjusted depending on the disease situation. Vaccine compositions may be administered in single or multiple doses, and may include administering a booster dose to initiate and/or maintain immunity.

Recombinant protein vaccines should be administered by an "effective amount", namely the amount of recombinant protein in the chosen route of administration is sufficient to elicit an immune response, and can effectively promote the protection of host to resistant related diseases.

The typical diseases include (but are not limited to): autoimmune diseases, cancer and the like.

The amount of recombinant proteins in the vaccine agents is determined as an amount which can cause an immune protective response without significant side effects. Typically, after infecting a host cells, each agent of the vaccine sufficiently contains about 1µg-1000mg, preferably 1µg-100mg, and more preferably 10µg-50mg protein. Available methods to determine the best dosage of specific vaccines include standard methods to observe the antibody titers and other reactions of the subject. Immunity levels provided by the vaccine can be monitored to determine whether it is necessary to enhance dose. After evaluating the titer of antibody in serum, an enhanced dose may be needed for immunization. Administration of adjuvant and/or immune stimulator can improve the immune response of the protein of the present invention.

A preferred method is administering an immunogenic composition via a parenteral (subcutaneous or intramuscular) route by injection.

In addition, the vaccine of the present invention may be administered in conjunction with other immunoregulatory agents, or in combination with other therapeutic agents.

The main advantages of the present invention include:
(1) An immune response of body can effectively be stimulated against a specific epitope target having low immunogenicity.
(2) The cost of producing recombinant proteins carrying epitopes is low and the administration is convenient.
(3) Compared with the fomulation containing a chemically coupled carrier protein, the antigen of the invention has a definite structure, controllable quality and high safety.

The present invention will be further illustrated below with reference to the specific examples. It should be understood that these examples are only to illustrate the invention, not to limit the scope of the invention. The experimental methods with no specific conditions described in the following examples are generally performed under the conventional conditions (e.g., the conditions described by Sambrook et al., Molecular Cloning: A Laboratory Manual (New York: Cold Spring Harbor Laboratory Press, 1989)), or according to the manufacture's instructions. Unless indicated otherwise, parts and percentage are calculated by weight.

### Experimental method:

### Method 1 Structure design of recombinant protein antigen

The structure design of recombinant protein antigen with single epitope is accomplished by transplanting an amino acid sequence of epitope peptide in a target protein into an epitope exhibiting position in DTT and by replacing the original amino acid residues so as to form a new protein structure. The structure design of recombinant protein antigen with multi-epitopes is accomplished by inserting a peptide fragment or domain with multi-epitopes of target protein to C-terminal of DTT, or by inserting to C-terminal of DTT via a linker so as to form a new protein structure.

### Method 2 Construction of an expression vector of the recombinant protein

DTT gene primers (DTT-F and DTT-R) and the target protein gene primers were designed according to mRNA of DTT gene and antigenic epitope region sequence of open reading frame of the target protein gene mRNA in GeneBank. The antigenic epitope region sequence was introduced into an appropriate epitope-exhibiting position of DTT based on principle of overlapping PCR. The restriction sites of BamHI and XhoI were introduced to the head and tail end and three protective bases were introduced (Figure 1). Each primer was synthesized by Nanjing GenScript Biotechnology Co., Ltd.
(1) Construction of recombinant protein expression vector with epitopes within DTT by inserting/replacing. The first round of PCR amplification was implemented using a DTT genomic DNA as a template and the primers of DTT-F (containing BamH I restriction site) and the target protein-2R; while another PCR amplification was implemented using a DTT genomic DNA as a template and the primers of DTT-R (containing XhoI restriction site) and the target protein-3F. After mixing above two amplification products, the recombinant protein gene was amplified by PCR using primers of DTT- F and DTT-R.
(2) Construction of recombinant protein expression vector with epitopes linked to C-terminal of DTT by inserting/replacing. The first round of PCR amplification was implemented using a DTT genomic DNA as a template and the primer of DTT-F (containing BamH I restriction site and sequence of enterokinase cleavage site DDDDK). And then the target protein antigenic epitope gene was amplified by PCR using the primer of the target protein gene (the reverse primer containing sequence of *Xho I* restriction site). After mixing above two amplification products, the recombinant protein gene was amplified by PCR using DTT-F primer and the reverse primer of the target protein gene.

The recombinant protein gene was digested with *BamH I* and *Xho I* and ligated into plasmid pGEX 6p-1, thereby forming an expression plasmid of pGEX-DTT-target protein epitopes. The correctness of the plasmid was confirmed by sequencing.

After preparation of PCR system, the PCR was conducted: 94°C denatured 2 min; 94°C 15s, 55°C 30s, 68°C 2 min, 35 cycles; and 68°C 10 min. 2µL amplification product was subjected to electrophoresis and a desired band was observed after Goldview staining.

The PCR products and pEGX-6P-1 vector after double digestion were recovered and purified, and cleaned with an AXYGEN gel cleaning kit. T4 ligase was used in the ligation reaction on a PCR instrument at 16°C for 5 h. The ligation product was transformed into *E. coli* by heat shocking. The 5 uL ligation product was added into competent cells and was gently mixed. The mixture was placed into an ice bath for 20 min and then was transferred into a 42°C water bath for 90s, and then into the ice bath for 2 min again. A pre-heated LB medium at 37°C was added in a volume of 900 µL, and shaked at 37°C at 150rpm for 1h for recovery. 200 µL sample was applied onto a LB plate containing Amp antibiotics and cultured at 37°C for 20 h for further identification of transformants. The plasmid extracted was amplified by PCR using the head and tail primers. After identification, sterile glycerol was added into positive clones with a final concentration of 15% and preserved at -80°C ultra-low temperature refrigerator for use as an *E. coli* bacteria containing recombinant protein expression plasmid. Positive clones were identified by sequencing and sequence comparison to determine whether the linkage was successful.

**Table 1. Universal primers for inserting epitope into C-terminal of DTT gene**

| Primer name | Primer sequences |
|---|---|
| DTT-F (SEQ ID NO.:3) | CGCGGATCCCTGGAAGTTCTGTTCCAGGGGCCCATAAATCTTGA TTGGGATGTC |
| DTT-R (SEQ ID NO.:4) | CCGCTCGAGCTAGGGACGATTATACGAATTATG |

### Method 3. Expression and identification of recombinant protein and large-scale production

A single colony of *E. coli* containing the recombinant protein expression plasmid was inoculated into 5mL LB medium, and cultured at 37°C overnight. And then the culture was diluted into 50mL LB medium containing 50ng/L Amp with a 1: 50 dilution, and cultured for about 4 hours so that OD600 was about 0.6. IPTG was added to a final concentration of 1mM, and it was induced at 37°C for 4 hours and subjected to identify expression. The induced culture with a volume of 50 mL was placed on ice for 30 min, centrifuged at 12000rpm for 8 min. Cells were collected and supernatant was discarded. Precipitate was suspended with 1xPBS (5mL/100mL culture) plus a same volume of cold sterile water. 300µg/mL lysozyme was added. After gently shacked on ice for 30 min, the mixture was set at -70°C cryopreservation for 12h. The sample was thawed under running water and protease inhibitor was added. The sample was decomposed by ultrasonic (200W power) in ice bath in an intermittent way which comprised decomposing 5s and interving 5s and which was repeated for 8 min. The results of decomposition could be observed under a microscope. The mixture was centrifuged at 12,000g for 12min at 4°C. Samples of mixture, supernatant and precipitate were stored at 4°C for use.

Protein level in samples of mixture, supernatant and precipitate was measured with Coomassie Brilliant Blue quantitative measurement. The samples were diluted to a certain concentration, and 200 µL were taken, and added 50µL 5xbuffer, and boiled for 5min. After cooling, it was centrifuged at 12,000 rpm for 8min and then loaded. 20µL of above mixture was taken with a micro-injector. The molecular weight and expression amount of the induced protein were analyzed by SDS-PAGE electrophoresis, so as to determine the consistency with theoretical molecular weight and solubility of expression.

The positive expression strains were picked, and inoculated into 50mL selective Amp LB liquid medium, and shaked overnight at 200rpm/min at 37°C. The next day, 1mL of culture was inoculated into 1L LB liquid medium, and a total of 4 bottles were made using the same operation, and were cultured at 37°C at 180rpm for 4h. A tube of sample was taken as a control without adding IPTG. The rest bacterium suspension was added 1 mol/L IPTG to a final concentration of 1 mM, and continuously cultured for 10 hours. After induction, all bacteria suspension was centrifuged at 6000rpm for 8 min. Cell precipitate was collected and resuspended with 1mL PBS (pH = 7.3) for wash. Cells were collected by centrifugation and added PBS (pH = 7.3) in a volume of 250 mL and decomposed by ultrasonic for 15min after resuspended. After pulse decomposition, samples were centrifuged in a 50 mL centrifuge tubes at 15000rpm for 40min. Supernatant was taken carefully, and stored at 4 °C for use.

A 5mL GST prepacked column (GE) was first balanced with 1xPBS (140mM NaCl, 2.7mM KCl, 10mM Na₂HPO₄, 2mM KH₂PO₄) with a flow rate of about 4mL/min and 5 column volumes for balance. The sample of protein after above treatment was loaded with a flow rate of 1mL/min. After loading, the column was washed with 1xPBS to remove impurities with a flow rate of 4mL/min and eluted at least 10 column volumes. Then the column was balanced with 1xPSP buffer for at least five volumes. 800 µL of PSP enzyme was added and flowed through the GST column so as to homogenously fill the whole column. After cleavaging at 4 °C for 12-16 hours, the protein was eluted with eluent (50mM Tris-HCl, 10m GSH, pH8.0) in 2-3 column volumes and a flow rate of 2mL/min. The used column and instruments was washed with 20% ethanol. Then the protein was dialyzed into saline. After quantification, the protein was diluted into 0.5mg/mL, and the purity was more than 90% by electrophoresis detection. After dialysis with saline, the protein was stored at -20 °C refrigerator for use. The purity of three proteins prepared was all above 90%.

### Method 4 Animal immunization using fusion protein and detection of antigen titer

The fusion protein antigen diluted to 0.5mg/mL and aluminum hydroxide (sigma) adjuvant or Freund's adjuvant diluted to 2mg/mL were mixed. Mice were injected subcutaneously with multi-points and a booster immunization was implemented every other week. After twice booster immunization, blood was collected from the orbit and standed at 37 °C for 2h, centrifuged at 4000rpm for 10min. The supernatant serum was collected for use.

Target cells or target proteins were diluted to a concentration of 100ng/100µl with a coating buffer (50mmol/L bicarbonate buffer, pH 9.6), transferred to a polystyrene 96-well plate (100µl for each well), incubated at 4 °C overnight and washed with PBST (0.02 M phosphate, 0.15 M NaCl, 0.15% Tween-20, pH 7.4) for 6 times and each wash was 5 minutes. Skimmed milk powder was added in a volume of 300µl (dissolved in PBST with a protein concentration of 5%), and incubated at 37 °C for 2 hours for blocking. Then it was washed with PBST (0.02 M phosphate, 0.15 M NaCl, 0.15% Tween-20, pH 7.4) for 6 times and each wash was 5 minutes. Then the immune serum was diluted to a certain concentration with skimmed milk powder (dissolved in PBST with a protein concentration of 5%). 100µl was added in each well, incubated at 37 °C for 1 hour and washed with PBST (0.02 M phosphate, 0.15 M NaCl, 0.15% Tween-20, pH 7.4) for 6 times and each wash was 5 minutes. Goat anti-rabbit-IgG-HRP conjugate (1: 5,000 dilution) was added in a volume of 100 µL, incubated at 37 °C for 1 hour and washed with PBST (0.02 M phosphate, 0.15 M NaCl, 0.15% Tween-20, pH 7.4) for 6 times and each wash was 5 minutes. Finally, 100µl of substrate solution was added (10ml of substrate solution contains 1mg tetramethylbenzidine (TMB), 0.0969g sodium citrate, 0.3496g Na₂HPO₄·12HO₂, 32µl 0.75% H₂O₂), incubated at 37 °C for 30 minutes. A volume of 50µl of H₂SO₄ (2mol/L) was added to terminate the reaction. Each well was measured at 450nm on a microplate reader

### Experiment 5 Construction of a mouse model of collagen

After the mice were immunized three times, a model of rheumatoid arthritis was made with the following specific operation. Type - collagen of bovine (2mg/mL) and CFA (containing 1mg/mL of *M. tuberculosis*) in a ratio of 1:1 were injected in a total volume of 200uL into the back on ice by multi-point injection on 42 day. On day 63, type 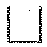 collagen of bovine (4mg/mL) and CFA (containing 1mg/mL of *M. tuberculosis*) in a ratio of 1:1 were blowed and beated with a tip on low temperature ice for emulsion (standard for emulsion was that when a droplet was dropped into water, the droplet did not immediately spread), and then 50uL was injected into a position 1.5cm from the base of mouse tail. On day 70, type 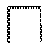 collagen of bovine (4mg/mL) and CFA (containing 1mg/mL of *M. tuberculosis.* Into each 1 ml CFA was newly added 3mg *M. tuberculosis*) in a ratio of 1:1 were blowed and beated with a tip on low temperature ice for emulsion, and then 50uL was injected into a position 1.5cm from the base of mouse tail. Hereafter, the weight and incidence of mice were detected and recorded twice a week.

Rating criteria. The degree of incidence of mice was evaluated with a four-point evaluation. 0: no evidence of redness and swelling; 1 point: erythema and mild swelling confined to the middle foot (tarsal) or ankle (there was one toe redness); 2 points: erythema and mild swelling spread from the ankle to the middle foot (there were two or more toes redness); 3 points: erythema and moderate swelling spread from the ankle to the metatarsal joints (ankle or hip swelling); 4 points: erythema and severe swelling including ankle, foot and toes (all the joints and toes were swollen and could not bend). All of four limbs were scored separately, so the highest score was 16 points.

### Method 6 Western Identification

The original sample was generally loaded in a quantity of 20-30ug and a constant current electrophoresis was implemented with a current strength at an initial voltage of 45V, and when the voltage reached 65V the electrophoresis was regulated to constant voltage electrophoresis and was finished when interested protein swimming > 1 cm away from the lower edge of gel. The gel was balanced for 10min by immersing in a transfer buffer. Membrane and filter paper were cutted into 6 slices depending on the size of gel and placed into the transfer buffer for balancing 10min. If using PVDF membrane, a saturated soaking was needed using pure methanol for 3-5 seconds. Assembly of transfer sandwich: 3 layers of sponge and 3 layers of filter gel and filter sponge were placed layer-by-layer, and bubbles were driven away using a tube. The transferring groove was placed in an ice bath and the sandwich was added (black face to black face). Transferring buffer was added and electrodes were inserted, so the transferring was conducted at 10mA for overnight or 100V for 1h (Pay attention to adjust time). After transferring membrane, the power was cut off and the hybrid membrane was removed.

The membrane was removed from electric transferring groove, rinsed slightly with deionized water and TTBS, immersed in a blocking solution and slowly shaked for 1h. The diluted primary antibody was loaded into a 10mL or 50mL imported centrifuge tubes. The Western membrane was removed from the blocking solution, dried slightly using a filter paper sticking on a cant, and attached to the primary antibody with face down, placed overnight at 4 °C (marked with a pencil at up and down of the front). After incubation with the primary antibody, membranes were rinsed with PBS and then dipped three times (every time was 10min). An appropriate secondary antibody was selected according to the primary antibody, and HRP-labeled antibody was selected according to identification method, diluted accordingly (1: 5000), and shacked at room temperature for 1h. After incubation with the secondary antibody was completed, the membranes were rinsed with PBS and then dipped three times (every time was 10min). The A and B emitting solutions were mixed and diluted proportionately. Membranes were rinsed with deionized water, dried with filter paper sticking on a cant, reverse posted on a droplet of mixture of A and B solutions, placed in a preservative film and fixed onto a cassette. A photographic film was placed quickly, the cassette was closed and exposure strength was adjusted.

### Method 7 T cell proliferation assay

Eight centrifuge tubes (50ml) were washed and sterilized. Sixteen centrifuges tube (15ml) were washed and sterilized. Eight sterile cell screen, 75% alcohol (300 ml), two sterile forceps, two small scissors, sterile PBS and RPM1640 + 20% FCS one bottle for each, two 6-well culture plate, and eight disposable syringes (10ml). Mice were killed by cervical vertebra luxation, and soaked into 75% ethanol for 15 minutes. During soaking, lymphocyte separation medium was taken and balanced at room temperature. The sterile forceps and scissors were take out from a super clean bench, wiped with alcohol cotton, and placed in 10cm cell culture dish. 6-well plates were prepared and the filters were put on 50ml centrifuge tubes.

The soaked mouse was placed on its back in the dish with tail outward. Cut off abdominal cavity from one centimeter above the right groin. Poke liver and carefully pull out the spleen with tweezers and put it on 6-well plate. Into each well were previously added 2 ml PBS. The spleen was crushed by syringe piston crown carefully, and rotary grinded to completely dispersed. The resultant cell suspension was carefully sucked on a filter in a centrifuge tube, and all of the cell suspension was filtered. 1ml PBS was used to rinse the wells and was sucked to wash the filter. The centrifuge tube (15ml) was prepared, 3ml lymphocyte separation medium liquid was added, lymphocytes were separated according to the instructions. The cells precipitate was washed again with PBS. Cells were adjusted to 5 x 10⁶/ml with 1640 containing 10% FCS. Into each well was plated 100 µL, and antigen was added in a final concentration of 10µg/ml for stimulation. It was cultured at 37°C with 5% CO₂ for 72 hour. 10 µL MTT was added and culture was continued for 4 hours. The culture supernatant and the suspended cells were discarded. Dimethylsulfoxide (100 µL) was added, shocked at room temperature to dissolve the blue precipitate. Absorbance were read at 550 nm and 630 nm and OD₅₇₀-OD₆₃₀ was used as the final value to determine the number of viable cells per well. The value of antigen stimulation group minus the control group was used as the activity of cell proliferation.

### Method 8 CTL killing assay

CFSE staining buffer and 7AAD staining buffer were prepared in accordance with the instructions of 7AAD/CFSE Cell-mediated cytotoxicity Assay kit (Abnova, US).

Labeling target cells. Tumor cells were treated with trypsin digestion, centrifuged at 1000 rpm for 5 min and the supernatant was discarded. Add 3 ml Cell-Based Assay Buffer to resuspend, count and then centrifuge at 1000 rpm for 5 min. The supernatant was discarded. The cells were resuspended with CFSE staining buffer to 10⁶ cells/ml and control cells were resuspended with the same volume of Cell-Based Assay Buffer. Cells were incubated in dark at room temperature for 15min and centrifuged at 1000 rpm for 5 min. The supernatant was discarded. The cells were resuspended to 10⁶ cells/ml with 1640 containing 10% FBS. The cells were incubated in a cell incubator for 30min to 1h before use. Lymphocytes of spleen were isolated according to the method of T cell proliferation assay. 6-well plates were plated with 2 ml per well and 10⁷ cells/ml. The antigen peptide in a final concentration of 10µg/ml and IL-2 in a final concentration of 10 unit/ml were added for stimulation, and cells were incubated at 37°C with 5% CO₂ for 5 days for use.

Detection: The labeled target cells were plated in 96-well plates with 10⁴ cells/well. Stimulated effector cells were added to target cells with a ratio of 10: 1, 20: 1, or 50: 1, and the medium was added until the total volume for each well was 200 µL. Incubation was at 37 °C with 5% CO₂ for 6 h. Cells were collected at 400g for 5min and 7-AAD Staining Solution in a volume of 50ul was added for resuspension. Cells were incubated at 4°C in dark for 15min, collected at 400g for 5min, and resuspended with 0.2ml Assay Buffer. Cells were collected at 400g for 5min, resuspended with 0.2ml Assay Buffer and then detected on FCM. Data were recorded. 20,000 cells were collected. A percentage of double positive cells (CFSE+, 7AAD+) in CFSE-positive cells (CFSE+) was calculated as the final killing rate.

Method 9 Culturing tumor cell, establishment of mice model for transplanted cancer and evaluation of immunotherapy effect

Cells and tumor inoculation. Materials comprise DMEM medium, 1640 medium, fetal bovine serum, antibiotics, trypsin (all purchased from Invitrogen Corporation), and PBS (prepared according to instruction of Takara). Incubator (Binder), centrifuge (Feige), and metal container for water bath (Wanhe).

Culturing and transplantation of tumor cells. CT26, B16-F10, Lewis and other tumor cells were purchased from Shanghai Cell Bank of Chinese Academy of Sciences, subcultured with routine method according to different characteristics of different tumor cells at 37 °C in an incubator with 5% CO₂. When the cells reached a fusion of 90%, cells were digested with 0.25% trypsin solution, collected into a serum-free culture medium, gently shaked, centrifuged at 500g for 5min, washed once, resuspended with PBS, adjusted to a concentration of 10⁶, stained with trypan, and counted with a blood counting plate. The proportion of living cells was more than 95%. According to the requirements of experiment, solid tumors were inoculated in forelimb armpit with 10⁵-10⁶ cells per mouse.

Evaluation of apparent effect. After about 7 days of tumor inoculation (different types of tumor differed slightly), the length and width of the tumor were measured every other day with a caliper. Tumor volume was calculated using the formula: tumor volume = width² x length x π/2. When the tumor volume was greater than 2000mm³ or 3000mm³ (different types of cancer differed slightly), the mice were judged as die for theoretical. The survival was counted, and survival curve was prepared. Mice were killed 20-30 days after tumor inoculation and the tumors were weighted. Inhibition rate was calculated using the formula: Inhibition rate = (mean tumor weight of control group - mean tumor weight of experiment group)/mean tumor weight of control group x 100%. The body weight of mice was measured with electronic balance and recorded before the mice were grouped and killed and the ratio of tumor weight to body weight was calculated.

### Example 1 Recombinant protein vaccine targeting TNF-α

Drugs of monoclonal antibody of anti-TNF-α have made great success in the field of treatment of rheumatoid arthritis in clinical, but antibody drugs must be used in large doses and frequently. High medical cost has hindered a wide use range and could not benefit more patients. In this example, a recombinant protein vaccine targeting TNF-α was developed for prevention and treatment of rheumatoid arthritis.

### Step 1 Design of antigen structure

Antigens were constructed by transplanting TNF epitope sequences in the table to the replaceable positions of DTT, and they were named as mTNF28, mTNF31, mTNF37, mTNF25, DTT-mTNFt, DTT-hTNFt, respectively. The tyrosine Y at position 87 of TNF was mutated to histidine H and the alanine A at position 145 was mutated to arginine R to get mutants of mTNFt and hTNFt ("t" referred site-directed mutagenesis) having a very low biological activity. Recombinant antigens of DTT-mTNFt and DTT-mTNFt were constructed by transplanting mTNFt and hTNFt to the C-terminal of DTT. Information of transplantation structure of other recombinant antigen used in this example was shown in Table 2.

**Table 2 TNF recombinant antigen design**

| Antigen Name | Position on mTNF | Sequence of mTNF epitope | Position replaced on DTT |
|---|---|---|---|
| mTNF28 | 80-96 | VSRFAISYQEKVNLLSA (SEQ ID NO.:5) | 295-297 |
| mTNF31 | 142-150 | LDFAESGQV (SEQ ID NO.:6) | 305-310 |
| mTNF37 | 28-35 | WLNRRANA (SEQ ID NO.:7) | 291-297 |
| mTNF25 | 40-50 | GMDLKDNQLVV (SEQ ID NO.:8) | 295-297 |
| DTT-mTNFt | Entire mTNF (A145R/Y87H) | | C-terminal of DTT |
| DTT-hTNFt | Entire hTNF (A145R/Y87H) | | C-terminal of DTT |

Amino acid sequence of antigen protein DTT-mTNFt (A145R/Y87H):

INLDWDVIRDKTKTKIESLKEHGPIKNKMSESPNKTVSEEKAKQYL
EEFHQTALEHPELSELKTVTGTNPVFAGANYAAWAVNVAQVIDSETAD
NLEKTTAALSILPGIGSVMGIADGAVHHNTEEIVAQSIALSSLMVAQAIP
LVGELVDIGFAAYNFVESIINLFQVVHNSYNRPMLRSSSQNSSDKPVAH
VVANHQVEEQLEWLSQRANALLANGMDLKDNQLVVPADGLYLVYSQ
VLFKGQGCPDYVLLTHTVSRFAISHQEKVNLLSAVKSPCPKDTPEGAEL
KPWYEPIYLGGVFQLEKGDQLSAEVNLPKYLDFRESGQVYFGVIAL
(SEQ ID NO.:9)

Amino acid sequence of mTNFt (A145R/Y87H):

MLRSSSQNSSDKPVAHVVANHQVEEQLEWLSQRANALLANGMDLKDNQL
VVPADGLYLVYSQVLFKGQGCPDYVLLTHTVSRFAISHQEKVNLLSAVKSPCPK
DTPEGAELKPWYEPIYLGGVFQLEKGDQLSAEVNLPKYLDFRESGQVYFGVIAL
(SEQ ID NO.:10)

Amino acid sequence of antigen protein DTT- hTNFt (A145R/Y87H):

INLDWDVIRDKTKTKIESLKEHGPIKNKMSESPNKTVSEEKAKQYLEEFHQT

ALEHPELSELKTVTGTNPVFAGANYAAWAVNVAQVIDSETADNLEKTTAALSILP
GIGSVMGIADGAVHHNTEEIVAQSIALSSLMVAQAIPLVGELVDIGFAAYNFVESII
NLFQVVHNSYNRPMVRSSSRTPSDKPVAHVVANPQAEGQLQWLNRRANALLAN
GVELRDNQLVVPSEGLYLIYSQVLFKGQGCPSTHVLLTHTISRIAVSHQTKVNLLS
AIKSPCQRETPEGAEAKPWYEPIYLGGVFQLEKGDRLSAEINRPDYLDFRESGQV
YFGIIAL (SEQ ID NO.:11)

Amino acid sequence of hTNFt (A145R/Y87H):

MVRSSSRTPSDKPVAHVVANPQAEGQLQWLNRRANALLANGVEL
RDNQLVVPSEGLYLIYSQVLFKGQGCPSTHVLLTHTISRIAVSHQTKVN
LLSAIKSPCQRETPEGAEAKPWYEPIYLGGVFQLEKGDRLSAEINRPDYL
DFRESGQVYFGIIAL (SEQ ID NO.:12)

Amino acid sequence of antigen protein mTNF31:

INLDWDVIRDKTKTKIESLKEHGPIKNKMSESPNKTVSEEKAKQYL
EEFHQTALEHPELSELKTVTGTNPVFAGANYAAWAVNVAQVIDSETAD
NLEKTTAALLDFAESGQVGSVMGIADGAVHHNTEEIVAQSIALSSLMV
AQAIPLVGELVDIGFAAYNFVESIINLFQVVHNSYNRP (SEQ ID NO.:13)

Amino acid sequence of antigen protein mTNF28 (SEQ ID NO.:112)

INLDWDVIRDKTKTKIESLKEHGPIKNKMSESPNKTVSEEKAKQYL
EEFHQTALEHPELSELKTVTGTNPVFAGANYAAWAVNVAQVIDSETAV
SRFAISYQEKVNLLSAEKTTAALSILPGIGSVMGIADGAVHHNTEEIVAQ
SIALSSLMVAQAIPLVGELVDIGFAAYNFVESIINLFQVVHNSYNRP

Nucleotide acid sequence of antigen protein mTNF28 was shown in SEQ ID NO.:121.

### Step 2 Expression and purification of recombinant protein and immunogenicity observation

Expression plasmids were constructed by using primers of TNF recombinant antigen gene shown in Table 3 according to Method 2, and the correctness was proven by sequencing.

Gene sequence of antigen protein DTT- mTNFt (A145R/Y87H) was shown in SEQ ID NO.:14.

Gene sequence of antigen protein DTT- hTNFt (A145R/Y87H) was shown in SEQ ID NO.:15.

Gene sequence of antigen protein mTNF32 was shown in SEQ ID NO.:16.

Gene sequence of antigen protein mTNF31 was shown in SEQ ID NO.:17.

**Table 3 Primers of TNF recombinant antigen gene**

| Antigen Name | Primer Name | Sequence |
|---|---|---|
| DTT3 | DTT-F | CGCGGATCCCTGGAAGTTCTGTTCCAGGGGCCCATAAATCTTGATTGGGATGTC (SEQ ID NO.:18) |
| | DTT-R | CCGCTCGAGCTAGGGACGATTATACGAATTATG (SEQ ID NO.:19) |
| mTNF28 | mT80-96 half down forward-P2 | GAAAAGACAACTGCTGCTC (SEQ ID NO.:20) |
| | mT80-96 down forward-P4 | TCAACCTCCTCTCTGCCGAAAAGACAACTGCTGCTC (SEQ ID NO.:21) |
| | mT80-96 up reverse-P3 | TCCTGGTATGAGATAGCAAATCGGCTGACAGCTGTTTCGCTATCGATAACTT (SEQ ID NO.:22) |
| | mT80-96 half up reverse-P1 | AGCTGTTTCGCTATCGATAACTTGC (SEQ ID NO.:23) |
| mTNF31 | mT142-150 half down forward-P2 | GGTAGCGTAATGGGCATTGCAGAC (SEQ ID NO.:24) |
| | mT142-150 down forward-P4 | TTAGACTTTGCGGAGTCCGGGCAGGTCGGTAGCGTAATGGGCATTGCAG (SEQ ID NO.:25) |
| | mT142-150 up reverse-P3 | GACCTGCCCGGACTCCGCAAAGTCTAAAAGAGCAGCAGTTGTCTTTTCCAAATT (SEQ ID NO.:26) |
| | mT142-150 half up reverse-P1 | AAGAGCAGCAGTTGTCTTTTCC (SEQ ID NO.:27) |
| mTNF37 | mT28-35 half down forward-P2 | GAAAAGACAACTGCTGCTCTTTCGATACT (SEQ ID NO.:28) |
| | mT28-35 down forward-P4 | TGGCTGAGCCAGCGCGCCAACGCCGAAAAGACAACTGCTGCTCTTTCGATACT (SEQ ID NO.:29) |
| | mT28-35 up reverse-P3 | GGCGTTGGCGCGCTGGCTCAGCCAATCGATAACTTGCGCAACGTTT (SEQ ID NO.:30) |
| | mT28-35 half up reverse-P1 | ATCGATAACTTGCGCAACGT (SEQ ID NO.:31) |
| mTNF25 | half up reverse-P1 | CACTAGTTGGTTGTCTTTGAGATCCATGCCAGCTGTTTCGCTATCG (SEQ ID NO.:32) |
| | half down forward-P2 | GGATCTCAAAGACAACCAACTAGTGGTGGAAAAGACAACTGCTGCTC (SEQ ID NO.:33) |

Expression and purification of recombinant protein antigens were operated in accordance with Method 3. The recombinant antigens were prepared with a purity of 90% -95%. Mice were immunized according to Method 4 and mTNF was used as a coating antigen. Final results of ELISA showed that when the antiserum was diluted 100 times, mean OD450 of TNF28 to mTNF was 0.33, mean OD450 of mTNF31 to mTNF was 0.09, and mean OD450 of mTNF37 to mTNF was 0.11, while the mean OD450 of Alum to mTNF was 0.05. When compared to the control group of Alum, antibody against mTNF induced by TNF28 was better than those induced by mTNF31 and mTNF37. After immunization, results of serum western showed that compared with DTT group (serum from mice immunized with DTT only), TNF28 group had significant antibody bands, and reaction signal from human TNF strip was stronger than that from mice TNF strip (Fig. 2A).

### Step 3 Biological activity assay after protein mutation

L292 cells in logarithmic growth phase (culture 2d in a 25cm² flask) were washed twice with 1xPBS after pouring the culture medium. After absorbing off 1 x PBS, 0.7mL digestion solution was added, digestion was at 37 °C for 3min. It was centrifuged at 1000rpm/min for 4min, and the digestion solution was absorbed. Cells were washed with 5mL 1640 to remove trypsin. The cell concentration was adjusted to 2x10⁵/ml with 1640 medium. L929 cell suspension was added to a 96-well cell culture plate with 0.1ml per well and cultured in a CO₂ incubator overnight or 24 hours until the cells covered 95% or more of plate wall, and then the sample were loaded. TNF to be tested was diluted by AcD-1640 medium in the 10-fold serial dilution according to the situation, and 100uL of dilution was added into each well, and there were three duplicate wells for each dilution. The cells were cultured in a CO₂ incubator for 20h, 20uL MTT (Beyotime) were directly added, and incubated at 37 °C for 4h. OD value of each well was detected at 450nm using a microplate reader (reference wavelength was 600-650nm). The killing rate was calculated by the formula: (OD₄₅₀ of control group - OD₄₅₀ of experiment group)/OD₄₅₀ of control group x 100%. The maximum killing concentration decreased from 0.2ng/ml to more than 100,000 (the maximum concentration had been done), and after site-directed mutagenesis, activity of recombinant protein dropped by more than 100,000 folds (Figure 2C).

### Step 4 Observation of Collagen-induced arthritis model of mice

Collagen-induced arthritis model of mice was constructed according to the steps in Method 5. As for target antigens of mTNF28, mTNF31, and mTNF37, when compared to the control group of Alum, the onset scores of the group using a transplanted epitope protein of mTNF28 in animal models of mice arthritis were lowered by 3-4 points during the period from 60 to 120 days. mTNF31 group and mTNF37 group only had a difference of 1-2 points. mTNF28 exhibited a superior effect of treatment compared with mTNF37 and mTNF31 (Figure 2B). As shown in FIG.2D, compared to the control group of Alum, the effect of site-directed mutagenesis fusion protein DTT-mTNFt in animal models of mice arthritis was that the onset score was lowered by 5-6 points during the period from 20 to 32 days. Compared with the control group of Alum, DTT-mTNFt had an obvious effect of disease inhibition based on the onset score.

### Example 2 Optimization of TNF80-96 epitope vaccine

### Step 1 antigen design

It could be seen from Example 1 that the effect of mTNF28 having TNF80-96 epitope was better. In the example, the epitope was improved. The same method as that in Example 1 was used except using protein epitopes and the replaced position as shown in the following table, thereby constructing a series of recombinant proteins including mTNF21, mTNF26, mTNF28, mTNF30, mTNF32, mTNF28-1, and mTNF29.

**Table 4 Epitomes and replaced position**

| Name | Position on mTNF | Replaced position of DT | Epitope sequence |
|---|---|---|---|
| mTNF21 | 80-96 | 296-297 | VSRFAISYQEKVNLLSA |
| mTNF26 | 80-96 | 293-297 | VSRFAISYQEKVNLLSA. |
| mTNF28 | 80-96 | 295-297 | VSRFAISYQEKVNLLSA |
| mTNF30 | 80-96 | 292-297 | VSRFAISYQEKVNLLSA |
| mTNF32 | 80-96 | 291-297 | VSRFAISYQEKVNLLSA |
| mTNF28-1 | 80-97 | 290-297 | VSRFAISYQEKVNLLSAV (SEQ ID NO.:34) |
| mTNF29 | 80-96 | C-terminal | VSRFAISYQEKVNLLSA |

Amino acid sequence of position 90-96 of hTNF:
ISRIAVSYQTKVNLLSA (SEQ ID NO.:117)

Amino acid sequence of position 90-97 hTNF:
ISRIAVSYQTKVNLLSAI (SEQ ID NO.: 118)

Amino acid sequence of antigen protein hTNF28-1:

INLDWDVIRDKTKTKIESLKEHGPIKNKMSESPNKTVSEEKAKQYLEEFHQT
ALEHPELSELKTVTGTNPVFAGANYAAWAVNVAQVIISRIAVSYQTKVNLLSAIEK
TTAALSILPGIGSVMGIADGAVHHNTEEIVAQSIALSSLMVAQAIPLVGELVDIGFA
AYNFVESIINLFQVVHNSYNRP (SEQ ID NO.:35)

Gene sequence of antigen protein hTNF28-1 was shown in SEQ ID NO.:36.

Amino acid sequence of antigen protein mTNF28-1:

INLDWDVIRDKTKTKIESLKEHGPIKNKMSESPNKTVSEEKAKQYLEEFHQT
ALEHPELSELKTVTGTNPVFAGANYAAWAVNVAQVIVSRFAISYQEKVNLLSAVE
KTTAALSILPGIGSVMGIADGAVHHNTEEIVAQSIALSSLMVAQAIPLVGELVDIGF
AAYNFVESIINLFQVVHNSYNRP (SEQ ID NO.:37)

Gene sequence of antigen protein mTNF28-1 was shown in SEQ ID NO.:38.

Amino acid sequence of antigen protein mTNF32:

INLDWDVIRDKTKTKIESLKEHGPIKNKMSESPNKTVSEEKAKQYLEEFHQT
ALEHPELSELKTVTGTNPVFAGANYAAWAVNVAQVIDVSRFAISYQEKVNLLSAE
KTTAALSILPGIGSVMGIADGAVHHNTEEIVAQSIALSSLMVAQAIPLVGELVDIGF

AAYNFVESIINLFQVVHNSYNRP (SEQ ID NO.:39)

### Step 2 Expression and purification of recombinant protein and immunogenicity observation

Construction, expression and purification method for specific recombinant protein antigen were described in Method 2 and Method 3. A series of recombinant proteins was prepared with a purity more than 95%, and placed at 4 °C for 2 days and 10 days. As shown by SDS-PAGE electrophoresis results, the structural stability of mTNF28-1 was far superior to other proteins. Comparing circular dichroisms of mTNF28-1 and DTT, the results showed that the two proteins had a consistent structure, and core framework of protein was not changed. For the series of proteins obtained from the optimiztion of the 80-96 epitope, Tm value was measured by DSC. Tm value of mTNF28-1 (Tm=66.58) and DTT (Tm=69.85) were similar, and were much higher than Tm value of mTNF28 (Tm = 61.01). Although there was only one amino acid difference between mTNF28-1 and mTNF28 protein, mTNF28-1 showed a higher stability in respect of structural stability.

Titers of serum produced after antigen immunization were measured by the Method 4 wherein mTNF and DTT were used as coating antigen separately. After the third booster immunization, percentage of mice that could produce a response to mTNF28-1 was 80%. Mean OD₄₅₀ of antiserum of mTNF protein after 100-fold dilution of titer was 0.28, and for mTNF32 they were 50% and 0.22, and for mTNF21 and mTNF28 they were 25% and 0.17, and 25% and 0.15, respectively. The mTNF28-1 had the highest antigenicity. The results of immunization showed that mTNF28-1 showed better immune effect over other transplantation.

### Step 3 Observation of Collagen-induced arthritis model of mice

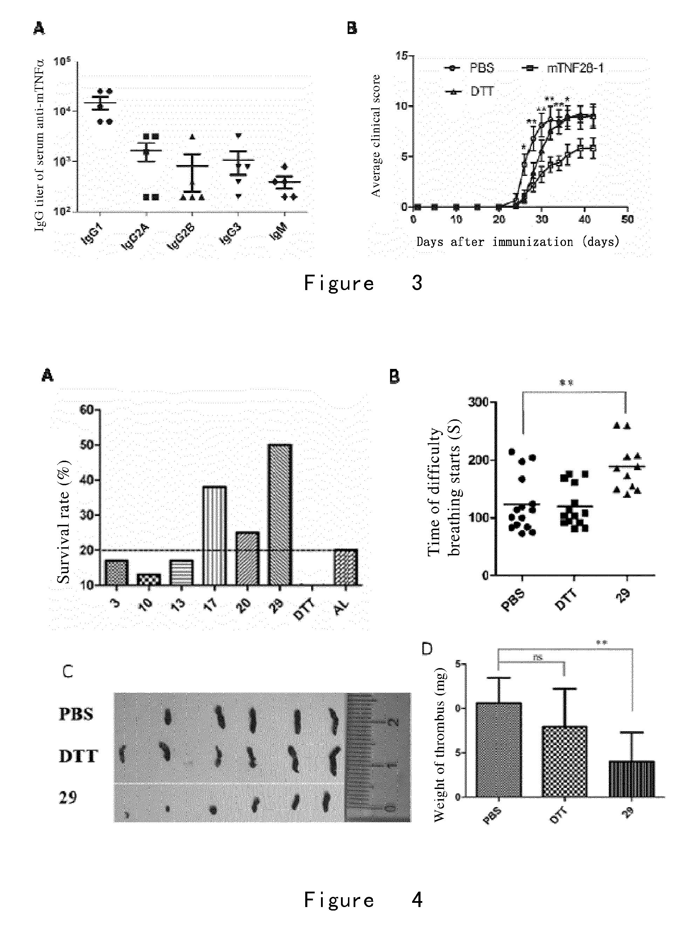

After 60 days of 4th booster immunization, antibody typing was implemented using mice blood, and the results were shown in Figure 3A. The results showed that 60 days after the booster immunization, the main type of antibody against mTNF in mice was IgG1, and the titer was still 10,000. It suggested that the recombinant proteins of the present invention caused an immune response which could maintain for a long time in the body. The therapeutic effect of mTNF28-1 in collagen-induced mice arthritis model was measured with according to Method 4. As shown in Figure 3B, during the period from 55 to 80 days, the inhibition rate to arthritis in mice of treatment group of mTNF28-1 was more than 40% compared to the group of Alum adjuvant and group of DTT carrier protein.

### Example 3 Antithrombotic vaccine targeting clotting factor FXI

FXI is a clotting factor involved in endogenous coagulation pathway. Recent literatures and pathological statistics supported that FXI deletion could help the body against thrombosis, but caused only minor bleeding of body. Therefore, FXI is considered as a safe and effective antithrombotic target. In this example, an antithrombotic vaccine against the clotting factor FXI was constructed, and the immunogenicity and antithrombotic effect were tested.

### Step 1: Recombinant antigens design

Antigens used in the present example were constructed by transplanting the candidate epitope sequence of human FXI to a replaceable position on DTT. Antigen No. 17 was constructed by transplanting amino acid sequence of TNEECQKRYRGHKITH (SEQ ID NO.: 40) of human FXI (523-538aa) to replace position 291 to 297 of DTT. Antigen No.20 was constructed by transplanting amino acid sequence TTKIKPRIVGGTASVRGE (SEQ ID NO.: 41) of human FXI (363-380aa) to replace position 291 to 297 of DTT. Antigen No.29 was constructed by transplanting a catalytic domain of human FXI (381-625aa) to the C-terminal of DTT.

Specific amino acid sequences of antigens were as follows:

Amino acid sequence of Antigen No.17:

INLDWDVIRDKTKTKIESLKEHGPIKNKMSESPNKTVSEEKAKQYLEEFHQT
ALEHPELSELKTVTGTNPVFAGANYAAWAVNVAQVIDTNEECQKRYRGHKITHE
KTTAALSILPGIGSVMGIADGAVHHNTEEIVAQSIALSSLMVAQAIPLVGELVDIGF
AAYNFVESIINLFQVVHNSYNRP (SEQ ID NO.:42)

Amino acid sequence of Antigen No.20:

INLDWDVIRDKTKTKIESLKEHGPIKNKMSESPNKTVSEEKAKQYLEEFHQT
ALEHPELSELKTVTGTNPVFAGANYAAWAVNVAQVIDTTKIKPRIVGGTASVRGE
EKTTAALSILPGIGSVMGIADGAVHHNTEEIVAQSIALSSLMVAQAIPLVGELVDIGF
AAYNFVESIINLFQVVHNSYNRP (SEQ ID NO.:43)

Amino acid sequence of Antigen No.29:

INLDWDVIRDKTKTKIESLKEHGPIKNKMSESPNKTVSEEKAKQYLEEFHQT
ALEHPELSELKTVTGTNPVFAGANYAAWAVNVAQVIDSETADNLEKTTAALSILP
GIGSVMGIADGAVHHNTEEIVAQSIALSSLMVAQAIPLVGELVDIGFAAYNFVESII
NLFQVVHNSYNRPTTKIKPRIVGGTASVRGEWPWQVTLHTTSPTQRHLCGGSIIG
NQWILTAAHCFYGVESPKILRVYSGILNQSEIKEDTSFFGVQEIIIHDQYKMAESGY
DIALLKLETTVNYTDSQRPICLPSKGDRNVIYTDCWVTGWGYRKLRDKIQNTLQ
KAKIPLVTNEECQKRYRGHKITHKMICAGYREGGKDACKGDSGGPLSCKHNEV
WHLVGITSWGEGCAQRERPGVYTNVVEYVDWILEKTQAV (SEQ ID NO.:44)

Amino acid sequence of the catalytic domain of human FXI (381-625aa)

TTKIKPRIVGGTASVRGEWPWQVTLHTTSPTQRHLCGGSIIGNQWILTAAHC
FYGVESPKILRVYSGILNQSEIKEDTSFFGVQEIIIHDQYKMAESGYDIALLKLETT
VNYTDSQRPICLPSKGDRNVIYTDCWVTGWGYRKLRDKIQNTLQKAKIPLVTNE
ECQKRYRGHKITHKMICAGYREGGKDACKGDSGGPLSCKHNEVWHLVGITSWG
EGCAQRERPGVYTNVVEYVDWILEKTQAV (SEQ ID NO.:45)

Gene sequence of antigen protein No. 17 was shown in SEQ ID NO.:46.

Gene sequence of antigen protein No.20 was shown in SEQ ID NO.:47.

Gene sequence of antigen protein No.29 was shown in SEQ ID NO.:48.

### Step 2: Construction of recombinant antigen vector, expression and identification and large-scale preparation of the protein

Gene of the recombinant antigen was constructed according to Method 2. Recombinant protein was expressed, identified and prepared according to Method 3. As determined by 12% SDS-PAGE detection, molecular weight of recombinant antigen No.29 was about 45KD, and molecular weight of recombinant antigen No.17 and No.20 were about 20KD. They were all consistent with the theoretical molecular weight. The purity of antigens was more than 90%.

### Step 3: Animal immunization and antibody titer detection

Immunization of mice and detection of antibody reaction were conducted according to Method 4, wherein the mice in experiment were female C57BL/6J (purchased from Shanghai Slaccas Co.), the immunizing dose was 30ng/mice, and there were eight mice per group. One week after the second booster immunization, blood was collected to prepare serum, and the antibody reaction against the FXI was detected by ELISA.

Using human FXI as an ELISA coating antigen, antiserum from the mice immunized by antigens of No.17, No.20, And No.29 was diluted by 1: 100 and the ELISA results of OD₄₅₀ values were 0.52 ± 0.11, 0.43 ± 0.08, 2.52 ± 0.3 (the value of negative serum was 0.14 ± 0.03). It showed that antigens of No. 17, No.20, No.29 could stimulate mice to produce antibodies that could recognize human FXI.

Using mice FXI as an ELISA coating antigen, antiserum from the mice immunized by antigens of No.29 was diluted by 1: 100, and the ELISA results OD₄₅₀ value was 1.87 ± 0.5 (the value of negative serum was 0.14 ± 0.03). It showed that the antigen of No.29 could stimulate mice to produce antibodies that could recognize mice FXI by cross reaction.

### Step 4: Effect of immunization on clotting time in mice

Immunization of mice and detection of antibody reaction were conducted according to Method 4, wherein the mice in experiment were female C57BL/6J (purchased from Shanghai Slaccas Co.), the immunizing dose was 30ng/mice, and there were five mice per group. 14 days after the second booster immunization, mice were narcotized (100mg/kg, by intraperitoneal injection) with sodium pentobarbital anesthesia. Incision was along the midline of abdomen, and postcava was exposed after turning over viscera. Blood was extracted in a volume of 450ul with a syringe containing 50ul 3.2% sodium citrate, and the syringe was gently turned to mix the blood and anticoagulants. All samples were centrifuged at room temperature at 3000g for 15min to prepare platelet-free plasma. APTT value and PT value were respectively measured within 4h using activated partial thromboplastin time assay kit (ellagic acid) (coagulation method) and prothrombin time assay kit (coagulation method) (Shanghai Sun Biotechnology Co., Ltd.) at a thromboscreen 400c semi-automatic coagulation analyzer (Pacific hemostasis).

APTT values of mice immunized by PBS, DTT, No.17 antigen, No.29 antigen were 25.2 ± 1.8s, 24.5 ± 2.1s, 26.7 ± 1.8s, 30.3 ± 2.5s, respectively. APTT value of mice immunized by PBS was similar with wild-type mice (APTT value of wild-type mice were 25.1s), indicating that PBS did not interfere with the coagulation function of endogenous pathway. APTT value of mice immunized by No.29 antigen was extended 1.2 times with respect to the value of PBS group (p <0.05, t-test), indicating that the antigen could inhibit the function of endogenous coagulation pathway in which FXI was involved.

PT values of mice immunized by PBS, DTT, No.17 antigen, No.29 antigen were 10.4 ± 0.2s, 10.6 ± 0.1 s, 10.2 ± 0.2s, 11.2 ± 0.1 s, respectively. PT value of mice immunized by No. 17 antigen was not significantly extended, and PT value of mice immunized by No.29 antigen only slightly extended (no clinical significance), indicating that the designed recombinant antigen did not interfere with the normal function of exogenous coagulation pathway.

### Step 5: Prevention of antigen to pulmonary embolism thrombosis

Immunization of mice and detection of antibody reaction were conducted according to Method 4, wherein the mice in experiment were female C57BL/6J (purchased from Shanghai Slaccas Co.), the immunizing dose was 30ng/mice, and there were eight mice per group. 14 days after the second booster immunization, the antithrombotic effect in mice of each group was evaluated by lethal pulmonary embolism model. Specific steps were as follows: thromborel S (Siemens) was redissolved with 10ml of distilled water according to the instruction. Mice were anesthetized with sodium pentobarbital with 50mg/kg by intraperitoneal injection, and then postcava was exposed and isolated. The redissolved thromborel S was injected into postcava at 7.5ul/g of body weight within 3s. Timing at once, the breathing of animals were observed, and animals without stopping breathing for 20 minutes were considered survival.

As shown in Figure 4A, the survival rates of mice immunized with PBS, DTT, No.17 antigen, No.20 antigen, No.29 antigen were 20%, 0%, 37.5%, 25%, 50% at 20th minute, indicating that No.17 antigen, and No.29 antigen could help mice against pulmonary embolism.

Immunization of mice and detection of antibody reaction were conducted according to Method 4, wherein the mice in experiment were female C57BL/6J (purchased from Shanghai Slaccas Co.), the immunizing dose was 30ng/mice, and there were 14 mice per group. 14 days after the second booster immunization, the antithrombotic effect in mice of No.29 antigen was evaluated by lethal pulmonary embolism model. Mice were anesthetized with sodium pentobarbital with 50mg/kg by intraperitoneal injection, and then postcava was exposed and isolated. A dose of human placenta extract of 30ug/g body weight was injected into postcava, and the start time appearing dyspnea of mice in each group was recorded.

As shown in Figure 4B, the time appearing dyspnea in mice immunized with PBS, DTT, and No.29 antigen was 121 ± 21 s, 118 ± 35s, and 182 ± 33s. The time appearing dyspnea in mice immunized with No.29 antigen was extended 1.5 times (p <0.01, t-test), indicating that the No.29 antigen could help a mouse against pulmonary embolism.

### Step 6: Prevention of antigens to thrombosis of postcava stenosis

Immunization of mice and detection of antibody reaction were conducted according to Method 4, wherein the mice in experiment was female C57BL/6J (purchased from Shanghai Slaccas Co.), the immunizing dose was 30ng/mice, and there were six mice per group. 14 days after the second booster immunization, the antithrombotic effect in mice of No.29 antigen was evaluated by postcava stenosis thrombus model. Specific steps were as follows: mice were weighed, and anesthetized with sodium pentobarbital with 100mg/kg body weight. The abdomen was shaved and disinfected with iodine. Abdomen was opened carefully with ophthalmic scissors, and viscera were turned over and binded up with gauze soaked saline to expose the postcava. Postcava and aortaventralis were separated below a tributary of the left renal vein, ligatured to a 30g needle with 6-0 silk (Shanghai Jinhuan Co.). The needle was removed to cause the vein stenosis. Postcava and aortaventralis were separated at the part of waist tributary. The vessels below ligature and at the part of waist tributary were clamped by two vascular clamp for 20s respectively. And the operating time was record. The intestines were replaced into abdominal cavity orderly, and muscle and skin were successively sutured with 5-0 nylon thread (Shanghai Jinhuan Co.), disinfected with povidone-iodine and mice were put back to clean cage. Emboli were removed after 24h, rinsed with saline, fixed with 4% paraformaldehyde for more than 24h, photographed, and weighed for wet weight after dried with a filter paper.

As shown in Figure 4C and 4D, the weights of emboli of mice immunized with PBS, DTT, No.29 antigen were 11.2 ± 2.1mg, 8.2 ± 4.5mg, 4.5 ± 2.3mg, respectively. The weight of emboli of mice immunized with No.29 antigen decreased 60% compared to the weight of emboli of mice in the control group, indicating that the No.29 antigen could help mouse to prevent thrombosis of postcava stenosis.

### Step 7 Effect of antibody to APTT value and PT value of human plasma

Antibodies in plasma of mice immunized with each antigen were purified with protein A-sepharose medium, quantitated with BCA quantification kit, diluted with 10mM PBS (pH7.4) and mixed with human plasma in a volume ratio of 1:1 to detect APTT values, PT values and the activity of FXI.

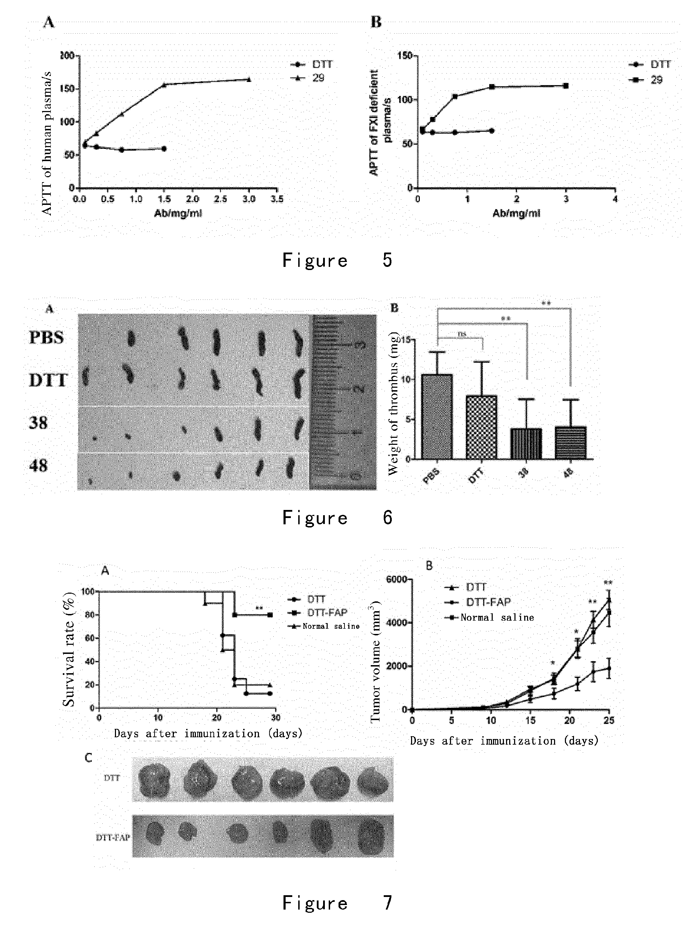

Antibody produced by mice stimulated with No.29 antigen could significantly prolong APTT values of normal human plasma, and the inhibitory effect had a clear dose-effect relationship (as shown in Figure 5A). The APTT value of normal human plasma was extend to 151s by the antibody with a final concentration of 0.75mg/ml, and the value of control group was 54s. Antibody produced by mice stimulated with No.29 antigen could significantly inhibit the activity of human FXI, and the inhibitory effect had a clear dose-effect relationship (as shown in Figure 5B). After mixing the antibody (in a final concentration of 0.75mg/ml) with normal human plasma, the mixture was added to the human plasma lacking of FXI. The APTT value of the human plasma was extended to 121 s, while the value of control group was 63s.

After incubating antibodies produced by mice stimulated by No.29 antigen or DTT with normal human plasma, PT values measured was 41s or 42s. The results showed that antibodies produced by mice stimulated by No.29 antigen did not interfere with human exogenous coagulation pathway required for normal hemostasis.

### Step 8 Safety detection of antigen

No.29 antigen did not affect the function of exogenous coagulation pathway in mice as shown in PT test, suggesting that this antigen has no side effect of bleeding. To further illustrate the safety of antigen, in the present invention, the skin and internal organs of mice were examined for bleeding. It was found that there was no abnormal bleeding on each viscera (liver, spleen, lung and kidney stomach, etc.) of the mice immunized by DTT or No.29 antigen through anatomical comparison. Furthermore, the shape and color of liver were checked. Macroscopic observation showed no difference in shape and color of liver between mice immunized by DTT and mice immunized by No. 29.

### Example 4 Antithrombotic vaccine targeting clotting factor FXII

FXII is a clotting factor involved in endogenous coagulation pathway. Recent literatures and pathological statistics supported that FXII deletion could help the body against thrombosis, but did not cause abnormal bleeding of body. Therefore, FXII is considered as a safe and effective antithrombotic target. In this example, an antithrombotic vaccine against clotting factor FXII was constructed, and the immunogenicity and antithrombotic effect were tested.

### Step 1: Recombinant antigens design

Antigens used in the present example were constructed by transplanting the candidate epitope sequence of human FXII to a replaceable position on DTT. Antigen No.38 was constructed by transplanting amino acid sequence EAFSPVSYQHDLA (SEQ ID NO.:128) of human FXII (79-91) to replace the position 295 to 297 of DTT. Antigen No.48 was constructed by transplanting a catalytic domain of human FXII (345-596) to the C-terminal of DTT.
79-91 amino acids of mFXII
EGFSSITYQHDLA (SEQ ID NO.:131)

Catalytic domain of hFXII (345-596)

RKSLSSMTRVVGGLVALRGAHPYIAALYWGHSFCAGSLIAPCWVL
TAAHCLQDRPAPEDLTVVLGQERRNHSCEPCQTLAVRSYRLHEAFSPV
SYQHDLALLRLQEDADGSCALLSPYVQPVCLPSGAARPSETTLCQVAG
WGHQFEGAEEYASFLQEAQVPFLSLERCSAPDVHGSSILPGMLCAGFLE
GGTDACQGDSGGPLVCEDQAAERRLTLQGIISWGSGCGDRNKPGVYTD
VAYYLAWIREHTVS (SEQ ID NO.:129)

Catalytic domain of mFXII (345-596)

RKGLSSFMRVVGGLVALPGSHPYIAALYWGNNFCAGSLIAPCWVL
TAAHCLQNRPAPEELTVVLGQDRHNQSCEWCQTLAVRSYRLHEGFSSI
TYQHDLALLRLQESKTNSCAILSPHVQPVCLPSGAAPPSETVLCEVAGW
GHQLEGAEEYSTFLQEAQVPFIALDRCSNSNVHGDAILPGMLCAGFLEG
GTDACQGDSGGPLVCEEGTAEHQLTLRGVISWGSGCGDRNKPGVYTD
VANYLAWIQKHIAS (SEQ ID NO.:130)

Amino acid sequence of Antigen No.38:

INLDWDVIRDKTKTKIESLKEHGPIKNKMSESPNKTVSEEKAKQYL
EEFHQTALEHPELSELKTVTGTNPVFAGANYAAWAVNVAQVIDSETAE
AFSPVSYQHDLAEKTTAALSILPGIGSVMGIADGAVHHNTEEIVAQSIAL
SSLMVAQAIPLVGELVDIGFAAYNFVESIINLFQVVHNSYNRP (SEQ ID
NO.:113)

Amino acid sequence of Antigen No.48:

INLDWDVIRDKTKTKIESLKEHGPIKNKMSESPNKTVSEEKAKQYL
EEFHQTALEHPELSELKTVTGTNPVFAGANYAAWAVNVAQVIDSETAD

NLEKTTAALSILPGIGSVMGIADGAVHHNTEEIVAQSIALSSLMVAQAIP
LVGELVDIGFAAYNFVESIINLFQVVHNSYNRPRKSLSSMTRVVGGLVA
LRGAHPYIAALYWGHSFCAGSLIAPCWVLTAAHCLQDRPAPEDLTVVL
GQERRNHSCEPCQTLAVRSYRLHEAFSPVSYQHDLALLRLQEDADGSC
ALLSPYVQPVCLPSGAARPSETTLCQVAGWGHQFEGAEEYASFLQEAQ
VPFLSLERCSAPDVHGSSILPGMLCAGFLEGGTDACQGDSGGPLVCEDQ
AAERRLTLQGIISWGSGCGDRNKPGVYTDVAYYLAWIREHTVS (SEQ
ID NO.:114)

Nucleotide sequence encoding antigen protein No.38 was shown in SEQ ID NO.:115.

Nucleotide sequence encoding antigen protein No.48 was shown in SEQ ID NO.:116.

### Step 2: Construction of recombinant antigen vector, expression and identification and large-scale preparation of the protein

Gene of the recombinant antigen was constructed according to Method 2. Recombinant protein was expressed, identified and prepared according to Method 3. As detected by 12% SDS-PAGE, molecular weight of recombinant antigen No.48 was about 45KD, and molecular weight of recombinant antigen No.38 was about 20KD. They were all consistent with the theoretical molecular weight and purity of antigens was more than 90%.

### Step 3: Animal immunization and antibody titer detection

Immunization of mice and detection of antibody reaction were conducted according to Method 4, wherein the mice in experiment was female C57BL/6J (purchased from Shanghai Slaccas Co.), the immunizing dose was 30ng/mice, and there were eight mice per group. One week after the second booster immunization, blood was collected to prepare serum, and the antibody reaction against the FXII was detected by ELISA.

Using human FXII as an ELISA coating antigen, antiserum from the mice immunized by antigens of NO.38, NO.48 was diluted by 1: 100 and the ELISA results of OD₄₅₀ values were 0.38±0.07, 1.32±0.51 (the value of negative serum was 0.11±0.03). It showed that antigens of NO. 38 and NO. 48, could stimulate mice to produce antibodies that could recognize human FXII.

Using mice FXII as an ELISA coating antigen, antiserum from the mice immunized by antigens of NO.38, NO.48 was diluted by 1: 100, and the OD₄₅₀ values were 0.11±0.04, 1.75±0.21 (the value of negative serum was 0.12±0.03). It showed that the antigen of NO.48 could stimulate mice to produce antibodies that could recognize mice FXII by cross reaction.

### Step 4: Effect of immunization to clotting time in mice

Immunization of mice and detection of antibody reaction were conducted according to Method 4. Preparation of platelet-free plasma and measurement of APTT value and PT value were according to Step 4 in Example 3.

APTT values of mice immunized by PBS, DTT, No.38 antigen, No.48 antigen were 25.2±1.8s, 24.5±2.1s, 31.2±3.2s, 27.1±5.1s, respectively. APTT value of mice immunized by PBS or DTT was similar to that of wild-type mice (APTT value of wild-type mice were 25.1s), indicating that PBS or DTT did not interfere with the coagulation function of endogenous pathway. APTT value of mice immunized by No.38 antigen was extended 1.24 times with respect to the value of PBS group (p <0.05, t-test), indicating that the antigen could inhibit the function of endogenous coagulation pathway in which FXII was involved.

PT values of mice immunized by PBS, DTT, No.38 antigen, and No.48 antigen were 10.4±0.2s, 10.6±0.1s, 10.1±0.5s, 10.2±0.4s, respectively. PT value of mice immunized by PBS or DTT was similar to that of wild-type mice (PT value of wild-type mice were 10.6s), indicating that PBS or DTT did not interfere with the coagulation function of exogenous pathway. PT value of mice immunized by No.38 antigen or No.48 antigen was not significantly extended, indicating that the designed recombinant antigen did not interfere with the function of exogenous coagulation pathway. Warfarin (2.5mg) was dissolved in 800ml of drinking water, and the mice were allowed free drink for three days. The PT value of mice was 60.1 ± 38.7s, which was extended 5.7 times relative to PT value of wild-type mice. The results showed that compared to warfarin (a common anticoagulant in clinical), the antigen of present invention did not cause abnormal bleeding in body.

### Step 5: Prevention of antigen to pulmonary embolism thrombosis

Immunization of mice and detection of antibody reaction were conducted according to Method 4. The experiment operation of pulmonary embolism was according to Step 5 in Example 3.

The survival rates of mice immunized with PBS, DTT, or No.38 antigen were 20%, 0%, or 37.5% at 20th minute, indicating that No.38 antigen could help mice against pulmonary embolism.

### Step 6: Prevention of antigens to thrombosis of postcava stenosis

Immunization of mice and detection of antibody reaction were conducted according to Method 4. The experiment operation of thrombosis of postcava stenosis was according to Step 6 in Example 3

As shown in Figure 6, the weight of emboli of mice immunized with PBS, DTT, No.38 antigen, and No.48 antigen were 11.2±2.1mg, 8.2±4.5mg, 4.7±2.7mg, and 4.8±2.5mg, respectively. The weight of emboli of mice immunized with No.38 antigen and No.48 antigen decreased 58%, 57% respectively compared to the weight of emboli of mice in the control group, indicating that the No.38 antigen and No.48 antigen could help mouse to prevent thrombosis of postcava stenosis.

### Example 5 Development of cancer vaccine targeting FAP

Fibroblast activation protein (FAP) is a membrane protein specifically expressed in carnicinoma associated fibroblasts (CAFs). FAP has a peptide chain with 6 amino acids as cytoplasmic domain, a hydrophobic segment with 19 amino acids as transmembrane domain, and an extracellular region comprising a β helix region and an αβ hydrolase region (amino acid sequence of 500th to 760th). FAP is specifically expressed in matrix of malignant epithelial tumors (including breast cancer, lung cancer, colon cancer, etc.), while it is not expressed in normal human tissues. It is important for FAP expression on tumor-associated fibroblasts for formation of microenvironment suitable for tumor growth. Studies have shown that overexpression of FAP can promote tumor microvessels generation and tumor growth, while knockout FAP can suppress tumor immune escape and cause immune response of body against tumor. In addition, compared with tumor antigens, FAP has a more stable genome. Therefore, many researchers have chosen FAP as a tumor therapeutic target. In animal experiments, inhibitors of FAP enzymatic activity have showed a good anti-tumor effect, and have entered clinical trials. The DNA vaccine targeting FAP stimulated a CTL effect in animal experiments, showing a good anti-tumor effect. Thus, in the application FAP was chosen as a target to develop cancer vaccines and to study its immunogenicity and anti-tumor effect.

### Step 1: Recombinant antigens design

A domain vaccine DTT-FAP was designed by fusing the catalytic domain of human FAP (position 500 to 760 of amino acid sequence) with C-terminal of DTT according to Method 1. In addition, a single epitope vaccine DTT-4B was designed, wherein the amino acid sequence position 291 to 297 (SETADNLE) of DTT was replaced by an epitope sequence position 239 to 246 (YGDEQYPR (SEQ ID NO.: 49)) of FAP. Amino acid sequences of antigens were as follows:

Amino acid sequence of DTT-FAP

DDDKINLDWDVIRDKTKTKIESLKEHGPIKNKMSESPNKTVSEEKA
KQYLEEFHQTALEHPELSELKTVTGTNPVFAGANYAAWAVNVAQVIDS
ETADNLEKTTAALSILPGIGSVMGIADGAVHHNTEEIVAQSIALSSLMVA
QAIPLVGELVDIGFAAYNFVESIINLFQVVHNSYNRPGGGGGNIQLPKEE
IKKLEVDEITLWYKMILPPQFDRSKKYPLLIQVYGGPCSQSVRSVFAVN
WISYLASKEGMVIALVDGRGTAFQGDKLLYAVYRKLGVYEVEDQITA
VRKFIEMGFIDEKRIAIWGWSYGGYVSSLALASGTGLFKCGIAVAPVSS
WEYYASVYTERFMGLPTKDDNLEHYKNSTVMARAEYFRNVDYLLIHG
TADDNVHFQNSAQIAKALVNAQVDFQAMWYSDQNHGLSGLSTNHLY
THMTHFLKQCFSLSD (SEQ ID NO.:50)

NOTE: DDDK was an enterokinase cleavage site which promotes expression of soluble fusion protein in pronucleus. Five glycine residues of GGGGG was a linker.

The catalytic domain of FAP (amino acid sequence 500-760)

NIQLPKEEIKKLEVDEITLWYKMILPPQFDRSKKYPLLIQVYGGPCS
QSVRSVFAVNWISYLASKEGMVIALVDGRGTAFQGDKLLYAVYRKLG
VYEVEDQITAVRKFIEMGFIDEKRIAIWGWSYGGYVSSLALASGTGLFK
CGIAVAPVSSWEYYASVYTERFMGLPTKDDNLEHYKNSTVMARAEYF
RNVDYLLIHGTADDNVHFQNSAQIAKALVNAQVDFQAMWYSDQNHG
LSGLSTNHLYTHMTHFLKQCFSLSD (SEQ ID NO.:108)

Amino acid sequence of DTT-4B

INLDWDVIRDKTKTKIESLKEHGPIKNKMSESPNKTVSEEKAKQYL
EEFHQTALEHPELSELKTVTGTNPVFAGANYAAWAVNVAQVIDYGDE
QYPRKTTAALSILPGIGSVMGIADGAVHHNTEEIVAQSIALSSLMVAQAI
PLVGELVDIGFAAYNFVESIINLFQVVHNSYNRP (SEQ ID NO.:51)

NOTE: YGDEQYPR was an epitope of FAP.

### Step 2: Construction of recombinant antigen vector, expression and identification and large-scale preparation of the protein

Referring to Method 2, a pGEX-6p-1 plasmid containing DTT gene was used as a template, and primer 1 and primer 2 were used in PCR to obtain DTT gene. A synthesized gene sequence of catalytic domain of FAP was used as template, and primer 3 and primer 4 were used in PCR to obtain gene of FAP domain. The DTT gene and FAP domain gene obtained in above operation were used as templates and primer 1 and primer 4 were used in overlap PCR to construct gene sequence of DTT-FAP fusion protein. Similarly, a pGEX-6p-1 plasmid containing DTT gene was used as a template, primer 5 and primer 6 were used to amplify upper part gene of DTT , primer 7 and primer 8 were used to amplify second part gene of DTT, primer 5 and primer 7 were used in overlap PCR to obtain the gene of DTT-4B. As detected by electrophoresis, DTT-FAP gene and DTT-4B gene were 1300 bp and 520 bp in length and in consistent with the theoretical values. Sequencing results showed that the sequence of fusion gene were correct. Sequences of recombinant protein and primers were as follows:
> Nucleotide sequence of DTT-FAP was shown in SEQ ID NO.:52.
> Primer sequence of DTT-FAP:

| Primers | Sequence 5'-3' |
|---|---|
| Primer 1: DTT forward (containing an enterokinase cleavage site) | CGCGGATCCGATGATGATGATAAGATAAATCTTGATTGG GATGTCATAAGG (SEQ ID NO.:53) |
| Primer 2: DTT reverse | CTCTTTAGGCAGCTGGATATTACCACCACCACCACCGGG ACGATTATACGAATTATG (SEQ ID NO.:54) |
| Primer 3: FAP forward | CATAATTCGTATAATCGTCCCGGTGGTGGTGGTGGTAATA TCCAGCTGCCTAAAGAG (SEQ ID NO.:55) |
| Primer 4: FAP reverse | CCGCTCGAGCTAGTCTGACAAAGAGAAACAC (SEQ ID NO.:56) |

> Nucleotide sequence ofDTT-4B was shown in SEQ ID NO.:57.
> Primer sequence of DTT-4B

| Primer name | Sequence 5'-3' |
|---|---|
| Primer 5: DTT(forward) | [?] CGCGGATCCCTGGAAGTTCTGTTCCAGGGGCCCA TAAATCTTGATTGGGATGTC (SEQ ID NO.:58) |
| Primer 6: DTT(reverse) | CCGCTCGAGCTAGGGACGATTATACGAATTATG (SEQ ID NO.:59) |
| Primer 7: 4B-1 (reverse) | [?] CACGCGGGTACTGTTCGTCACCGTAATCGATAAC TTGCGCAACGTTTACTGC (SEQ ID NO.:60) |
| Primer 8: 4B-2(forward) | TTACGGTGACGAACAGTACCCGCGTGATAATTTG GAAAAGACAACTGCTGC (SEQ ID NO.:61) |

The recombinant gene was loaded in pGEX-6P-1 vector and expressed by prokaryotic BL21 (DE3). Expression of recombinant proteins were implemented according to Method 3 and purified by GST-sepharose affinity purification. Electrophoresis analysis showed that antigens of DTT-FAP and DTT-4B were 51 kD and 20 kD, respectively which were consistent with the theoretical molecular weight. The purity was more than 90%.

### Step 3: Animal immunization and antibody titer detection

Immunization was implemented according to Method 4, wherein the mice in experiment was female Balb/C (purchased from Changzhou Cavens Co.). DTT-FAP group, DTT-4B group and the control group had 10, 8, and 8 mice separately. Freund's adjuvant was used in immunization and the immunizing dose was 30ng antigen/dose. One week after the third booster immunization, blood was collected to prepare serum.

ELISA was implemented according to Method 4. Human FAP (purchased from R & D System) was used as coating antigen, and the anti-serum sample of mice was diluted to 100 times as the primary antibody. A₄₅₀ values of DTT-FAP group and DTT-4B group detected by ELISA were 0.55 ± 0.3 and 0.48 ± 0.5, while the value of negative serum was 0.55 ± 0.06 (p <0.01). It was showed that DTT-FAP and DTT-4B could stimulate antibodies in mice against human FAP. The serum samples were diluted with dilution to 100 fold, 500 fold, 1000 fold, 5000 fold, 10,000 fold, 50,000 fold, or 1,000,000 fold, and tested with ELISA, and a curve of A₄₅₀ to dilution factor was made. The maximum dilution fold with A₄₅₀>0.2 and P/N≥2.1 (i.e. the ratio of absorbance value of the experimental group to that of control group was ≥2.1) was used as the titer of anti-serum. Titers of DTT-FAP group and DTT-4B group determined were 5000 and 1000, respectively.

### Step 4 Effect of anti-tumor

Referring to Method 9, one week after the third booster immunization, mice were inoculated with murine colon cancer of CT26. Compared with the control group, the speed of tumor formation of DTT-FAP group was slow. 12 days after tumor inoculation, tumor formation rate of DTT-FAP group was 90%, while the tumor formation rate of control group was 100%. 7 days after tumor inoculation, the length and width of the tumor were measured every other day with a caliper, tumor volume was calculated and survival curve was plotted (Figure 7B). 18 days after tumor inoculation, the tumor volume of DTT-FAP group was only 53.8% of control group (p <0.01). 27 days after tumor inoculation, the mice were killed, and tumors were removed, observed and photographed. Compared with DTT group, the tumor of DTT-FAP group was significantly smaller (Figure 7C). 27 days after tumor inoculation, the survival rate of DTT-FAP group mice were 80%, while survival rates of the other two groups of mice were below 30% (p <0.01) (Fig. 7A). These results suggested that DTT-FAP vaccine had significant anti-tumor effect.

### Example 6 Anti-cancer vaccine targeting VEGF

Anticancer drugs with VEGF as a target had extended lives of many cancer patients clinically. The inventors developed an antigen vaccine of DTT-VEGF targeting VEGF which was proven to have significant anti-tumor effects in mice tumor models.

### Step 1 DTT-VEGF antigen protein design

The inventor chose VEGF (8-109) insert to C-terminal of DTT (202-378) according to Method 1 to construct antigen protein of DTT-VEGF (or named as DTT-VEGF).

Amino acid sequence of antigen protein of DTT-VEGF

INLDWDVIRDKTKTKIESLKEHGPIKNKMSESPNKTVSEEKAKQYLEEFHQT
ALEHPELSELKTVTGTNPVFAGANYAAWAVNVAQVIDSETADNLEKTTAALSILP
GIGSVMGIADGAVHHNTEEIVAQSIALSSLMVAQAIPLVGELVDIGFAAYNFVESII

NLFQVVHNSYNRPGQNHHEVVKFMDVYQRSYCHPIETLVDIFQEYPDEIEYIFKP
SCVPLMRCGGCCNDEGLECVPTEESNITMQIMRIKPHQGQHIGEMSFLQHNKCEC
RPKKD (SEQ ID NO.:62)

Amino acid sequence of VEGF (8-109):


 (SEQ
ID NO.:63)

### Step 2 Preparation of antigen protein

Expression plasmid of pGEX-DTT-VEGF was constructed according to Method 2, and using a primer pair of VEGF-F and VEGF-R as primers of target protein gene. The correctness was proved by sequencing data. *E. coli BL21* was transformed with the expression plasmid of pGEX-DTT-VEGF, cultured, induced, and analyzed by SDS-PAGE. The protein had a molecular weight of about 30KD, and it proved that DTT-VEGF protein could be expressed. Cultures were magnified to 1L, and DTT-VEGF protein was prepared according to Method 3. Purity of the protein reached 90% as analyzed by 12% SDS-PAGE electrophoresis.

| | |
|---|---|
| VEGF-F: | CAAGTAGTTCATAATTCGTATAATCGTCCCGGTCAGAACCACCACGAGGTTGT (SEQ ID NO.:64) |
| VEGF-R: | CCGCTCGAGCTAATCTTTCTTCGGACGGCATTCGCACTTGTTGT (SEQ ID NO.:65) |

Gene sequence of DTT-VEGF antigen was shown in SEQ ID NO.:66.

### Step 3: Mice immunization

Immunization was implemented according to Method 4. Recombinant hVEGF protein (purchased from Beijing Sino Biological Technology Co., Ltd.) was used as coating antigen. ELISA results showed that, after immunized with DTT-VEGF, the OD₄₅₀ value of antiserum of mice (diluted 100 fold) to hVEGF165 reached 1.3, and the OD₄₅₀ value of antiserum, after immunized with VEGF alone, was about 0.1. The OD₄₅₀ values of DTT control group and alum adjuvant group were all less than 0.05. The results proved that antigenicity of antigen designed in the present application against VEGF was significantly increased compared with using VEGF alone. In addition, after immunized with DTT-VEGF, the OD₄₅₀ of the antiserum against mice VEGF (mVEGF164) reached 0.4, proving that after immunized with DTT-VEGF there were cross-reaction with mice VEGF.

### Step 4 Detection of humoral immunity and cellular immunity responses

### (1) Detection of the humoral immunity response

In order to verify whether immune tolerance could be breaked by the antigen designed, the inventor immunized mice with DTT-VEGF and aluminum hydroxide as adjuvant. It was found that high-intensity antibodies against hVEGF165 were produced after immunization, and the antibody also had cross-reactivity to VEGF of mice. Antibodies could effectively neutralize VEGF binding to receptor of VEGF2. If VEGF did not fused with DTT, it could not stimulate the production of antibodies. It was shown that Th epitopes on DTT played a key role in the process of the body breaking immune tolerance of VEGF to B cells.

### (2) Detection of cytotoxic T lymphocyte

A week after the third immunization, mice were killed to take spleen cells. Cells were added at a concentration of 10⁶ cells to a 6-well plate, and corresponding antigen was added to a final concentration of 25 µg/ml, and then stimulated and cultured for 3 days which was useful as effector cells. B16-F10 tumor cells were used as target cells. The effector cells and target cells were added into a 96-well plate with a certain concentration, and co-cultured in 37 °C incubator for 4 h. The concentration of LDH was measured with CytoTox96 non-radioactive cytotoxicity assay kit (Promega) using 50µl supernatant according to the standard procedure and lysis rate was calculated according to the the formula provided in the instruction.

CTL test showed that as the ratio of effector cells: target cells gradually increased, the lysis rate of target cells was gradually increased. When the ratio was 5: 1, the lysis rate of DTT-VEGF immunized group was about 5%, while the ratio was 40: 1, the lysis rate of DTT-VEGF immunized group reached 40% and the control group of DTT carrier protein was only 15%. There was a significant difference between them. It proven that DTT-VEGF could induce an antigen-specific CTL.

### Step 5 Detection of anti-tumor efficacy of the vaccine

### 1) Preventive effect in B16-F10 model

Mice were randomly divided into four groups, and each group had eight mice. Group A: D23V, Group B: VEGF (8-109), Group C: DTT, Group D: adjuvant. Mice were immunized with a dose of 0.03mg/mice through subcutaneous back. Booster immunization was conducted once every two weeks, totally three times. One week after every booster immunization, blood was taken via tail vein, and stored at -70 °C for use. One week after the second immunization, B16-F10 cell suspension (10⁵ cells/mice) was injected by subcutaneous and intravenous injection. The situation of tumor emergence was observed, the survival rate and other abnormalities were recorded.

### 2) Therapeutic efficacy in B16-F10 model

Mouse melanoma B16-F10 cells were cultured using DMEM culture solution with 10% fetal bovine serum, 100 U/ml penicillin and 100 µ/ml streptomycin, placed in 5% CO₂ incubator, and cultured at 37 °C. Cells in Exponential growth phase were digested with 0.25% trypsin, collected in a serum-free culture medium and gently shaked, centrifuged at 500g for 5 min, washed once, resuspended with PBS, adjusted to a concentration of 10⁶, and stained with trypan blue to detect cell viability which was above 95%. Armpit skin of mice was disinfected with 75% alcohol and mouse melanoma B16-F10 cells adjusted to the certain density were inoculated subcutaneously, 100µl for each animal, and 10⁵ cells were inoculated for each mouse (pre-experiment had confirmed that 10⁵ cells/mice could lead to tumor for 100%, i.e. the mice were assumed had got tumor). Two days after tumor inoculation, mice were injected the vaccine at a dose of 0.03 mg/mice to start treatment, once a week for three weeks. After tumor inoculation, the situation of tumor emergence was observed, the survival rate and other abnormalities were recorded.

### 3) Therapeutic efficacy on CT26.WT

Forty Balb/C mice with 6-8 week old were randomly divided into two groups, twenty mice for each group. The first group was immunized with DTT-VEGF using a dose of 0.03mg/mice through subcutaneous back. Booster immunization was conducted once every two weeks, totally three times. The other twenty mice were immunized by the same procedure with PBS as control. One week after every booster immunization, blood was taken via tail vein, and stored at -70 °C for use. One week after the second immunization, CT26.WT cell suspension (3x10⁵ cells/mice) was injected by subcutaneously. The situation of tumor emergence was observed, the survival rate and other abnormalities were recorded.

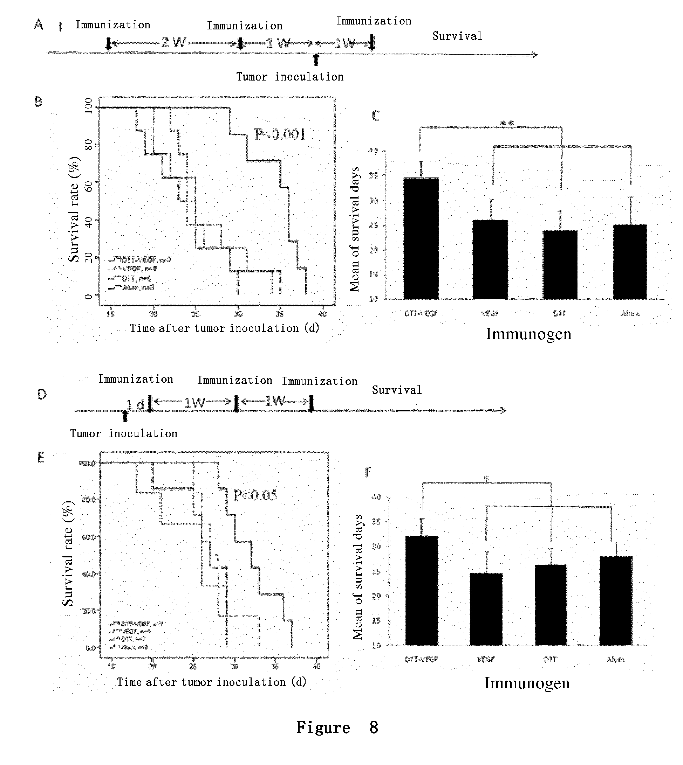

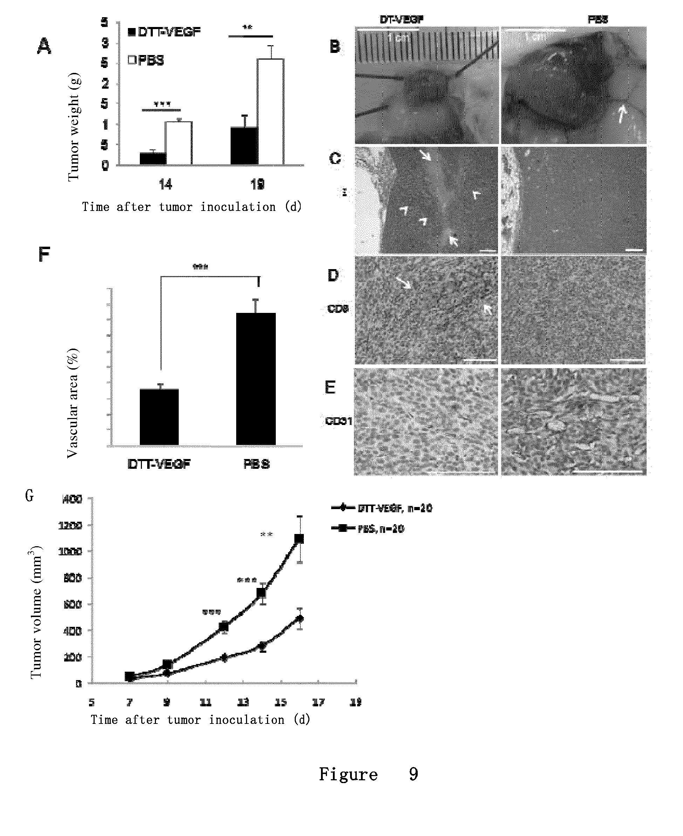

The results of preventive efficacy of B16-F10 model showed that the average survival time of DTT-VEGF group mice were extended to 35 days while the control group was 25 days (Figure 8B, and 8C) and survival time was greatly increased. It was found from treatment experiment of B16-F10 model that survival time of DTT-VEGF group mice was significantly extended. The average survival time was extended to 32 days compared with 25 days of the control group (Fig. 8E and 8F). In CT26 tumor therapy model, DTT-VEGF as a vaccine could significantly inhibit the growth of tumor cells (Fig. 9G); and the average survival time of mice was also extended from 25 days of the control group to 35 days of DTT-VEGF group. It was proven that immunization with DTT- VEGF could significantly extend the survival time of tumor-bearing mice.

### Step 6 Biopsy of tumor tissue

Tumor tissue without obvious necrosis on the edge of tumor tissue was taken for conventional paraffin biopsy, stained with HE, and detected by CD8 and CD31 immunohistochemistry. The results suggested that 14 days after tumor inoculation, the mean tumor weight of the group immunized with DTT-VEGF was less than 0.5g, while the control group of PBS reached 1g and 19 days after tumor inoculation, the mean tumor weight of the group immunized with DTT-VEGF reached 0.5g, while the control group of PBS reached exceeded 2.5g (Figure 9A). This proved that DTT-VEGF immunication could significantly inhibit tumor growth. Further observations indicated that around the tumor of DTT-VEGF group there were no significant blood vessels, while there were a lot of vessels surrounding the tumor in control group (Fig. 9B). This proved that DTT-VEGF immunication significantly inhibited tumor angiogenesis. Further, HE sections showed that after DTT-VEGF immunication, boundary between tumor cell nucleus and cell membrane was not obvious, and the nuclear membrane and the cell membrane was not complete, and a lot of cells were in necrosis (Figure 9C). While boundary between cell nucleus and cell membrane was obvious in the tumor of the control group, cells had a regular shape, and the majority of cells were living cells (FIG. 9C). Vascular density within the tumor was further analyzed by immunohistochemistry with anti-CD31 antibody and the results showed that vascular density within tumor of DTT-VEGF group was only 3%, while it reached 8% in the control group (Fig. 9E, F).

Lymphocytes infiltration was further analyzed. The staining results with anti-CD8 antibody showed that there were vast areas infiltrated by CD8+ cells within tumor of DTT-VEGF groups, while only scattered CD8 + lymphocytes were found in the tumor of control group (Fig. 9D). These results suggested that DTT-VEGF immunication suppressed tumor angiogenesis and promoted lymphocyte infiltration and necrosis.

### Example 7 Recombinant tumor vaccine targeting PDL1

Activated PDL1 and PD1 pathway can suppress activation of T lymphocyte and reduce the secretion of immune-related cytokines. PDL1/PD1 and other immunity negative regulator signaling pathways can reduce the recognization of tumor marker by immune system, thereby leading to immune escape of tumor. Monoclonal antibodies targeting PDL1 have entered into clinic; the recombinant vaccines targeting PDL1 and designed in this application also had good tumor inhibiting effect.

### Step 1: Antigen design

According to Method 1, PDL1E extracellular domain of PDL1 and PDL1E extracellular domain containing PDL1 and PD1 non-binding sites were selected, and inserted to C-terminal of DTT respectively to obtain recombinant proteins DTT-PDL1E and DTT-PDL1E1.

Amino acid sequence of mPDL1:

MRIFAGIIFTACCHLLRAFTITAPKDLYVVEYGSNVTMECRFPVERE
LDLLALVVYWEKEDEQVIQFVAGEEDLKPQHSNFRGRASLPKDQLLKG
NAALQITDVKLQDAGVYCCIISYGGADYKRITLKVNAPYRKINQRISVD
PATSEHELICQAEGYPEAEVIWTNSDHQPVSGKRSVTTSRTEGMLLNVT
SSLRVNATANDVFYCTFWRSQPGQNHTAELIIPELPATHPPQNRTHWVL
LGSILLFLIVVSTVLLFLRKQVRMLDVEKCGVEDTSSKNRNDTQFEET
(SEQ ID NO.:122)

Amino acid sequence of mPDL1E:

FTITAPKDLYVVEYGSNVTMECRFPVERELDLLALVVYWEKEDEQ
VIQFVAGEEDLKPQHSNFRGRASLPKDQLLKGNAALQITDVKLQDAGV
YCCIISYGGADYKRITLKVNAPYRKINQRISVDPATSEHELICQAEGYPE
AEVIWTNSDHQPVSGKRSVTTSRTEGMLLNVTSSLRVNATANDVFYCT
FWRSQPGQNHTAELIIPEL (SEQ ID NO.:67)

Amino acid sequence of mPDL1E1

APYNKINQRILVVDPVTSEHELTCQAEGYPKAEVIWTSSDHQVLSGKTTTTN
SKREEKLFNVTSTLRINTTTNEIFYCTFRRLDPEENHTAELVIPEL 10 (SEQ ID
NO.:68)

Full sequence of recombinant protein DTT-PDL1E

INLDWDVIRDKTKTKIESLKEHGPIKNKMSESPNKTVSEEKAKQYLEEFHQT
ALEHPELSELKTVTGTNPVFAGANYAAWAVNVAQVIDSETADNLEKTTAALSILP
GIGSVMGIADGAVHHNTEEIVAQSIALSSLMVAQAIPLVGELVDIGFAAYNFVESII
NLFQVVHNSYNRPFTITAPKDLYVVEYGSNVTMECRFPVERELDLLALVVYWEK
EDEQVIQFVAGEEDLKPQHSNFRGRASLPKDQLLKGNAALQITDVKLQDAGVYC
CIISYGGADYKRITLKVNAPYRKINQRISVDPATSEHELICQAEGYPEAEVIWTNSD
HQPVSGKRSVTTSRTEGMLLNVTSSLRVNATANDVFYCTFWRSQPGQNHTAELII
PEL (SEQ ID NO.:69)

Full sequence of recombinant protein DTT-PDL1E1

INLDWDVIRDKTKTKIESLKEHGPIKNKMSESPNKTVSEEKAKQYLEEFHQT
ALEHPELSELKTVTGTNPVFAGANYAAWAVNVAQVIDSETADNLEKTTAALSILP
GIGSVMGIADGAVHHNTEEIVAQSIALSSLMVAQAIPLVGELVDIGFAAYNFVESII
NLFQVVHNSYNRPAPYNKINQRILVVDPVTSEHELTCQAEGYPKAEVIWTSSDHQ
VLSGKTTTTTNSKREEKLFNVTSTLRINTTTNEIFYCTFRRLDPEENHTAELVIPEL
(SEQ ID NO.:70)

Amino acid sequence of hPDL1:

MRIFAVFIFMTYWHLLNAFTVTVPKDLYVVEYGSNMTIECKFPVEKQLDLA
ALIVYWEMEDKNIIQFVHGEEDLKVQHSSYRQRARLLKDQLSLGNAALQITDVK
LQDAGVYRCMISYGGADYKRITVKVNAPYNKINQRILVVDPVTSEHELTCQAEG
YPKAEVIWTSSDHQVLSGKTTTTNSKREEKLFNVTSTLRINTTTNEIFYCTFRRLD
PEENHTAELVIPELPLAHPPNERTHLVILGAILLCLGVALTFIFRLRKGRMMDVKKC

GIQDTNSKKQSDTHLEET (SEQ ID NO.:123)

Amino acid sequence of hPDL1E:

FTVTVPKDLYVVEYGSNMTIECKFPVEKQLDLAALIVYWEMEDKNIIQFVH
GEEDLKVQHSSYRQRARLLKDQLSLGNAALQITDVKLQDAGVYRCMISYGGAD
YKRITVKVNAPYNKINQRILVVDPVTSEHELTCQAEGYPKAEVIWTSSDHQVLSG
KTTTTNSKREEKLFNVTSTLRINTTTNEIFYCTFRRLDPEENHTAELVIPEL (SEQ
ID NO.:124)

Amino acid sequence of hPDL1E1:

APYNKINQRILVVDPVTSEHELTCQAEGYPKAEVIWTSSDHQVLSG
KTTTTNSKREEKLFNVTSTLRINTTTNEIFYCTFRRLDPEENHTAELVIPE
L (SEQ ID NO.:125)

Full sequence of recombinant protein DTT-hPDL1E:

INLDWDVIRDKTKTKIESLKEHGPIKNKMSESPNKTVSEEKAKQYL
EEFHQTALEHPELSELKTVTGTNPVFAGANYAAWAVNVAQVIDSETAD
NLEKTTAALSILPGIGSVMGIADGAVHHNTEEIVAQSIALSSLMVAQAIP
LVGELVDIGFAAYNFVESIINLFQVVHNSYNRPFTVTVPKDLYVVEYGS
NMTIECKFPVEKQLDLAALIVYWEMEDKNIIQFVHGEEDLKVQHSSYR
QRARLLKDQLSLGNAALQITDVKLQDAGVYRCMISYGGADYKRITVK
VNAPYNKINQRILVVDPVTSEHELTCQAEGYPKAEVIWTSSDHQVLSGK
TTTTNSKREEKLFNVTSTLRINTTTNEIFYCTFRRLDPEENHTAELVIPEL
(SEQ ID NO.:126)

Full sequence of recombinant protein DTT-hPDL1E1

INLDWDVIRDKTKTKIESLKEHGPIKNKMSESPNKTVSEEKAKQYL
EEFHQTALEHPELSELKTVTGTNPVFAGANYAAWAVNVAQVIDSETAD
NLEKTTAALSILPGIGSVMGIADGAVHHNTEEIVAQSIALSSLMVAQAIP

LVGELVDIGFAAYNFVESIINLFQVVHNSYNRPAPYNKINQRILVVDPVT
SEHELTCQAEGYPKAEVIWTSSDHQVLSGKTTTTNSKREEKLFNVTSTL
RINTTTNEIFYCTFRRLDPEENHTAELVIPEL (SEQ ID NO.:127)

### Step 2 Construction of plasmid and expression protein

Competent BL21 and Top 10 were purchased from TransGen Biotech. Vectors of pGEX-6p-1 and PMD18-T, ExTaq enzyme, T4 ligase, and reverse transcription kit were purchased from TaKaRa. PDL1 gene was expressed highly in melanoma, colon cancer, and lung cancer cells. The CT26 and B16-F10 were recovered and subcultured. Cells were collected and crushed by freeze-thaw. mRNAs were extracted and reverse transcribed into cDNA. PCR was implemented using the cDNA as template and primers P1 and P2. Full-length gene of mus-PDL1 was obtained and proved to be correct by sequencing. According to Method 2, plasmid mus-PDL1-DTT-E1-pGEX-6p-1 was obtained by nested PCR using DTT gene and full-length gene of PDL1 as a template, and primers P3, P4, P5, and P6; plasmid mus-PDL1-E-pGEX-6p-1 was obtained by PCR using PDL1 full-length gene as a template, and primers P11 and P12. Recombinant expression vectors of PDL1 were successfully constructed and verified by sequencing. Recombinant proteins of DTT (20KD), DTT-PDL1E (42KD), DTT-PDL1E1 (30KD), and PDL1E (23KD) were purified according to Method 3, and detected with SDS-PAGE. Single bands with size of target proteins were obtained and the purity as analyzed was more than 90% which is suitable for using as a vaccine for animal immunization.

### Sequence of primers

| Primer name | Sequence of primer 5'- 3' |
|---|---|
| Mus-PdL1-F (P1) | AGGATATTTGCTGGCATTATATTC (SEQ ID NO.:71) |
| Mus-PdlL1-R (P2) | TTACGTCTCCTCGAATTGTGT (SEQ ID NO.:72) |
| mus-DTT-PDL1E1-P1 (P3) | CGGAATTCGATGATGATGATAAGATAAATCTTGATTGGGATG TCAT (SEQ ID NO.:73) |
| mus-DTT-PDL1E1-P2 (P4) | CTCTGGTTGATTTTGCGGTATGGGGCGGGACGATTATACGA ATTATGAACTACTTG (SEQ ID NO.:74) |
| mus-DTT-PDL1E1-P3 (P5) | CAAGTAGTTCATAATTCGTATAATCGTCCCGCCCCATACCGC AAAATCAACCAGAG (SEQ ID NO.:75) |
| mus-DTT-PDL1E1-P4 (P6) | CGGCTCGAGCAGTTCTGGGATGATCAGCTC (SEQ ID NO.:76) |
| mus-DTT-PDL1E-P1 (P7) | CGGAATTCGATGATGATGATAAGATAAATCTTGATTGGGATG TCAT (SEQ ID NO.:77) |
| mus-DTT-PDL1E-P2 (P8) | CCTTTGGAGCCGTGATAGTAAAGGGACGATTATACGAATTA TG (SEQ ID NO.:78) |
| mus-DTT-PDL1E-P3 (P9) | CATAATTCGTATAATCGTCCCTTTACTATCACGGCTCCAAAG G (SEQ ID NO.:79) |
| mus-DTT-PDL1E-P4 (P10) | CTCGAGATTGACTTTCAGCGTGATTCGC (SEQ ID NO.:80) |
| mus-PDL1E-F (P11) | GGAATTCTTTACTATCACGGCTCCAAAG (SEQ ID NO.:81) |
| mus-PDL1E-R (P12) | GCTCGAGTCACAGTTCTGGGATGATC (SEQ ID NO.:82) |

### Step 3 Immune protection for animal

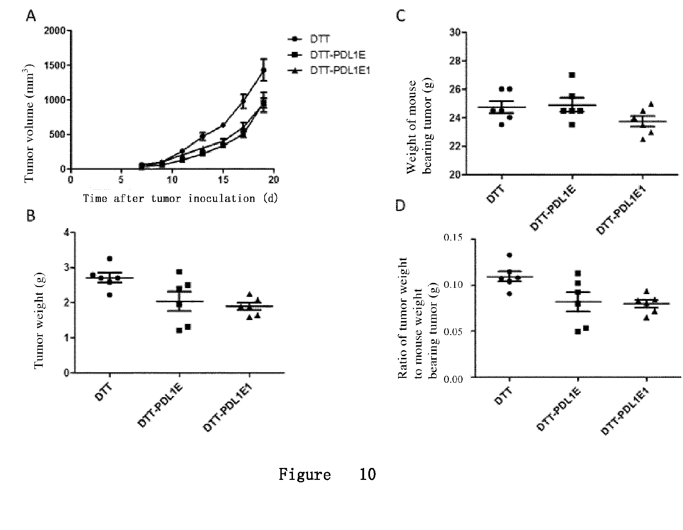

Eighteen female Balb/c mice of 9-week old were purchased from Beijing Charles River Laboratory Animal Technology Co., Ltd. The mice weighing 21 0.55 ± 1 were chosen as the experimental subjects and randomly divided into three groups: DTT group, DTT-PDL1E group, and DTT-PDL1E1 group (six mice in each group). Animal immunization was implemented according to Method 4. Serum was collected before immunization and serum was collected one day before each immunization. After third immunization, tumor cell cultures and tumor inoculation were implemented according to Method 9. Seven days after tumor inoculation, change of tumor volume was observed every other day and recorded. Change trend of tumor volume in mice (Tumor volume = width² x length x π/2) (Figure 10A) showed that DTT-PDL1E group, DTT-PDL1E1 group had a protective effect compared with the control group (P <0.05, n = 6). Twenty two days after tumor inoculation, the mice were mercy killed, autopsy of tumors was implemented, and tumors of every group were weighed (Figure 10B). Statistical analysis showed that there were significant difference between DTT and DTT-PDL1E (P <0.05), very significant difference between DTT and DTT-PDL1E1 (P <0.001), and no statistical difference between two immune protection groups (P> 0.05). Ratio of tumor weight and body weight had significant difference between DTT control group and DTT-PDL1E group (P <0.05), significant difference between DTT control group and DTT-PDL1E1 group (P <0.05), and no statistical difference between two immune protection groups (P> 0.05) (Figure 10C, D). Tumor inhibition rate of DTT-PDL1E obtained according to Method 9 was 24.6% and tumor inhibition rate of DTT-PDL1E1 was 30%. The results showed that the vaccine targeting DTT-PDL1 had significant anti-tumor effect.

### Example 8 Anti-tumor vaccines targeting EGFR

### Step 1 antigens design

Epidermal growth factor receptor (EGFR) is a multifunctional cell membrane glycoprotein widely distributed in human tissues. Its inhibitors (such as gefitinib and erlotinib) and monoclonal antibody drugs (such as cetuximab and panitumumab) have been approved for treatment of advanced colorectal cancer. Extracellular domain of EGFR is consisted of four sub-regions: sub-region⁻, sub-region⁻, sub-region⁻, and sub-region 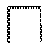 . Sub-region ⁻ interacts with ligand through main conserved amino acid residues. Sub-region 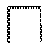 is the main site mediating dimer formation. The inventors fused sub-region 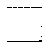 of EGFR to the C-terminal of DTT, and transplanted major epitopes (hEGFR 237-267 DTCPPLMLYNPTTYQMDVNPEGKYSFGATCV (SEQ ID NO.: 83), PPLMLYNPTTYQMDVNPE (SEQ ID NO.: 109)) of sub-region 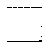 participating in dimer formation to the appropriate location on DTT based on principle of near distance of C-terminal to N-terminal, thereby designing a vaccine with multi-epitopes. Design steps could be seen in Method 1.

Four antigen proteins were designed in this example. They were DTT-mEGFRIII, DTT-hEGFRIII, DTT-mEGFRIII-pep, and DTT-pep-mEGFRIII. Amino acid sequences after fusion were as follows:

Amino acid sequence of antigen protein of DTT-mEGFRIII:

INLDWDVIRDKTKTKIESLKEHGPIKNKMSESPNKTVSEEKAKQYL
EEFHQTALEHPELSELKTVTGTNPVFAGANYAAWAVNVAQVIDSETAD
NLEKTTAALSILPGIGSVMGIADGAVHHNTEEIVAQSIALSSLMVAQAIP

LVGELVDIGFAAYNFVESIINLFQVVHNSYNRPRKVCNGIGIGEFKDTLS
INATNIKHFKYCTAISGDLHILPVAFKGDSFTRTPPLDPRELEILKTVKEIT
GFLLIQAWPDNWTDLHAFENLEIIRGRTKQHGQFSLAVVGLNITSLGLR
SLKEISDGDVIISGNRNLCYANTINWKKLFGTPNQKTKIMNNRAEKDCK
AVNHV (SEQ ID NO.:84)

Amino acid sequence of sub-region 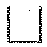 of mEGFR:

RKVCNGIGIGEFKDTLSINATNIKHFKYCTAISGDLHILPVAFKGDS
FTRTPPLDPRELEILKTVKEITGFLLIQAWPDNWTDLHAFENLEIIRGRTK
QHGQFSLAVVGLNITSLGLRSLKEISDGDVIISGNRNLCYANTINWKKLF
GTPNQKTKIMNNRAEKDCKAVNHV (SEQ ID NO.:110)

Amino acid sequence of antigen protein of DTT-hEGFRIII:

INLDWDVIRDKTKTKIESLKEHGPIKNKMSESPNKTVSEEKAKQYL
EEFHQTALEHPELSELKTVTGTNPVFAGANYAAWAVNVAQVIDSETAD
NLEKTTAALSILPGIGSVMGIADGAVHHNTEEIVAQSIALSSLMVAQAIP
LVGELVDIGFAAYNFVESIINLFQVVHNSYNRPRKVCNGIGIGEFKDSLSI
NATNIKHFKNCTSISGDLHILPVAFRGDSFTHTPPLDPQELDILKTVKEIT
GFLLIQAWPENRTDLHAFENLEIIRGRTKQHGQFSLAVVSLNITSLGLRS
LKEISDGDVIISGNKNLCYANTINWKKLFGTSGQKTKIISNRGENKCKAT
GQV (SEQ ID NO.:85)

Amino acid sequence of sub-region 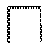 of hEGFR:

RKVCNGIGIGEFKDSLSINATNIKHFKNCTSISGDLHILPVAFRGDSF
THTPPLDPQELDILKTVKEITGFLLIQAWPENRTDLHAFENLEIIRGRTKQ
HGQFSLAVVSLNITSLGLRSLKEISDGDVIISGNKNLCYANTINWKKLFG
TSGQKTKIISNRGENKCKATGQV (SEQ ID NO.:111)

Amino acid sequence of antigen protein of DTT-mEGFRIII-pep:

INLDWDVIRDKTKTKIESLKEHGPIKNKMSESPNKTVSEEKAKQYL
EEFHQTALEHPELSELKTVTGTNPVFAGANYAAWAVNVAQVIDSETAD

NLEKTTAALSILPGIGSVMGIADGAVHHNTEEIVAQSIALSSLMVAQAIP
LVGELVDIGFAAYNFVESIINLFQVVHNSYNRPRKVCNGIGIGEFKDTLS
INATNIKHFKYCTAISGDLHILPVAFKGDSFTRTPPLDPRELEILKTVKEIT
GFLLIQAWPDNWTDLHAFENLEIIRGRTKQHGQFSLAVVGLNITSLGLR
SLKEISDGDVIISGNRNLCYANTINWKKLFGTPNQKTKIMNNRAEKDCK
AVNHVGGGGSDTCPPLMLYNPTTYQMDVNPEGKYSFGATCV (SEQ ID
NO.:86)

Amino acid sequence of antigen protein of DTT-hEGFRIII-pep:

INLDWDVIRDKTKTKIESLKEHGPIKNKMSESPNKTVSEEKAKQYL
EEFHQTALEHPELSELKTVTGTNPVFAGANYAAWAVNVAQVIDSETAD
NLEKTTAALSILPGIGSVMGIADGAVHHNTEEIVAQSIALSSLMVAQAIP
LVGELVDIGFAAYNFVESIINLFQVVHNSYNRPRKVCNGIGIGEFKDSLSI
NATNIKHFKNCTSISGDLHILPVAFRGDSFTHTPPLDPQELDILKTVKEIT
GFLLIQAWPENRTDLHAFENLEIIRGRTKQHGQFSLAVVSLNITSLGLRS
LKEISDGDVIISGNKNLCYANTINWKKLFGTSGQKTKIISNRGENKCKAT
GQVGGGGSDTCPPLMLYNPTTYQMDVNPEGKYSFGATCV (SEQ ID
NO.:119)

Amino acid sequence of antigen protein of DTT-pep-mEGFRIII (DSETADN in DTT was replaced with PPLMLYNPTTYQMDVNPE):

INLDWDVIRDKTKTKIESLKEHGPIKNKMSESPNKTVSEEKAKQYL
EEFHQTALEHPELSELKTVTGTNPVFAGANYAAWAVNVAQVIPPLMLY
NPTTYQMDVNPELEKTTAALSILPGIGSVMGIADGAVHHNTEEIVAQSI
ALSSLMVAQAIPLVGELVDIGFAAYNFVESIINLFQVVHNSYNRPRKVC
NGIGIGEFKDTLSINATNIKHFKYCTAISGDLHILPVAFKGDSFTRTPPLD
PRELEILKTVKEITGFLLIQAWPDNWTDLHAFENLEIIRGRTKQHGQFSL
AVVGLNITSLGLRSLKEISDGDVIISGNRNLCYANTINWKKLFGTPNQKT
KIMNNRAEKDCKAVNHV (SEQ ID NO.:87)

Amino acid sequence of antigen protein of DTT-pep-hEGFRIII (DSETADN in DTT was replaced by PPLMLYNPTTYQMDVNPE):

INLDWDVIRDKTKTKIESLKEHGPIKNKMSESPNKTVSEEKAKQYL
EEFHQTALEHPELSELKTVTGTNPVFAGANYAAWAVNVAQVIPPLMLY
NPTTYQMDVNPELEKTTAALSILPGIGSVMGIADGAVHHNTEEIVAQSI
ALSSLMVAQAIPLVGELVDIGFAAYNFVESIINLFQVVHNSYNRPRKVC
NGIGIGEFKDSLSINATNIKHFKNCTSISGDLHILPVAFRGDSFTHTPPLDP
QELDILKTVKEITGFLLIQAWPENRTDLHAFENLEIIRGRTKQHGQFSLA
VVSLNITSLGLRSLKEISDGDVIISGNKNLCYANTINWKKLFGTSGQKTK
IISNRGENKCKATGQV (SEQ ID NO.:120)

### Step 2: Construction of vector, expression and purification of the fusion protein

B epitope was introduced to T domain of DT by overlapping PCR using designed primers according to the sequence of open reading frame of mRNA of T domain of DT gene and mRNA of mEGFR and hEGFR gene in GeneBank. Primers and templates were synthesized by Nanjing GenScript Biotechnology Co., Ltd. PCR products were prepared by overlapping PCR. Two pairs of primers of 1F and 3R, 2F and 4R were used in the first round of PCR for each protein. Primers of 1F and 4R were used in the second round of PCR. PCR and construction step of vector were described in Method 2.

After obtaining positive clones, plasmids were extracted and identified by PCR. As identified, PCR products of four antigen proteins (DTT-mEGFRIII, DTT-hEGFRIII, DTT-mEGFRIII-pep and DTT-pep-mEGFRIII) were all about 1200KD, and were consistent with expectation. Positive clones were sequenced by GenScript Sequence Co., and sequencing results were consistent with the design expectation. Expression and preparation method of recombinant protein were described in Method 3. Proteins were detected by SDS-PAGE. The results showed that molecular weights of DTT-mEGFR, DTT-hEGFR, DTT-pep-mEGFR, and DTT-mEGFR-pep were about 40KD which were consistent with expectation. Purity of proteins was more than 90%.

The sequences of nucleic acid and primers for four antigen proteins designed in the application were as follows:

**Table of primers sequences in Example7**

| Primer name | Primer sequence |
|---|---|
| DTT-mEGFRIII-1F: | CGCGGATCCCTGGAAGTTCTGTTCCAGGGGCCCCGCAAAGTTTGT AATGGCATAGGC (SEQ ID NO.:88) |
| DTT-mEGFRIII-2F: | GTAGTTCATAATTCGTATAATCGTCCCCGCAAAGTTTGTAATGGC ATAGGC (SEQ ID NO.:89) |
| DTT-mEGFRIII-3R: | GCCTATGCCATTACAAACTTTGCGGGACGATTATACGAATTATG AACTAC (SEQ ID NO.:90) |
| DTT-mEGFRIII-4R: | CCGCTCGAGCTAGACGTGGTTCACGGCCTTGCAGTCTTTC (SEQ ID NO.:91) |
| DTT-hEGFRIII-1F: | CGCGGATCCCTGGAAGTTCTGTTCCAGGGGCCCCGCAAAGTTTGT AATGGCATAGGC (SEQ ID NO.:92) |
| DTT-hEGFRIII-2F: | GTAGTTCATAATTCGTATAATCGTCCCCGCAAAGTGTGTAACGGA ATAGGTATTGG (SEQ ID NO.:93) |
| DTT-hEGFRIII-3R: | CCAATACCTATTCCGTTACACACTTTGCGGGGACGATTATACGAA TTATGAACTAC (SEQ ID NO.:94) |
| DTT-hEGFRIII-4R: | CCGCTCGAGCTAGACCTGGCCTGTGGCCTTGCAGCTGTTTTC (SEQ ID NO.:95) |
| DTT-pep-mEGFRIII-1F | CGCGGATCCCTGGAAGTTCTGTTCCAGGGGCCCCGCAAAGTTTGT AATGGCATAGGC (SEQ ID NO.:96) |
| DTT-pep-mEGFRIII-2F | CAACCCCACCACCTATCAGATGGATGTCAACCCTGAATTGGAAA AGACAACTGCTGCTC (SEQ ID NO.:97) |
| DTT-pep-mEGFRI1n-3R | GTTGACATCCATCTGATAGGTGGTGGGTTGTACAGCATGAGTG GTGGGATAACTTGCGCAACGTTTACTG (SEQ ID NO.:98) |
| DTT-pep-mEGFRIII-4R | CCGCTCGAGCTAGACGTGGTTCACGGCCTTGCAGTCTTTC (SEQ ID NO.:99) |
| DTT-mEGFRIII-pep-1F | CGCGGATCCCTGGAAGTTCTGTTCCAGGGGCCCCGCAAAGTTTGT |
| | AATGGCATAGGC (SEQ ID NO.:100) |
| DTT-mEGFRIII-pep-2F | GTAGTTCATAATTCGTATAATCGTCCCCGCAAAGTTTGTAATGGC ATAGGC (SEQ ID NO.:101) |
| DTT-mEGFRIII-pep-3R | GCCTATGCCATTACAAACTTTGCGGGACGATTATACGAATTATG AACTAC (SEQ ID NO.:102) |
| DTT-mEGFRIII-pep-4R | CCGCTCGAGCTACACACAGGTGGCACCAAAGCTGTACTTC (SEQ ID NO.:103) |

| | |
|---|---|
| Gene sequence of antigen protein of DTT-mEGFRIII was shown in SEQ ID NO.:104. Gene sequence of antigen protein of DTT-hEGFRIII was shown in SEQ ID NO.:105. Gene sequence of antigen protein of DTT-pep-mEGFRIII was shown in SEQ ID NO.:106. Gene sequence of antigen protein of DTT-mEGFRIII-pep was shown in SEQ ID NO.:107. | |

### Step 3 Detection of immunogenicity

Serum of ten mice of every group was detected and mEGFR standard (purchased from Beijing Sino Biological Technology Co., Ltd.) was coated and detected by ELISA according to Method 4. ELISA assay showed that the mean value of PBS group was 0.31, the mean value of DTT group was 0.34, the mean value of DTT-mEGFR group was 1.84, the mean value of DTT-hEGFR group was 1.65, the mean value of DTT-pep-mEGFR group was 1.91, and the mean value of DTT-mEGFR-pep group was 1.31. Four experimental groups all produced better antibodies against mEGFR, indicating that the method of this invention successfully broke the immune tolerance and produced antibodies against mEGFR (p <0.05).

As for coated Her1 standard, ELISA results showed that the mean value of PBS group was 0.17, the mean value of DTT group was 0.20, the mean value of DTT-mEGFR group was 0.60, the mean value of DTT-hEGFR group was 1.25, the mean value of DTT-pep-mEGFR group was 0.87, and the mean value of DTT-mEGFR-pep group was 0.29. Four experimental groups all produced better antibodies against mEGFR indicating that the method of invention successfully broke the immune tolerance and produced antibodies against its own proteins (p <0.05). mEGFR induced antibodies against Her1 while Her1 induced antibodies against mEGFR, indicating the existence of cross reaction.

### Step 4 Western Identification

Electrophoresis samples were tested using A431 cell lysates, while B16F10 cell lysates were selected as a negative control. Specific experimental procedures were described in Method 6. By color reaction, there were significant antibody bands of the experimental group against A431 cell lysates, and the location was about 105KD which was in consistent with EGFR molecular weight. This showed that there the antibody of the invention had binding specificity to EGFR on cell surface.

### Step 5 Immunological therapy to tumor-bearing mice

Mice were immunized according to Method 4. Culture, migration, and apparent effect evaluation of the tumor cells were implemented according to Method 9. Lewis lung tumor cells of mice were selected to establish tumor model, and inoculated with 5 x 10⁵ cells per mice. Tumor growth curve was made using inoculation days as abscissa and tumor volume as ordinate. The results showed that there were significant differences between four experimental groups and PBS and DTT control groups from 16 days (p <0.05). Tumor inhibition rate of DTT-mEGFR group was 16% relative to the PBS group. Tumor inhibition rate of DTT-hEGFR group was 32% relative to the PBS group. Tumor inhibition rate of DTT-mEGFR-pep group was 21% relative to the PBS group. Tumor inhibition rate of DTT-pep-mEGFR group was 37% relative to the PBS group. The results suggested that our vaccine could break immune tolerance and had better effect of tumor inhibition.

### Step 6 T cell proliferation assay

A mEGFR standard (purchased from Beijing Sino Biological Technology Co., Ltd.) was used as an antigen to stimulate spleen cells, and experimental procedures were described in Method 7. Three mice were selected in every group. The mean reading of PBS group was 0.12, the mean reading of DTT group was 0.12, and the mean reading of DTT-pep-mEGFR group was 0.20. There was significant difference between the experimental group and the two control groups (p <0.05), indicating that DTT-pep-mEGFR vaccine could stimulate T cell proliferation, and break the immune tolerance of cell.

### Step 7 CTL killing assay

A mEGFR standard (purchased from Beijing Sino Biological Technology Co., Ltd.) was used as antigen to stimulate the spleen cells and Lewis lung tumor cells were used as target cell. Specific experimental procedures were described in Method 8. Three mice were selected in every group, the mean killing rate of PBS group to Lewis cell was 4%, the mean killing rate of DTT group was 8%, and the mean killing rate of DTT-pep-mEGFR group was 29.8%. There was significant difference between the experimental group and the two control groups (p <0.05), indicating that DTT-pep-mEGFR vaccine generated CTL killing effect against the target cell.

A11 documents referred to in the present invention are incorporated by reference as if each reference is cited alone as a reference in the present application. In addition, it should be understood that after reading the teachings of the present invention described above, a skilled person in the art can make various changes or modifications of the invention, and these equivalent forms also fall into the scope as defined by the appended claims of the present application.

## Claims

1. A recombinant protein with an antigenic epitope, wherein the recombinant protein has a skeleton structure of a carrier protein which has at least one T cell epitope, and the antigenic epitope is imported into at least one molecular surface amino acid residue area of the carrier protein by splicing, replacement, and/or insertion.

2. The recombinant protein of claim 1, wherein the antigenic epitope is an epitope from a low immunogenic protein, and preferably the low immunogenic protein comprises human and non-human mammalian protein.

3. The recombinant protein of claim 1, wherein the carrier protein is a pathogen protein, including a viral protein, a bacterial protein, a chlamydia protein, a mycoplasma protein, a non-human animal protein or combination thereof.

4. The recombinant protein of claim 2, wherein the low immunogenic protein is selected from the group consisting of VEGF, TNF-α, Her2 protein, clotting factor, interleukins, fibroblast activation protein (FAP), EGFR, PDL1 or combination thereof.

5. The recombinant protein of claim 1, wherein the molecular surface amino acid residue area comprises C-terminal of the carrier protein, and/or the molecular surface amino acid residue area includes N-terminal of the carrier protein.

6. The recombinant protein of claim 1, wherein the carrier protein is a transmembrane domain of diphtheria toxin (or DTT).

7. The recombinant protein of claim 6, wherein the molecular surface amino acid residue area is within amino acid position 287 to 299 of DTT or the molecular surface amino acid residue area is C-terminal or N-terminal of DTT,
preferably the molecular surface amino acid residue area is selected from the group consisting of: amino acid position 290-297 of DTT, amino acid position 291-297 of DTT, amino acid position 292-297 of DTT, amino acid position 293-297 of DTT, amino acid position 294-297 of DTT, amino acid position 295-297 of DTT and amino acid position 296-297 of DTT.

8. A polynucleotide encoding the recombinant protein according to claim 1. 1.

9. A vector containing the polynucleotide according to claim 8.

10. A host cell, wherein the host cell contains the vector according to claim 9, or has the polynucleotide according to claim 8 integrated in its genome.

11. A pharmaceutical composition, wherein the composition comprises the recombinant protein according to claim 1, the polynucleotide according to claim 8, or the vector according to claim 9, or the host cell according to claim 10, and a pharmaceutically acceptable carrier and/or excipient.

12. The pharmaceutical composition of claim 11, wherein the composition is vaccine.

13. A vaccine composition, wherein the composition comprises the recombinant protein according to claim 1, the polynucleotide according to claim 8, or the vector according to claim 9, or the host cell according to claim 10, and an immunologically acceptable carrier and/or excipient.

14. A use of the recombinant protein with an antigenic epitope according to claim 1, wherein,
(a) the recombinant protein is used for preparing an antibody against the antigenic epitope; and/or
(b) the recombinant protein is used for preparing a medicine for treatment of an antigenic epitope-related disease.

15. An antigenic epitope peptide, wherein the antigenic epitope peptide is derived from a low immunogenic protein of mammal (such as human), and contains one or more antigenic epitopes,
and the length of amino acid sequence of the antigenic epitope peptide is 5-100% of the full-length of corresponding low immunogenic protein, and the length of the antigenic epitope peptide is 5-500 amino acids;
and a recombinant protein formed by the antigenic epitope peptide with a carrier protein of the present invention can induce an immune response against the low immunogenic protein in a mammal of the same species.

16. A method of treatment comprising a step of administering the recombinant protein of claim 1, the pharmaceutical composition of claim 11 or the vaccine composition of claim 13 to a subject in need thereof.


SEQUENCE LISTING

<110>  SHANGHAI HYCHARM INC

<120>   Epitope Vaccine For Low Immunogenic Protein and Preparing Method
        and Usage Thereof

<130>  P2014-0411

<150>  201310178135.2
<151>  2013-05-14

<160>  131

<170>  PatentIn version 3.5

<210>  1
<211>  535
<212>  PRT
<213>  Corynebacterium diphtheriae

<400>  1

Gly Ala Asp Asp Val Val Asp Ser Ser Lys Ser Phe Val Met Glu Asn
1               5                   10                  15

Phe Ser Ser Tyr His Gly Thr Lys Pro Gly Tyr Val Asp Ser Ile Gln
            20                  25                  30

Lys Gly Ile Gln Lys Pro Lys Ser Gly Thr Gln Gly Asn Tyr Asp Asp
        35                  40                  45

Asp Trp Lys Gly Phe Tyr Ser Thr Asp Asn Lys Tyr Asp Ala Ala Gly
    50                  55                  60

Tyr Ser Val Asp Asn Glu Asn Pro Leu Ser Gly Lys Ala Gly Gly Val
65                  70                  75                  80

Val Lys Val Thr Tyr Pro Gly Leu Thr Lys Val Leu Ala Leu Lys Val
                85                  90                  95

Asp Asn Ala Glu Thr Ile Lys Lys Glu Leu Gly Leu Ser Leu Thr Glu
            100                 105                 110

Pro Leu Met Glu Gln Val Gly Thr Glu Glu Phe Ile Lys Arg Phe Gly
        115                 120                 125

Asp Gly Ala Ser Arg Val Val Leu Ser Leu Pro Phe Ala Glu Gly Ser
    130                 135                 140

Ser Ser Val Glu Tyr Ile Asn Asn Trp Glu Gln Ala Lys Ala Leu Ser
145                 150                 155                 160

Val Glu Leu Glu Ile Asn Phe Glu Thr Arg Gly Lys Arg Gly Gln Asp
                165                 170                 175

Ala Met Tyr Glu Tyr Met Ala Gln Ala Cys Ala Gly Asn Arg Val Arg
            180                 185                 190

Arg Ser Val Gly Ser Ser Leu Ser Cys Ile Asn Leu Asp Trp Asp Val
        195                 200                 205

Ile Arg Asp Lys Thr Lys Thr Lys Ile Glu Ser Leu Lys Glu His Gly
    210                 215                 220

Pro Ile Lys Asn Lys Met Ser Glu Ser Pro Asn Lys Thr Val Ser Glu
225                 230                 235                 240

Glu Lys Ala Lys Gln Tyr Leu Glu Glu Phe His Gln Thr Ala Leu Glu
                245                 250                 255

His Pro Glu Leu Ser Glu Leu Lys Thr Val Thr Gly Thr Asn Pro Val
            260                 265                 270

Phe Ala Gly Ala Asn Tyr Ala Ala Trp Ala Val Asn Val Ala Gln Val
        275                 280                 285

Ile Asp Ser Glu Thr Ala Asp Asn Leu Glu Lys Thr Thr Ala Ala Leu
    290                 295                 300

Ser Ile Leu Pro Gly Ile Gly Ser Val Met Gly Ile Ala Asp Gly Ala
305                 310                 315                 320

Val His His Asn Thr Glu Glu Ile Val Ala Gln Ser Ile Ala Leu Ser
                325                 330                 335

Ser Leu Met Val Ala Gln Ala Ile Pro Leu Val Gly Glu Leu Val Asp
            340                 345                 350

Ile Gly Phe Ala Ala Tyr Asn Phe Val Glu Ser Ile Ile Asn Leu Phe
        355                 360                 365

Gln Val Val His Asn Ser Tyr Asn Arg Pro Ala Tyr Ser Pro Gly His
    370                 375                 380

Lys Thr Gln Pro Phe Leu His Asp Gly Tyr Ala Val Ser Trp Asn Thr
385                 390                 395                 400

Val Glu Asp Ser Ile Ile Arg Thr Gly Phe Gln Gly Glu Ser Gly His
                405                 410                 415

Asp Ile Lys Ile Thr Ala Glu Asn Thr Pro Leu Pro Ile Ala Gly Val
            420                 425                 430

Leu Leu Pro Thr Ile Pro Gly Lys Leu Asp Val Asn Lys Ser Lys Thr
        435                 440                 445

His Ile Ser Val Asn Gly Arg Lys Ile Arg Met Arg Cys Arg Ala Ile
    450                 455                 460

Asp Gly Asp Val Thr Phe Cys Arg Pro Lys Ser Pro Val Tyr Val Gly
465                 470                 475                 480

Asn Gly Val His Ala Asn Leu His Val Ala Phe His Arg Ser Ser Ser
                485                 490                 495

Glu Lys Ile His Ser Asn Glu Ile Ser Ser Asp Ser Ile Gly Val Leu
            500                 505                 510

Gly Tyr Gln Lys Thr Val Asp His Thr Lys Val Asn Ser Lys Leu Ser
        515                 520                 525

Leu Phe Phe Glu Ile Lys Ser
    530                 535

<210>  2
<211>  177
<212>  PRT
<213>  Corynebacterium diphtheriae

<400>  2

Ile Asn Leu Asp Trp Asp Val Ile Arg Asp Lys Thr Lys Thr Lys Ile
1               5                   10                  15

Glu Ser Leu Lys Glu His Gly Pro Ile Lys Asn Lys Met Ser Glu Ser
            20                  25                  30

Pro Asn Lys Thr Val Ser Glu Glu Lys Ala Lys Gln Tyr Leu Glu Glu
        35                  40                  45

Phe His Gln Thr Ala Leu Glu His Pro Glu Leu Ser Glu Leu Lys Thr
    50                  55                  60

Val Thr Gly Thr Asn Pro Val Phe Ala Gly Ala Asn Tyr Ala Ala Trp
65                  70                  75                  80

Ala Val Asn Val Ala Gln Val Ile Asp Ser Glu Thr Ala Asp Asn Leu
                85                  90                  95

Glu Lys Thr Thr Ala Ala Leu Ser Ile Leu Pro Gly Ile Gly Ser Val
            100                 105                 110

Met Gly Ile Ala Asp Gly Ala Val His His Asn Thr Glu Glu Ile Val
        115                 120                 125

Ala Gln Ser Ile Ala Leu Ser Ser Leu Met Val Ala Gln Ala Ile Pro
    130                 135                 140

Leu Val Gly Glu Leu Val Asp Ile Gly Phe Ala Ala Tyr Asn Phe Val
145                 150                 155                 160

Glu Ser Ile Ile Asn Leu Phe Gln Val Val His Asn Ser Tyr Asn Arg
                165                 170                 175

Pro

<210>  3
<211>  54
<212>  DNA
<213>  Artificial sequence

<220>
<223>  Polynucleotide

<400>  3
cgcggatccc tggaagttct gttccagggg cccataaatc ttgattggga tgtc          54

<210>  4
<211>  33
<212>  DNA
<213>  Artificial sequence

<220>
<223>  Polynucleotide

<400>  4
ccgctcgagc tagggacgat tatacgaatt atg                                 33

<210>  5
<211>  17
<212>  PRT
<213>  Artificial sequence

<220>
<223>  Polypeptide

<400>  5

Val Ser Arg Phe Ala Ile Ser Tyr Gln Glu Lys Val Asn Leu Leu Ser
1               5                   10                  15

Ala

<210>  6
<211>  9
<212>  PRT
<213>  Artificial sequence

<220>
<223>  Polypeptide

<400>  6

Leu Asp Phe Ala Glu Ser Gly Gln Val
1               5

<210>  7
<211>  8
<212>  PRT
<213>  Artificial sequence

<220>
<223>  Polypeptide

<400>  7

Trp Leu Asn Arg Arg Ala Asn Ala
1               5

<210>  8
<211>  11
<212>  PRT
<213>  Artificial sequence

<220>
<223>  Polypeptide

<400>  8

Gly Met Asp Leu Lys Asp Asn Gln Leu Val Val
1               5                   10

<210>  9
<211>  334
<212>  PRT
<213>  artificial sequence

<220>
<223>  Polypeptide

<400>  9

Ile Asn Leu Asp Trp Asp Val Ile Arg Asp Lys Thr Lys Thr Lys Ile
1               5                   10                  15

Glu Ser Leu Lys Glu His Gly Pro Ile Lys Asn Lys Met Ser Glu Ser
            20                  25                  30

Pro Asn Lys Thr Val Ser Glu Glu Lys Ala Lys Gln Tyr Leu Glu Glu
        35                  40                  45

Phe His Gln Thr Ala Leu Glu His Pro Glu Leu Ser Glu Leu Lys Thr
    50                  55                  60

Val Thr Gly Thr Asn Pro Val Phe Ala Gly Ala Asn Tyr Ala Ala Trp
65                  70                  75                  80

Ala Val Asn Val Ala Gln Val Ile Asp Ser Glu Thr Ala Asp Asn Leu
                85                  90                  95

Glu Lys Thr Thr Ala Ala Leu Ser Ile Leu Pro Gly Ile Gly Ser Val
            100                 105                 110

Met Gly Ile Ala Asp Gly Ala Val His His Asn Thr Glu Glu Ile Val
        115                 120                 125

Ala Gln Ser Ile Ala Leu Ser Ser Leu Met Val Ala Gln Ala Ile Pro
    130                 135                 140

Leu Val Gly Glu Leu Val Asp Ile Gly Phe Ala Ala Tyr Asn Phe Val
145                 150                 155                 160

Glu Ser Ile Ile Asn Leu Phe Gln Val Val His Asn Ser Tyr Asn Arg
                165                 170                 175

Pro Met Leu Arg Ser Ser Ser Gln Asn Ser Ser Asp Lys Pro Val Ala
            180                 185                 190

His Val Val Ala Asn His Gln Val Glu Glu Gln Leu Glu Trp Leu Ser
        195                 200                 205

Gln Arg Ala Asn Ala Leu Leu Ala Asn Gly Met Asp Leu Lys Asp Asn
    210                 215                 220

Gln Leu Val Val Pro Ala Asp Gly Leu Tyr Leu Val Tyr Ser Gln Val
225                 230                 235                 240

Leu Phe Lys Gly Gln Gly Cys Pro Asp Tyr Val Leu Leu Thr His Thr
                245                 250                 255

Val Ser Arg Phe Ala Ile Ser His Gln Glu Lys Val Asn Leu Leu Ser
            260                 265                 270

Ala Val Lys Ser Pro Cys Pro Lys Asp Thr Pro Glu Gly Ala Glu Leu
        275                 280                 285

Lys Pro Trp Tyr Glu Pro Ile Tyr Leu Gly Gly Val Phe Gln Leu Glu
    290                 295                 300

Lys Gly Asp Gln Leu Ser Ala Glu Val Asn Leu Pro Lys Tyr Leu Asp
305                 310                 315                 320

Phe Arg Glu Ser Gly Gln Val Tyr Phe Gly Val Ile Ala Leu
                325                 330

<210>  10
<211>  157
<212>  PRT
<213>  Artificial sequence

<220>
<223>  Polypeptide

<400>  10

Met Leu Arg Ser Ser Ser Gln Asn Ser Ser Asp Lys Pro Val Ala His
1               5                   10                  15

Val Val Ala Asn His Gln Val Glu Glu Gln Leu Glu Trp Leu Ser Gln
            20                  25                  30

Arg Ala Asn Ala Leu Leu Ala Asn Gly Met Asp Leu Lys Asp Asn Gln
        35                  40                  45

Leu Val Val Pro Ala Asp Gly Leu Tyr Leu Val Tyr Ser Gln Val Leu
    50                  55                  60

Phe Lys Gly Gln Gly Cys Pro Asp Tyr Val Leu Leu Thr His Thr Val
65                  70                  75                  80

Ser Arg Phe Ala Ile Ser His Gln Glu Lys Val Asn Leu Leu Ser Ala
                85                  90                  95

Val Lys Ser Pro Cys Pro Lys Asp Thr Pro Glu Gly Ala Glu Leu Lys
            100                 105                 110

Pro Trp Tyr Glu Pro Ile Tyr Leu Gly Gly Val Phe Gln Leu Glu Lys
        115                 120                 125

Gly Asp Gln Leu Ser Ala Glu Val Asn Leu Pro Lys Tyr Leu Asp Phe
    130                 135                 140

Arg Glu Ser Gly Gln Val Tyr Phe Gly Val Ile Ala Leu
145                 150                 155

<210>  11
<211>  335
<212>  PRT
<213>  Artificial sequence

<220>
<223>  Polypeptide

<400>  11

Ile Asn Leu Asp Trp Asp Val Ile Arg Asp Lys Thr Lys Thr Lys Ile
1               5                   10                  15

Glu Ser Leu Lys Glu His Gly Pro Ile Lys Asn Lys Met Ser Glu Ser
            20                  25                  30

Pro Asn Lys Thr Val Ser Glu Glu Lys Ala Lys Gln Tyr Leu Glu Glu
        35                  40                  45

Phe His Gln Thr Ala Leu Glu His Pro Glu Leu Ser Glu Leu Lys Thr
    50                  55                  60

Val Thr Gly Thr Asn Pro Val Phe Ala Gly Ala Asn Tyr Ala Ala Trp
65                  70                  75                  80

Ala Val Asn Val Ala Gln Val Ile Asp Ser Glu Thr Ala Asp Asn Leu
                85                  90                  95

Glu Lys Thr Thr Ala Ala Leu Ser Ile Leu Pro Gly Ile Gly Ser Val
            100                 105                 110

Met Gly Ile Ala Asp Gly Ala Val His His Asn Thr Glu Glu Ile Val
        115                 120                 125

Ala Gln Ser Ile Ala Leu Ser Ser Leu Met Val Ala Gln Ala Ile Pro
    130                 135                 140

Leu Val Gly Glu Leu Val Asp Ile Gly Phe Ala Ala Tyr Asn Phe Val
145                 150                 155                 160

Glu Ser Ile Ile Asn Leu Phe Gln Val Val His Asn Ser Tyr Asn Arg
                165                 170                 175

Pro Met Val Arg Ser Ser Ser Arg Thr Pro Ser Asp Lys Pro Val Ala
            180                 185                 190

His Val Val Ala Asn Pro Gln Ala Glu Gly Gln Leu Gln Trp Leu Asn
        195                 200                 205

Arg Arg Ala Asn Ala Leu Leu Ala Asn Gly Val Glu Leu Arg Asp Asn
    210                 215                 220

Gln Leu Val Val Pro Ser Glu Gly Leu Tyr Leu Ile Tyr Ser Gln Val
225                 230                 235                 240

Leu Phe Lys Gly Gln Gly Cys Pro Ser Thr His Val Leu Leu Thr His
                245                 250                 255

Thr Ile Ser Arg Ile Ala Val Ser His Gln Thr Lys Val Asn Leu Leu
            260                 265                 270

Ser Ala Ile Lys Ser Pro Cys Gln Arg Glu Thr Pro Glu Gly Ala Glu
        275                 280                 285

Ala Lys Pro Trp Tyr Glu Pro Ile Tyr Leu Gly Gly Val Phe Gln Leu
    290                 295                 300

Glu Lys Gly Asp Arg Leu Ser Ala Glu Ile Asn Arg Pro Asp Tyr Leu
305                 310                 315                 320

Asp Phe Arg Glu Ser Gly Gln Val Tyr Phe Gly Ile Ile Ala Leu
                325                 330                 335

<210>  12
<211>  158
<212>  PRT
<213>  Artificial sequence

<220>
<223>  Polypeptide

<400>  12

Met Val Arg Ser Ser Ser Arg Thr Pro Ser Asp Lys Pro Val Ala His
1               5                   10                  15

Val Val Ala Asn Pro Gln Ala Glu Gly Gln Leu Gln Trp Leu Asn Arg
            20                  25                  30

Arg Ala Asn Ala Leu Leu Ala Asn Gly Val Glu Leu Arg Asp Asn Gln
        35                  40                  45

Leu Val Val Pro Ser Glu Gly Leu Tyr Leu Ile Tyr Ser Gln Val Leu
    50                  55                  60

Phe Lys Gly Gln Gly Cys Pro Ser Thr His Val Leu Leu Thr His Thr
65                  70                  75                  80

Ile Ser Arg Ile Ala Val Ser His Gln Thr Lys Val Asn Leu Leu Ser

85                  90                  95

Ala Ile Lys Ser Pro Cys Gln Arg Glu Thr Pro Glu Gly Ala Glu Ala
            100                 105                 110

Lys Pro Trp Tyr Glu Pro Ile Tyr Leu Gly Gly Val Phe Gln Leu Glu
        115                 120                 125

Lys Gly Asp Arg Leu Ser Ala Glu Ile Asn Arg Pro Asp Tyr Leu Asp
    130                 135                 140

Phe Arg Glu Ser Gly Gln Val Tyr Phe Gly Ile Ile Ala Leu
145                 150                 155

<210>  13
<211>  180
<212>  PRT
<213>  Artificial sequence

<220>
<223>  Polypeptide

<400>  13

Ile Asn Leu Asp Trp Asp Val Ile Arg Asp Lys Thr Lys Thr Lys Ile
1               5                   10                  15

Glu Ser Leu Lys Glu His Gly Pro Ile Lys Asn Lys Met Ser Glu Ser
            20                  25                  30

Pro Asn Lys Thr Val Ser Glu Glu Lys Ala Lys Gln Tyr Leu Glu Glu
        35                  40                  45

Phe His Gln Thr Ala Leu Glu His Pro Glu Leu Ser Glu Leu Lys Thr
    50                  55                  60

Val Thr Gly Thr Asn Pro Val Phe Ala Gly Ala Asn Tyr Ala Ala Trp
65                  70                  75                  80

Ala Val Asn Val Ala Gln Val Ile Asp Ser Glu Thr Ala Asp Asn Leu
                85                  90                  95

Glu Lys Thr Thr Ala Ala Leu Leu Asp Phe Ala Glu Ser Gly Gln Val
            100                 105                 110

Gly Ser Val Met Gly Ile Ala Asp Gly Ala Val His His Asn Thr Glu
        115                 120                 125

Glu Ile Val Ala Gln Ser Ile Ala Leu Ser Ser Leu Met Val Ala Gln
    130                 135                 140

Ala Ile Pro Leu Val Gly Glu Leu Val Asp Ile Gly Phe Ala Ala Tyr
145                 150                 155                 160

Asn Phe Val Glu Ser Ile Ile Asn Leu Phe Gln Val Val His Asn Ser
                165                 170                 175

Tyr Asn Arg Pro
            180

<210>  14
<211>  1002
<212>  DNA
<213>  Artificial sequence

<220>
<223>  Polynucleotide

<400>  14
ataaatcttg attgggatgt cataagggat aaaactaaga caaagataga gtctttgaaa      60

gagcatggcc ctatcaaaaa taaaatgagc gaaagtccca ataaaacagt atctgaggaa     120

aaagctaaac aatacctaga agaatttcat caaacggcat tagagcatcc tgaattgtca     180

gaacttaaaa ccgttactgg gaccaatcct gtattcgctg gggctaacta tgcggcgtgg     240

gcagtaaacg ttgcgcaagt tatcgatagc gaaacagctg ataatttgga aaagacaact     300

gctgctcttt cgatacttcc tggtatcggt agcgtaatgg gcattgcaga cggtgccgtt     360

caccacaata cagaagagat agtggcacaa tcaatagctt tatcatcttt aatggttgct     420

caagctattc cattggtagg agagctagtt gatattggtt tcgctgcata taattttgta     480

gagagtatta tcaatttatt tcaagtagtt cataattcgt ataatcgtcc catgctgcgt     540

agcagcagcc agaatagcag cgataaaccg gtggcgcatg tggtggcgaa tcatcaggtg     600

gaagaacagc tggaatggct gagccagcgt gcgaacgcgc tgctggcgaa cggcatggat     660

ctgaaagaca atcagcttgt tgtaccagcc gatggcctgt atttggtgta cagtcaagtg     720

ctgttcaaag gccagggctg cccggattac gtgcttctga cgcataccgt gtcgcgcttc     780

gcgattagcc atcaggaaaa ggtgaatctg ctgtcggcgg tcaaaagccc gtgtccgaaa     840

gataccccgg agggggctga attgaaaccg tggtatgaac cgatctattt gggtggcgtg     900

tttcaactgg aaaaagggga ccagctttcc gctgaggtaa acctgcccaa gtatctggat     960

tttcgggaga gcgggcaagt gtattttggc gtgattgcgc tg                       1002

<210>  15
<211>  1008
<212>  DNA
<213>  Artificial sequence

<220>
<223>  Polynucleotide

<400>  15
ataaatcttg attgggatgt cataagggat aaaactaaga caaagataga gtctttgaaa      60

gagcatggcc ctatcaaaaa taaaatgagc gaaagtccca ataaaacagt atctgaggaa     120

aaagctaaac aatacctaga agaatttcat caaacggcat tagagcatcc tgaattgtca     180

gaacttaaaa ccgttactgg gaccaatcct gtattcgctg gggctaacta tgcggcgtgg     240

gcagtaaacg ttgcgcaagt tatcgatagc gaaacagctg ataatttgga aaagacaact     300

gctgctcttt cgatacttcc tggtatcggt agcgtaatgg gcattgcaga cggtgccgtt     360

caccacaata cagaagagat agtggcacaa tcaatagctt tatcatcttt aatggttgct     420

caagctattc cattggtagg agagctagtt gatattggtt tcgctgcata taattttgta     480

gagagtatta tcaatttatt tcaagtagtt cataattcgt ataatcgtcc catggtcaga     540

tcatcttctc gaaccccgag tgacaagcct gtagcccatg ttgtagcaaa ccctcaagct     600

gaggggcagc tccagtggct gaaccgccgg gccaatgccc tcctggccaa tggcgtggag     660

ctgagagata accagctggt ggtgccatca gagggcctgt acctcatcta ctcccaggtc     720

ctcttcaagg gccaaggctg cccctccacc catgtgctcc tcacccacac catcagccgc     780

atcgccgtct cccaccagac caaggtcaac ctcctctctg ccatcaagag cccctgccag     840

agggagaccc cagagggggc tgaggccaag ccctggtatg agcccatcta tctgggaggg     900

gtcttccagc tggagaaggg tgaccgactc agcgctgaga tcaatcggcc cgactatctc     960

gactttcgcg agtctgggca ggtctacttt gggatcattg ccctgtga                 1008

<210>  16
<211>  563
<212>  DNA
<213>  Artificial sequence

<220>
<223>  Polynucleotide

<400>  16
ataaatcttg attgggatgt cataagggat aaaactaaga caaagataga gtctttgaaa      60

gagcatggcc ctatcaaaaa taaaatgagc gaaagtccca ataaaacagt atctgaggaa     120

aaagctaaac aatacctaga agaatttcat caaacggcat tagagcatcc tgaattgtca     180

gaacttaaaa ccgttactgg gaccaatcct gtattcgctg gggctaacta tgcggcgtgg     240

gcagtaaacg ttgcgcaagt tatcgatgtc agccgatttg ctatctcata ccaggagaaa     300

gtcaacctcc tctctgcgtc gaaaagacaa ctgctgctct ttcgatactt cctggtatcg     360

gtagcgtaat gggcattgca gacggtgccg ttcaccacaa tacagaagag atagtggcac     420

aatcaatagc tttatcatct ttaatggttg ctcaagctat tccattggta ggagagctag     480

ttgatattgg tttcgctgca tataattttg tagagagtat tatcaattta tttcaagtag 540

ttcataattc gtataatcgt ccc 563

<210> 17
<211> 540
<212> DNA
<213> Artificial sequence

<220>
<223> Polynucleotide

<400> 17
ataaatcttg attgggatgt cataagggat aaaactaaga caaagataga gtctttgaaa 60

gagcatggcc ctatcaaaaa taaaatgagc gaaagtccca ataaaacagt atctgaggaa 120

aaagctaaac aatacctaga agaatttcat caaacggcat tagagcatcc tgaattgtca 180

gaacttaaaa ccgttactgg gaccaatcct gtattcgctg gggctaacta tgcggcgtgg 240

gcagtaaacg ttgcgcaagt tatcgatagc gaaacagctg ataatttgga aaagacaact 300

gctgctcttt tagactttgc ggagtccggg caggtcggta gcgtaatggg cattgcagac 360

ggtgccgttc accacaatac agaagagata gtggcacaat caatagcttt atcatcttta 420

atggttgctc aagctattcc attggtagga gagctagttg atattggttt cgctgcatat 480

aattttgtag agagtattat caatttattt caagtagttc ataattcgta taatcgtccc 540

<210> 18
<211> 54
<212> DNA
<213> Artificial sequence

<220>
<223> Polynucleotide

<400> 18
cgcggatccc tggaagttct gttccagggg cccataaatc ttgattggga tgtc 54

<210> 19
<211> 33
<212> DNA
<213> Artificial sequence

<220>
<223> Polynucleotide

<400> 19
ccgctcgagc tagggacgat tatacgaatt atg 33

<210> 20
<211> 19
<212> DNA
<213> Artificial sequence

<220>
<223>  Polynucleotide

<400>  20
gaaaagacaa ctgctgctc                                                    19


<210>  21
<211>  36
<212>  DNA
<213>  Artificial sequence

<220>
<223>  Polynucleotide

<400>  21
tcaacctcct ctctgccgaa aagacaactg ctgctc                                 36


<210>  22
<211>  52
<212>  DNA
<213>  Artificial sequence

<220>
<223>  Polynucleotide

<400>  22
tcctggtatg agatagcaaa tcggctgaca gctgtttcgc tatcgataac tt               52


<210>  23
<211>  25
<212>  DNA
<213>  Artificial sequence

<220>
<223>  Polynucleotide

<400>  23
agctgtttcg ctatcgataa cttgc                                             25


<210>  24
<211>  24
<212>  DNA
<213>  Artificial sequence

<220>
<223>  Polynucleotide

<400>  24
ggtagcgtaa tgggcattgc agac                                              24


<210>  25
<211>  49
<212>  DNA
<213>  Artificial sequence

<220>
<223>  Polynucleotide

<400>  25
ttagactttg cggagtccgg gcaggtcggt agcgtaatgg gcattgcag                    49


<210>  26
<211>  54
<212>  DNA
<213>  Artificial sequence

<220>
<223>  Polynucleotide

<400>  26
gacctgcccg gactccgcaa agtctaaaag agcagcagtt gtcttttcca aatt              54


<210>  27
<211>  22
<212>  PRT
<213>  Artificial sequence

<220>
<223>  Polypeptide

<400>  27

Ala Ala Gly Ala Gly Cys Ala Gly Cys Ala Gly Thr Thr Gly Thr Cys
1               5                   10                  15


Thr Thr Thr Thr Cys Cys
            20


<210>  28
<211>  29
<212>  DNA
<213>  Artificial sequence

<220>
<223>  Polynucleotide

<400>  28
gaaaagacaa ctgctgctct ttcgatact                                          29


<210>  29
<211>  53
<212>  DNA
<213>  Artificial sequence

<220>
<223>  Polynucleotide

<400>  29
tggctgagcc agcgcgccaa cgccgaaaag acaactgctg ctctttcgat act               53


<210>  30
<211>  46
<212>  DNA
<213>  Artificial sequence

<220>
<223>  Polynucleotide

<400>  30
ggcgttggcg cgctggctca gccaatcgat aacttgcgca acgttt                    46


<210>  31
<211>  20
<212>  DNA
<213>  Artificial sequence

<220>
<223>  Polynucleotide

<400>  31
atcgataact tgcgcaacgt                                                 20


<210>  32
<211>  46
<212>  DNA
<213>  Artificial sequence

<220>
<223>  Polynucleotide

<400>  32
cactagttgg ttgtctttga gatccatgcc agctgtttcg ctatcg                    46


<210>  33
<211>  47
<212>  DNA
<213>  Artificial sequence

<220>
<223>  Polynucleotide

<400>  33
ggatctcaaa gacaaccaac tagtggtgga aaagacaact gctgctc                   47


<210>  34
<211>  18
<212>  PRT
<213>  Artificial sequence

<220>
<223>  Polypeptide

<400>  34

Val Ser Arg Phe Ala Ile Ser Tyr Gln Glu Lys Val Asn Leu Leu Ser
1               5                   10                  15


Ala Val



<210>  35
<211>  187

<212>  PRT
<213>  Artificial sequence

<220>
<223>  Polypeptide

<400>  35

Ile Asn Leu Asp Trp Asp Val Ile Arg Asp Lys Thr Lys Thr Lys Ile
1               5                   10                  15

Glu Ser Leu Lys Glu His Gly Pro Ile Lys Asn Lys Met Ser Glu Ser
            20                  25                  30

Pro Asn Lys Thr Val Ser Glu Glu Lys Ala Lys Gln Tyr Leu Glu Glu
        35                  40                  45

Phe His Gln Thr Ala Leu Glu His Pro Glu Leu Ser Glu Leu Lys Thr
    50                  55                  60

Val Thr Gly Thr Asn Pro Val Phe Ala Gly Ala Asn Tyr Ala Ala Trp
65                  70                  75                  80

Ala Val Asn Val Ala Gln Val Ile Ile Ser Arg Ile Ala Val Ser Tyr
                85                  90                  95

Gln Thr Lys Val Asn Leu Leu Ser Ala Ile Glu Lys Thr Thr Ala Ala
            100                 105                 110

Leu Ser Ile Leu Pro Gly Ile Gly Ser Val Met Gly Ile Ala Asp Gly
        115                 120                 125

Ala Val His His Asn Thr Glu Glu Ile Val Ala Gln Ser Ile Ala Leu
    130                 135                 140

Ser Ser Leu Met Val Ala Gln Ala Ile Pro Leu Val Gly Glu Leu Val
145                 150                 155                 160

Asp Ile Gly Phe Ala Ala Tyr Asn Phe Val Glu Ser Ile Ile Asn Leu
                165                 170                 175

Phe Gln Val Val His Asn Ser Tyr Asn Arg Pro
            180                 185


<210>  36
<211>  561
<212>  DNA
<213>  Artificial sequence

<220>
<223>  Polynucleotide

<400>  36
ataaatcttg attgggatgt cataagggat aaaactaaga caaagataga gtctttgaaa      60

gagcatggcc ctatcaaaaa taaaatgagc gaaagtccca ataaaacagt atctgaggaa     120

aaagctaaac aatacctaga agaatttcat caaacggcat tagagcatcc tgaattgtca     180

gaacttaaaa ccgttactgg gaccaatcct gtattcgctg gggctaacta tgcggcgtgg     240

gcagtaaacg ttgcgcaagt tatcatcagc cgcatcgccg tctcctacca gaccaaggtc     300

aacctcctct ctgccatcga aaagacaact gctgctcttt cgatacttcc tggtatcggt     360

agcgtaatgg gcattgcaga cggtgccgtt caccacaata cagaagagat agtggcacaa     420

tcaatagctt tatcatcttt aatggttgct caagctattc cattggtagg agagctagtt     480

gatattggtt tcgctgcata taattttgta gagagtatta tcaatttatt tcaagtagtt     540

cataattcgt ataatcgtcc c                                               561


<210>  37
<211>  187
<212>  PRT
<213>  Artificial sequence

<220>
<223>  Polypeptide

<400>  37

Ile Asn Leu Asp Trp Asp Val Ile Arg Asp Lys Thr Lys Thr Lys Ile
1               5                   10                  15


Glu Ser Leu Lys Glu His Gly Pro Ile Lys Asn Lys Met Ser Glu Ser
            20                  25                  30


Pro Asn Lys Thr Val Ser Glu Glu Lys Ala Lys Gln Tyr Leu Glu Glu
        35                  40                  45


Phe His Gln Thr Ala Leu Glu His Pro Glu Leu Ser Glu Leu Lys Thr
    50                  55                  60


Val Thr Gly Thr Asn Pro Val Phe Ala Gly Ala Asn Tyr Ala Ala Trp
65                  70                  75                  80


Ala Val Asn Val Ala Gln Val Ile Val Ser Arg Phe Ala Ile Ser Tyr
                85                  90                  95


Gln Glu Lys Val Asn Leu Leu Ser Ala Val Glu Lys Thr Thr Ala Ala
            100                 105                 110


Leu Ser Ile Leu Pro Gly Ile Gly Ser Val Met Gly Ile Ala Asp Gly
        115                 120                 125

Ala Val His His Asn Thr Glu Glu Ile Val Ala Gln Ser Ile Ala Leu
    130                 135                 140

Ser Ser Leu Met Val Ala Gln Ala Ile Pro Leu Val Gly Glu Leu Val
145                 150                 155                 160

Asp Ile Gly Phe Ala Ala Tyr Asn Phe Val Glu Ser Ile Ile Asn Leu
                165                 170                 175

Phe Gln Val Val His Asn Ser Tyr Asn Arg Pro
            180                 185

<210>  38
<211>  561
<212>  DNA
<213>  Artificial sequence

<220>
<223>  Polynucleotide

<400>  38
ataaatcttg attgggatgt cataagggat aaaactaaga caaagataga gtctttgaaa      60

gagcatggcc ctatcaaaaa taaaatgagc gaaagtccca ataaaacagt atctgaggaa     120

aaagctaaac aatacctaga agaatttcat caaacggcat tagagcatcc tgaattgtca     180

gaacttaaaa ccgttactgg gaccaatcct gtattcgctg gggctaacta tgcggcgtgg     240

gcagtaaacg ttgcgcaagt tatcgtcagc cgatttgcta tctcatacca ggagaaagtc     300

aacctcctct ctgccgtcga aaagacaact gctgctcttt cgatacttcc tggtatcggt     360

agcgtaatgg gcattgcaga cggtgccgtt caccacaata cagaagagat agtggcacaa     420

tcaatagctt tatcatcttt aatggttgct caagctattc cattggtagg agagctagtt     480

gatattggtt tcgctgcata taattttgta gagagtatta tcaatttatt tcaagtagtt     540

cataattcgt ataatcgtcc c                                               561

<210>  39
<211>  187
<212>  PRT
<213>  Artificial sequence

<220>
<223>  Polypeptide

<400>  39

Ile Asn Leu Asp Trp Asp Val Ile Arg Asp Lys Thr Lys Thr Lys Ile
1               5                   10                  15

Glu Ser Leu Lys Glu His Gly Pro Ile Lys Asn Lys Met Ser Glu Ser

20                  25                  30

Pro Asn Lys Thr Val Ser Glu Glu Lys Ala Lys Gln Tyr Leu Glu Glu
        35                  40                  45

Phe His Gln Thr Ala Leu Glu His Pro Glu Leu Ser Glu Leu Lys Thr
    50                  55                  60

Val Thr Gly Thr Asn Pro Val Phe Ala Gly Ala Asn Tyr Ala Ala Trp
65                  70                  75                  80

Ala Val Asn Val Ala Gln Val Ile Asp Val Ser Arg Phe Ala Ile Ser
                85                  90                  95

Tyr Gln Glu Lys Val Asn Leu Leu Ser Ala Glu Lys Thr Thr Ala Ala
            100                 105                 110

Leu Ser Ile Leu Pro Gly Ile Gly Ser Val Met Gly Ile Ala Asp Gly
        115                 120                 125

Ala Val His His Asn Thr Glu Glu Ile Val Ala Gln Ser Ile Ala Leu
    130                 135                 140

Ser Ser Leu Met Val Ala Gln Ala Ile Pro Leu Val Gly Glu Leu Val
145                 150                 155                 160

Asp Ile Gly Phe Ala Ala Tyr Asn Phe Val Glu Ser Ile Ile Asn Leu
                165                 170                 175

Phe Gln Val Val His Asn Ser Tyr Asn Arg Pro
            180                 185

<210>  40
<211>  16
<212>  PRT
<213>  Homo sapiens

<400>  40

Thr Asn Glu Glu Cys Gln Lys Arg Tyr Arg Gly His Lys Ile Thr His
1               5                   10                  15

<210>  41
<211>  18
<212>  PRT
<213>  Homo sapiens

<400>  41

Thr Thr Lys Ile Lys Pro Arg Ile Val Gly Gly Thr Ala Ser Val Arg
1               5                   10                  15

Gly Glu

<210> 42
<211> 186
<212> PRT
<213> Artificial sequence

<220>
<223> Polypeptide

<400> 42

Ile Asn Leu Asp Trp Asp Val Ile Arg Asp Lys Thr Lys Thr Lys Ile
1               5                   10                  15

Glu Ser Leu Lys Glu His Gly Pro Ile Lys Asn Lys Met Ser Glu Ser
            20                  25                  30

Pro Asn Lys Thr Val Ser Glu Glu Lys Ala Lys Gln Tyr Leu Glu Glu
        35                  40                  45

Phe His Gln Thr Ala Leu Glu His Pro Glu Leu Ser Glu Leu Lys Thr
    50                  55                  60

Val Thr Gly Thr Asn Pro Val Phe Ala Gly Ala Asn Tyr Ala Ala Trp
65                  70                  75                  80

Ala Val Asn Val Ala Gln Val Ile Asp Thr Asn Glu Glu Cys Gln Lys
                85                  90                  95

Arg Tyr Arg Gly His Lys Ile Thr His Glu Lys Thr Thr Ala Ala Leu
            100                 105                 110

Ser Ile Leu Pro Gly Ile Gly Ser Val Met Gly Ile Ala Asp Gly Ala
        115                 120                 125

Val His His Asn Thr Glu Glu Ile Val Ala Gln Ser Ile Ala Leu Ser
    130                 135                 140

Ser Leu Met Val Ala Gln Ala Ile Pro Leu Val Gly Glu Leu Val Asp
145                 150                 155                 160

Ile Gly Phe Ala Ala Tyr Asn Phe Val Glu Ser Ile Ile Asn Leu Phe
                165                 170                 175

Gln Val Val His Asn Ser Tyr Asn Arg Pro
            180                 185

<210>  43
<211>  188
<212>  PRT
<213>  Artificial sequence

<220>
<223>  Polypeptide

<400>  43

Ile Asn Leu Asp Trp Asp Val Ile Arg Asp Lys Thr Lys Thr Lys Ile
1               5                   10                  15

Glu Ser Leu Lys Glu His Gly Pro Ile Lys Asn Lys Met Ser Glu Ser
            20                  25                  30

Pro Asn Lys Thr Val Ser Glu Glu Lys Ala Lys Gln Tyr Leu Glu Glu
        35                  40                  45

Phe His Gln Thr Ala Leu Glu His Pro Glu Leu Ser Glu Leu Lys Thr
    50                  55                  60

Val Thr Gly Thr Asn Pro Val Phe Ala Gly Ala Asn Tyr Ala Ala Trp
65                  70                  75                  80

Ala Val Asn Val Ala Gln Val Ile Asp Thr Thr Lys Ile Lys Pro Arg
                85                  90                  95

Ile Val Gly Gly Thr Ala Ser Val Arg Gly Glu Glu Lys Thr Thr Ala
            100                 105                 110

Ala Leu Ser Ile Leu Pro Gly Ile Gly Ser Val Met Gly Ile Ala Asp
        115                 120                 125

Gly Ala Val His His Asn Thr Glu Glu Ile Val Ala Gln Ser Ile Ala
    130                 135                 140

Leu Ser Ser Leu Met Val Ala Gln Ala Ile Pro Leu Val Gly Glu Leu
145                 150                 155                 160

Val Asp Ile Gly Phe Ala Ala Tyr Asn Phe Val Glu Ser Ile Ile Asn
                165                 170                 175

Leu Phe Gln Val Val His Asn Ser Tyr Asn Arg Pro
            180                 185


<210>  44
<211>  422
<212>  PRT
<213>  Artificial sequence

<220>
<223>  Polypeptide

<400>  44

Ile Asn Leu Asp Trp Asp Val Ile Arg Asp Lys Thr Lys Thr Lys Ile
1               5                   10                  15

Glu Ser Leu Lys Glu His Gly Pro Ile Lys Asn Lys Met Ser Glu Ser
            20                  25                  30

Pro Asn Lys Thr Val Ser Glu Glu Lys Ala Lys Gln Tyr Leu Glu Glu
        35                  40                  45

Phe His Gln Thr Ala Leu Glu His Pro Glu Leu Ser Glu Leu Lys Thr
    50                  55                  60

Val Thr Gly Thr Asn Pro Val Phe Ala Gly Ala Asn Tyr Ala Ala Trp
65                  70                  75                  80

Ala Val Asn Val Ala Gln Val Ile Asp Ser Glu Thr Ala Asp Asn Leu
                85                  90                  95

Glu Lys Thr Thr Ala Ala Leu Ser Ile Leu Pro Gly Ile Gly Ser Val
            100                 105                 110

Met Gly Ile Ala Asp Gly Ala Val His His Asn Thr Glu Glu Ile Val
        115                 120                 125

Ala Gln Ser Ile Ala Leu Ser Ser Leu Met Val Ala Gln Ala Ile Pro
    130                 135                 140

Leu Val Gly Glu Leu Val Asp Ile Gly Phe Ala Ala Tyr Asn Phe Val
145                 150                 155                 160

Glu Ser Ile Ile Asn Leu Phe Gln Val Val His Asn Ser Tyr Asn Arg
                165                 170                 175

Pro Thr Thr Lys Ile Lys Pro Arg Ile Val Gly Gly Thr Ala Ser Val
            180                 185                 190

Arg Gly Glu Trp Pro Trp Gln Val Thr Leu His Thr Thr Ser Pro Thr
        195                 200                 205

Gln Arg His Leu Cys Gly Gly Ser Ile Ile Gly Asn Gln Trp Ile Leu
    210                 215                 220

Thr Ala Ala His Cys Phe Tyr Gly Val Glu Ser Pro Lys Ile Leu Arg

225                 230                 235                 240

Val Tyr Ser Gly Ile Leu Asn Gln Ser Glu Ile Lys Glu Asp Thr Ser
                245                 250                 255

Phe Phe Gly Val Gln Glu Ile Ile Ile His Asp Gln Tyr Lys Met Ala
            260                 265                 270

Glu Ser Gly Tyr Asp Ile Ala Leu Leu Lys Leu Glu Thr Thr Val Asn
        275                 280                 285

Tyr Thr Asp Ser Gln Arg Pro Ile Cys Leu Pro Ser Lys Gly Asp Arg
    290                 295                 300

Asn Val Ile Tyr Thr Asp Cys Trp Val Thr Gly Trp Gly Tyr Arg Lys
305                 310                 315                 320

Leu Arg Asp Lys Ile Gln Asn Thr Leu Gln Lys Ala Lys Ile Pro Leu
                325                 330                 335

Val Thr Asn Glu Glu Cys Gln Lys Arg Tyr Arg Gly His Lys Ile Thr
            340                 345                 350

His Lys Met Ile Cys Ala Gly Tyr Arg Glu Gly Gly Lys Asp Ala Cys
        355                 360                 365

Lys Gly Asp Ser Gly Gly Pro Leu Ser Cys Lys His Asn Glu Val Trp
    370                 375                 380

His Leu Val Gly Ile Thr Ser Trp Gly Glu Gly Cys Ala Gln Arg Glu
385                 390                 395                 400

Arg Pro Gly Val Tyr Thr Asn Val Val Glu Tyr Val Asp Trp Ile Leu
                405                 410                 415

Glu Lys Thr Gln Ala Val
            420

<210>  45
<211>  245
<212>  PRT
<213>  Homo sapiens

<400>  45

Thr Thr Lys Ile Lys Pro Arg Ile Val Gly Gly Thr Ala Ser Val Arg
1               5                   10                  15

Gly Glu Trp Pro Trp Gln Val Thr Leu His Thr Thr Ser Pro Thr Gln

20                  25                  30

Arg His Leu Cys Gly Gly Ser Ile Ile Gly Asn Gln Trp Ile Leu Thr
        35                  40                  45

Ala Ala His Cys Phe Tyr Gly Val Glu Ser Pro Lys Ile Leu Arg Val
    50                  55                  60

Tyr Ser Gly Ile Leu Asn Gln Ser Glu Ile Lys Glu Asp Thr Ser Phe
65                  70                  75                  80

Phe Gly Val Gln Glu Ile Ile Ile His Asp Gln Tyr Lys Met Ala Glu
                85                  90                  95

Ser Gly Tyr Asp Ile Ala Leu Leu Lys Leu Glu Thr Thr Val Asn Tyr
            100                 105                 110

Thr Asp Ser Gln Arg Pro Ile Cys Leu Pro Ser Lys Gly Asp Arg Asn
        115                 120                 125

Val Ile Tyr Thr Asp Cys Trp Val Thr Gly Trp Gly Tyr Arg Lys Leu
    130                 135                 140

Arg Asp Lys Ile Gln Asn Thr Leu Gln Lys Ala Lys Ile Pro Leu Val
145                 150                 155                 160

Thr Asn Glu Glu Cys Gln Lys Arg Tyr Arg Gly His Lys Ile Thr His
                165                 170                 175

Lys Met Ile Cys Ala Gly Tyr Arg Glu Gly Gly Lys Asp Ala Cys Lys
            180                 185                 190

Gly Asp Ser Gly Gly Pro Leu Ser Cys Lys His Asn Glu Val Trp His
        195                 200                 205

Leu Val Gly Ile Thr Ser Trp Gly Glu Gly Cys Ala Gln Arg Glu Arg
    210                 215                 220

Pro Gly Val Tyr Thr Asn Val Val Glu Tyr Val Asp Trp Ile Leu Glu
225                 230                 235                 240

Lys Thr Gln Ala Val
                245

<210>  46
<211>  558
<212>  DNA
<213>  Artificial sequence

<220>
<223>  Polynucleotide

<400>  46
ataaatcttg attgggatgt cataagggat aaaactaaga caaagataga gtctttgaaa      60

gagcatggcc ctatcaaaaa taaaatgagc gaaagtccca ataaaacagt atctgaggaa     120

aaagctaaac aatacctaga agaatttcat caaacggcat tagagcatcc tgaattgtca     180

gaacttaaaa ccgttactgg gaccaatcct gtattcgctg gggctaacta tgcggcgtgg     240

gcagtaaacg ttgcgcaagt tatcgatacc aacgaagagt gccagaagag atacagagga     300

cataaaataa cccatgaaaa gacaactgct gctctttcga tacttcctgg tatcggtagc     360

gtaatgggca ttgcagacgg tgccgttcac cacaatacag aagagatagt ggcacaatca     420

atagctttat catctttaat ggttgctcaa gctattccat tggtaggaga gctagttgat     480

attggtttcg ctgcatataa ttttgtagag agtattatca atttatttca agtagttcat     540

aattcgtata atcgtccc                                                   558


<210>  47
<211>  564
<212>  DNA
<213>  Artificial sequence

<220>
<223>  Polynucleotide

<400>  47
ataaatcttg attgggatgt cataagggat aaaactaaga caaagataga gtctttgaaa      60

gagcatggcc ctatcaaaaa taaaatgagc gaaagtccca ataaaacagt atctgaggaa     120

aaagctaaac aatacctaga agaatttcat caaacggcat tagagcatcc tgaattgtca     180

gaacttaaaa ccgttactgg gaccaatcct gtattcgctg gggctaacta tgcggcgtgg     240

gcagtaaacg ttgcgcaagt tatcgatacc accaaaatca agcccaggat cgttggagga     300

actgcgtctg ttcgtggtga ggaaaagaca actgctgctc tttcgatact tcctggtatc     360

ggtagcgtaa tgggcattgc agacggtgcc gttcaccaca atacagaaga gatagtggca     420

caatcaatag ctttatcatc tttaatggtt gctcaagcta ttccattggt aggagagcta     480

gttgatattg gtttcgctgc atataatttt gtagagagta ttatcaattt atttcaagta     540

gttcataatt cgtataatcg tccc                                            564


<210>  48
<211>  1266
<212>  DNA
<213>  Artificial sequence

<220>
<223>  Polynucleotide

<400>  48
   ataaatcttg attgggatgt cataagggat aaaactaaga caaagataga gtctttgaaa      60
   gagcatggcc ctatcaaaaa taaaatgagc gaaagtccca ataaaacagt atctgaggaa     120
   aaagctaaac aatacctaga agaatttcat caaacggcat tagagcatcc tgaattgtca     180
   gaacttaaaa ccgttactgg gaccaatcct gtattcgctg gggctaacta tgcggcgtgg     240
   gcagtaaacg ttgcgcaagt tatcgatagc gaaacagctg ataatttgga aaagacaact     300
   gctgctcttt cgatacttcc tggtatcggt agcgtaatgg gcattgcaga cggtgccgtt     360
   caccacaata cagaagagat agtggcacaa tcaatagctt tatcatcttt aatggttgct     420
   caagctattc cattggtagg agagctagtt gatattggtt tcgctgcata taattttgta     480
   gagagtatta tcaatttatt tcaagtagtt cataattcgt ataatcgtcc caccaccaaa     540
   atcaagccca ggatcgttgg aggaactgcg tctgttcgtg gtgagtggcc gtggcaggtg     600
   accctgcaca caacctcacc cactcagaga cacctgtgtg gaggctccat cattggaaac     660
   cagtggatat taacagccgc tcactgtttc tatggggtag agtcacctaa gattttgcgt     720
   gtctacagtg gcattttaaa tcaatctgaa ataaaagagg acacatcttt ctttggggtt     780
   caagaaataa taatccatga tcagtataaa atggcagaaa gcgggtatga tattgccttg     840
   ttgaaactgg aaaccacagt gaattacaca gattctcaac gacccatatg cctgccttcc     900
   aaaggagata gaaatgtaat atacactgat tgctgggtga ctggatgggg gtacagaaaa     960
   ctaagagaca aaatacaaaa tactctccag aaagccaaga tacccttagt gaccaacgaa    1020
   gagtgccaga agagatacag aggacataaa ataacccata agatgatctg tgccggctac    1080
   agggaaggag ggaaggacgc ttgcaaggga gattcgggag gccctctgtc ctgcaaacac    1140
   aatgaggtct ggcatctggt aggcatcacg agctggggcg aaggctgtgc tcaaagggag    1200
   cggccaggtg tttacaccaa cgtggtcgag tacgtggact ggattctgga gaaaactcaa    1260
   gcagtg                                                                1266

<210>  49
<211>  8
<212>  PRT
<213>  Homo sapiens

<400>  49

Tyr Gly Asp Glu Gln Tyr Pro Arg
1               5

<210>  50
<211>  447
<212>  PRT
<213>  Artificial sequence

<220>
<223>  Polypeptide

<400>  50

Asp Asp Asp Lys Ile Asn Leu Asp Trp Asp Val Ile Arg Asp Lys Thr
1               5                   10                  15

Lys Thr Lys Ile Glu Ser Leu Lys Glu His Gly Pro Ile Lys Asn Lys
            20                  25                  30

Met Ser Glu Ser Pro Asn Lys Thr Val Ser Glu Glu Lys Ala Lys Gln
        35                  40                  45

Tyr Leu Glu Glu Phe His Gln Thr Ala Leu Glu His Pro Glu Leu Ser
    50                  55                  60

Glu Leu Lys Thr Val Thr Gly Thr Asn Pro Val Phe Ala Gly Ala Asn
65                  70                  75                  80

Tyr Ala Ala Trp Ala Val Asn Val Ala Gln Val Ile Asp Ser Glu Thr
                85                  90                  95

Ala Asp Asn Leu Glu Lys Thr Thr Ala Ala Leu Ser Ile Leu Pro Gly
            100                 105                 110

Ile Gly Ser Val Met Gly Ile Ala Asp Gly Ala Val His His Asn Thr
        115                 120                 125

Glu Glu Ile Val Ala Gln Ser Ile Ala Leu Ser Ser Leu Met Val Ala
    130                 135                 140

Gln Ala Ile Pro Leu Val Gly Glu Leu Val Asp Ile Gly Phe Ala Ala
145                 150                 155                 160

Tyr Asn Phe Val Glu Ser Ile Ile Asn Leu Phe Gln Val Val His Asn
                165                 170                 175

Ser Tyr Asn Arg Pro Gly Gly Gly Gly Gly Asn Ile Gln Leu Pro Lys
            180                 185                 190

Glu Glu Ile Lys Lys Leu Glu Val Asp Glu Ile Thr Leu Trp Tyr Lys
        195                 200                 205

Met Ile Leu Pro Pro Gln Phe Asp Arg Ser Lys Lys Tyr Pro Leu Leu
    210                 215                 220

Ile Gln Val Tyr Gly Gly Pro Cys Ser Gln Ser Val Arg Ser Val Phe
225                 230                 235                 240

Ala Val Asn Trp Ile Ser Tyr Leu Ala Ser Lys Glu Gly Met Val Ile
                245                 250                 255

Ala Leu Val Asp Gly Arg Gly Thr Ala Phe Gln Gly Asp Lys Leu Leu
            260                 265                 270

Tyr Ala Val Tyr Arg Lys Leu Gly Val Tyr Glu Val Glu Asp Gln Ile
        275                 280                 285

Thr Ala Val Arg Lys Phe Ile Glu Met Gly Phe Ile Asp Glu Lys Arg
    290                 295                 300

Ile Ala Ile Trp Gly Trp Ser Tyr Gly Gly Tyr Val Ser Ser Leu Ala
305                 310                 315                 320

Leu Ala Ser Gly Thr Gly Leu Phe Lys Cys Gly Ile Ala Val Ala Pro
                325                 330                 335

Val Ser Ser Trp Glu Tyr Tyr Ala Ser Val Tyr Thr Glu Arg Phe Met
            340                 345                 350

Gly Leu Pro Thr Lys Asp Asp Asn Leu Glu His Tyr Lys Asn Ser Thr
        355                 360                 365

Val Met Ala Arg Ala Glu Tyr Phe Arg Asn Val Asp Tyr Leu Leu Ile
    370                 375                 380

His Gly Thr Ala Asp Asp Asn Val His Phe Gln Asn Ser Ala Gln Ile
385                 390                 395                 400

Ala Lys Ala Leu Val Asn Ala Gln Val Asp Phe Gln Ala Met Trp Tyr
                405                 410                 415

Ser Asp Gln Asn His Gly Leu Ser Gly Leu Ser Thr Asn His Leu Tyr
            420                 425                 430

Thr His Met Thr His Phe Leu Lys Gln Cys Phe Ser Leu Ser Asp
        435                 440                 445

<210>  51
<211>  177
<212>  PRT
<213>  Artificial sequence

<220>
<223>  Polypeptide

<400>  51

Ile Asn Leu Asp Trp Asp Val Ile Arg Asp Lys Thr Lys Thr Lys Ile
1               5                   10                  15

Glu Ser Leu Lys Glu His Gly Pro Ile Lys Asn Lys Met Ser Glu Ser
            20                  25                  30

Pro Asn Lys Thr Val Ser Glu Glu Lys Ala Lys Gln Tyr Leu Glu Glu
        35                  40                  45

Phe His Gln Thr Ala Leu Glu His Pro Glu Leu Ser Glu Leu Lys Thr
    50                  55                  60

Val Thr Gly Thr Asn Pro Val Phe Ala Gly Ala Asn Tyr Ala Ala Trp
65                  70                  75                  80

Ala Val Asn Val Ala Gln Val Ile Asp Tyr Gly Asp Glu Gln Tyr Pro
                85                  90                  95

Arg Lys Thr Thr Ala Ala Leu Ser Ile Leu Pro Gly Ile Gly Ser Val
            100                 105                 110

Met Gly Ile Ala Asp Gly Ala Val His His Asn Thr Glu Glu Ile Val
        115                 120                 125

Ala Gln Ser Ile Ala Leu Ser Ser Leu Met Val Ala Gln Ala Ile Pro
    130                 135                 140

Leu Val Gly Glu Leu Val Asp Ile Gly Phe Ala Ala Tyr Asn Phe Val
145                 150                 155                 160

Glu Ser Ile Ile Asn Leu Phe Gln Val Val His Asn Ser Tyr Asn Arg
                165                 170                 175

Pro

<210>  52
<211>  1344
<212>  DNA
<213>  Artificial sequence

<220>
<223>  Polynucleotide

<400>  52
gatgatgatg ataagataaa tcttgattgg gatgtcataa gggataaaac taagacaaag      60

atagagtctt tgaaagagca tggccctatc aaaaataaaa tgagcgaaag tcccaataaa     120

acagtatctg aggaaaaagc taaacaatac ctagaagaat ttcatcaaac ggcattagag     180

catcctgaat tgtcagaact taaaaccgtt actgggacca atcctgtatt cgctggggct 240

aactatgcgg cgtgggcagt aaacgttgcg caagttatcg atagcgaaac agctgataat 300

ttggaaaaga caactgctgc tctttcgata cttcctggta tcggtagcgt aatgggcatt 360

gcagacggtg ccgttcacca caatacagaa gagatagtgg cacaatcaat agctttatca 420

tctttaatgg ttgctcaagc tattccattg gtaggagagc tagttgatat tggtttcgct 480

gcatataatt ttgtagagag tattatcaat ttatttcaag tagttcataa ttcgtataat 540

cgtcccggtg gtggtggtgg taatatccag ctgcctaaag aggaaattaa gaaacttgaa 600

gtagatgaaa ttactttatg gtacaagatg attcttcctc ctcaatttga cagatcaaag 660

aagtatccct tgctaattca agtgtatggt ggtccctgca gtcagagtgt aaggtctgta 720

tttgctgtta attggatatc ttatcttgca agtaaggaag ggatggtcat tgccttggtg 780

gatggtcgag gaacagcttt ccaaggtgac aaactcctct atgcagtgta tcgaaagctg 840

ggtgtttatg aagttgaaga ccagattaca gctgtcagaa aattcataga aatgggtttc 900

attgatgaaa aaagaatagc catatggggc tggtcctatg gaggatacgt ttcatcactg 960

gcccttgcat ctggaactgg tcttttcaaa tgtggtatag cagtggctcc agtctccagc 1020

tgggaatatt acgcgtctgt ctacacagag agattcatgg gtctcccaac aaaggatgat 1080

aatcttgagc actataagaa ttcaactgtg atggcaagag cagaatattt cagaaatgta 1140

gactatcttc tcatccacgg aacagcagat gataatgtgc actttcaaaa ctcagcacag 1200

attgctaaag ctctggttaa tgcacaagtg gatttccagg caatgtggta ctctgaccag 1260

aaccacggct tatccggcct gtccacgaac cacttataca cccacatgac ccacttccta 1320

aagcagtgtt tctctttgtc agac 1344

<210> 53
<211> 51
<212> DNA
<213> Artificial sequence

<220>
<223> Polynucleotide

<400> 53
cgcggatccg atgatgatga taagataaat cttgattggg atgtcataag g 51


<210> 54
<211> 57
<212> DNA
<213> Artificial sequence

<220>
<223> Polynucleotide

<400> 54

ctctttaggc agctggatat taccaccacc accaccggga cgattatacg aattatg          57

<210>  55
<211>  57
<212>  DNA
<213>  Artificial sequence

<220>
<223>  Polynucleotide

<400>  55
cataattcgt ataatcgtcc cggtggtggt ggtggtaata tccagctgcc taaagag          57

<210>  56
<211>  31
<212>  DNA
<213>  Artificial sequence

<220>
<223>  Polynucleotide

<400>  56
ccgctcgagc tagtctgaca aagagaaaca c                                      31

<210>  57
<211>  543
<212>  DNA
<213>  Artificial sequence

<220>
<223>  Polynucleotide

<400>  57
ataaatcttg attgggatgt cataagggat aaaactaaga caaagataga gtctttgaaa      60

gagcatggcc ctatcaaaaa taaaatgagc gaaagtccca ataaaacagt atctgaggaa     120

aaagctaaac aatacctaga agaatttcat caaacggcat tagagcatcc tgaattgtca     180

gaacttaaaa ccgttactgg gaccaatcct gtattcgctg gggctaacta tgcggcgtgg     240

gcagtaaacg ttgcgcaagt tatcgattac ggtgacgaac agtacccgcg tgataatttg     300

gaaaagacaa ctgctgctct ttcgatactt cctggtatcg gtagcgtaat gggcattgca     360

gacggtgccg ttcaccacaa tacagaagag atagtggcac aatcaatagc tttatcatct     420

ttaatggttg ctcaagctat tccattggta ggagagctag ttgatattgg tttcgctgca     480

tataattttg tagagagtat tatcaattta tttcaagtag ttcataattc gtataatcgt     540

ccc                                                                   543

<210>  58
<211>  54
<212>  DNA
<213>  Artificial sequence

<220>

<223>  Polynucleotide

<400>  58
cgcggatccc tggaagttct gttccagggg cccataaatc ttgattggga tgtc          54


<210>  59
<211>  33
<212>  DNA
<213>  Artificial sequence

<220>
<223>  Polynucleotide

<400>  59
ccgctcgagc tagggacgat tatacgaatt atg                                 33


<210>  60
<211>  52
<212>  DNA
<213>  Artificial sequence

<220>
<223>  Polynucleotide

<400>  60
cacgcgggta ctgttcgtca ccgtaatcga taacttgcgc aacgtttact gc            52


<210>  61
<211>  51
<212>  DNA
<213>  Artificial sequence

<220>
<223>  Polynucleotide

<400>  61
ttacggtgac gaacagtacc cgcgtgataa tttggaaaag acaactgctg c             51


<210>  62
<211>  279
<212>  PRT
<213>  Artificial sequence

<220>
<223>  Polypeptide

<400>  62

Ile Asn Leu Asp Trp Asp Val Ile Arg Asp Lys Thr Lys Thr Lys Ile
1               5                   10                  15


Glu Ser Leu Lys Glu His Gly Pro Ile Lys Asn Lys Met Ser Glu Ser
            20                  25                  30


Pro Asn Lys Thr Val Ser Glu Glu Lys Ala Lys Gln Tyr Leu Glu Glu
        35                  40                  45

Phe His Gln Thr Ala Leu Glu His Pro Glu Leu Ser Glu Leu Lys Thr
    50                  55                  60

Val Thr Gly Thr Asn Pro Val Phe Ala Gly Ala Asn Tyr Ala Ala Trp
65                  70                  75                  80

Ala Val Asn Val Ala Gln Val Ile Asp Ser Glu Thr Ala Asp Asn Leu
                85                  90                  95

Glu Lys Thr Thr Ala Ala Leu Ser Ile Leu Pro Gly Ile Gly Ser Val
            100                 105                 110

Met Gly Ile Ala Asp Gly Ala Val His His Asn Thr Glu Glu Ile Val
        115                 120                 125

Ala Gln Ser Ile Ala Leu Ser Ser Leu Met Val Ala Gln Ala Ile Pro
    130                 135                 140

Leu Val Gly Glu Leu Val Asp Ile Gly Phe Ala Ala Tyr Asn Phe Val
145                 150                 155                 160

Glu Ser Ile Ile Asn Leu Phe Gln Val Val His Asn Ser Tyr Asn Arg
                165                 170                 175

Pro Gly Gln Asn His His Glu Val Val Lys Phe Met Asp Val Tyr Gln
            180                 185                 190

Arg Ser Tyr Cys His Pro Ile Glu Thr Leu Val Asp Ile Phe Gln Glu
        195                 200                 205

Tyr Pro Asp Glu Ile Glu Tyr Ile Phe Lys Pro Ser Cys Val Pro Leu
    210                 215                 220

Met Arg Cys Gly Gly Cys Cys Asn Asp Glu Gly Leu Glu Cys Val Pro
225                 230                 235                 240

Thr Glu Glu Ser Asn Ile Thr Met Gln Ile Met Arg Ile Lys Pro His
                245                 250                 255

Gln Gly Gln His Ile Gly Glu Met Ser Phe Leu Gln His Asn Lys Cys
            260                 265                 270

Glu Cys Arg Pro Lys Lys Asp
        275

<210>  63
<211>  102

<212>  PRT
<213>  Artificial sequence

<220>
<223>  Polypeptide

<400>  63


<210>  64
<211>  53
<212>  DNA
<213>  Artificial sequence

<220>
<223>  Polynucleotide

<400>  64
caagtagttc ataattcgta taatcgtccc ggtcagaacc accacgaggt tgt              53


<210>  65
<211>  44
<212>  DNA
<213>  Artificial sequence

<220>
<223>  Polynucleotide

<400>  65
ccgctcgagc taatctttct tcggacggca ttcgcacttg ttgt                        44


<210>  66
<211>  837
<212>  DNA

<213>  Artificial sequence

<220>
<223>  Polynucleotide

<400>  66
ataaatcttg attgggatgt cataagggat aaaactaaga caaagataga gtctttgaaa      60

gagcatggcc ctatcaaaaa taaaatgagc gaaagtccca ataaaacagt atctgaggaa     120

aaagctaaac aatacctaga agaatttcat caaacggcat tagagcatcc tgaattgtca     180

gaacttaaaa ccgttactgg gaccaatcct gtattcgctg gggctaacta tgcggcgtgg     240

gcagtaaacg ttgcgcaagt tatcgatagc gaaacagctg ataatttgga aaagacaact     300

gctgctcttt cgatacttcc tggtatcggt agcgtaatgg gcattgcaga cggtgccgtt     360

caccacaata cagaagagat agtggcacaa tcaatagctt tatcatcttt aatggttgct     420

caagctattc cattggtagg agagctagtt gatattggtt tcgctgcata taattttgta     480

gagagtatta tcaatttatt tcaagtagtt cataattcgt ataatcgtcc cggtcagaac     540

caccacgagg ttgtcaaatt catggacgtg taccagcgca gctactgcca cccgatcgaa     600

actctggtcg acatcttcca ggagtacccg gacgagatcg agtacatctt taagccgagc     660

tgcgttcctc tgatgcgttg cggtggttgc tgtaacgatg agggtctgga atgcgtcccg     720

accgaagaat ctaacatcac catgcagatc atgcgtatca aaccgcacca gggtcagcac     780

atcggtgaaa tgtctttcct gcagcacaac aagtgcgaat gccgtccgaa gaaagat        837


<210>  67
<211>  210
<212>  PRT
<213>  Artificial sequence

<220>
<223>  Polypeptide

<400>  67

Phe Thr Ile Thr Ala Pro Lys Asp Leu Tyr Val Val Glu Tyr Gly Ser
1               5                   10                  15


Asn Val Thr Met Glu Cys Arg Phe Pro Val Glu Arg Glu Leu Asp Leu
            20                  25                  30


Leu Ala Leu Val Val Tyr Trp Glu Lys Glu Asp Glu Gln Val Ile Gln
        35                  40                  45


Phe Val Ala Gly Glu Glu Asp Leu Lys Pro Gln His Ser Asn Phe Arg
    50                  55                  60


Gly Arg Ala Ser Leu Pro Lys Asp Gln Leu Leu Lys Gly Asn Ala Ala
65                  70                  75                  80

Leu Gln Ile Thr Asp Val Lys Leu Gln Asp Ala Gly Val Tyr Cys Cys
                85                  90                  95

Ile Ile Ser Tyr Gly Gly Ala Asp Tyr Lys Arg Ile Thr Leu Lys Val
            100                 105                 110

Asn Ala Pro Tyr Arg Lys Ile Asn Gln Arg Ile Ser Val Asp Pro Ala
        115                 120                 125

Thr Ser Glu His Glu Leu Ile Cys Gln Ala Glu Gly Tyr Pro Glu Ala
    130                 135                 140

Glu Val Ile Trp Thr Asn Ser Asp His Gln Pro Val Ser Gly Lys Arg
145                 150                 155                 160

Ser Val Thr Thr Ser Arg Thr Glu Gly Met Leu Leu Asn Val Thr Ser
                165                 170                 175

Ser Leu Arg Val Asn Ala Thr Ala Asn Asp Val Phe Tyr Cys Thr Phe
            180                 185                 190

Trp Arg Ser Gln Pro Gly Gln Asn His Thr Ala Glu Leu Ile Ile Pro
        195                 200                 205

Glu Leu
    210

<210>  68
<211>  98
<212>  PRT
<213>  Artificial sequence

<220>
<223>  Polypeptide

<400>  68

Ala Pro Tyr Asn Lys Ile Asn Gln Arg Ile Leu Val Val Asp Pro Val
1               5                   10                  15

Thr Ser Glu His Glu Leu Thr Cys Gln Ala Glu Gly Tyr Pro Lys Ala
            20                  25                  30

Glu Val Ile Trp Thr Ser Ser Asp His Gln Val Leu Ser Gly Lys Thr
        35                  40                  45

Thr Thr Thr Asn Ser Lys Arg Glu Glu Lys Leu Phe Asn Val Thr Ser
    50                  55                  60

Thr Leu Arg Ile Asn Thr Thr Thr Asn Glu Ile Phe Tyr Cys Thr Phe
65                  70                  75                  80

Arg Arg Leu Asp Pro Glu Glu Asn His Thr Ala Glu Leu Val Ile Pro
                85                  90                  95

Glu Leu

<210>  69
<211>  387
<212>  PRT
<213>  Artificial sequence

<220>
<223>  Polypeptide

<400>  69

Ile Asn Leu Asp Trp Asp Val Ile Arg Asp Lys Thr Lys Thr Lys Ile
1               5                   10                  15

Glu Ser Leu Lys Glu His Gly Pro Ile Lys Asn Lys Met Ser Glu Ser
            20                  25                  30

Pro Asn Lys Thr Val Ser Glu Glu Lys Ala Lys Gln Tyr Leu Glu Glu
        35                  40                  45

Phe His Gln Thr Ala Leu Glu His Pro Glu Leu Ser Glu Leu Lys Thr
    50                  55                  60

Val Thr Gly Thr Asn Pro Val Phe Ala Gly Ala Asn Tyr Ala Ala Trp
65                  70                  75                  80

Ala Val Asn Val Ala Gln Val Ile Asp Ser Glu Thr Ala Asp Asn Leu
                85                  90                  95

Glu Lys Thr Thr Ala Ala Leu Ser Ile Leu Pro Gly Ile Gly Ser Val
            100                 105                 110

Met Gly Ile Ala Asp Gly Ala Val His His Asn Thr Glu Glu Ile Val
        115                 120                 125

Ala Gln Ser Ile Ala Leu Ser Ser Leu Met Val Ala Gln Ala Ile Pro
    130                 135                 140

Leu Val Gly Glu Leu Val Asp Ile Gly Phe Ala Ala Tyr Asn Phe Val
145                 150                 155                 160

Glu Ser Ile Ile Asn Leu Phe Gln Val Val His Asn Ser Tyr Asn Arg
            165                 170                 175

Pro Phe Thr Ile Thr Ala Pro Lys Asp Leu Tyr Val Val Glu Tyr Gly
        180                 185                 190

Ser Asn Val Thr Met Glu Cys Arg Phe Pro Val Glu Arg Glu Leu Asp
    195                 200                 205

Leu Leu Ala Leu Val Val Tyr Trp Glu Lys Glu Asp Glu Gln Val Ile
    210                 215                 220

Gln Phe Val Ala Gly Glu Glu Asp Leu Lys Pro Gln His Ser Asn Phe
225                 230                 235                 240

Arg Gly Arg Ala Ser Leu Pro Lys Asp Gln Leu Leu Lys Gly Asn Ala
            245                 250                 255

Ala Leu Gln Ile Thr Asp Val Lys Leu Gln Asp Ala Gly Val Tyr Cys
        260                 265                 270

Cys Ile Ile Ser Tyr Gly Gly Ala Asp Tyr Lys Arg Ile Thr Leu Lys
    275                 280                 285

Val Asn Ala Pro Tyr Arg Lys Ile Asn Gln Arg Ile Ser Val Asp Pro
    290                 295                 300

Ala Thr Ser Glu His Glu Leu Ile Cys Gln Ala Glu Gly Tyr Pro Glu
305                 310                 315                 320

Ala Glu Val Ile Trp Thr Asn Ser Asp His Gln Pro Val Ser Gly Lys
            325                 330                 335

Arg Ser Val Thr Thr Ser Arg Thr Glu Gly Met Leu Leu Asn Val Thr
        340                 345                 350

Ser Ser Leu Arg Val Asn Ala Thr Ala Asn Asp Val Phe Tyr Cys Thr
    355                 360                 365

Phe Trp Arg Ser Gln Pro Gly Gln Asn His Thr Ala Glu Leu Ile Ile
    370                 375                 380

Pro Glu Leu
385

<210>  70
<211>  275
<212>  PRT

<213>  Artificial sequence

<220>
<223>  Polypeptide

<400>  70

Ile Asn Leu Asp Trp Asp Val Ile Arg Asp Lys Thr Lys Thr Lys Ile
1               5                   10                  15

Glu Ser Leu Lys Glu His Gly Pro Ile Lys Asn Lys Met Ser Glu Ser
            20                  25                  30

Pro Asn Lys Thr Val Ser Glu Glu Lys Ala Lys Gln Tyr Leu Glu Glu
        35                  40                  45

Phe His Gln Thr Ala Leu Glu His Pro Glu Leu Ser Glu Leu Lys Thr
    50                  55                  60

Val Thr Gly Thr Asn Pro Val Phe Ala Gly Ala Asn Tyr Ala Ala Trp
65                  70                  75                  80

Ala Val Asn Val Ala Gln Val Ile Asp Ser Glu Thr Ala Asp Asn Leu
                85                  90                  95

Glu Lys Thr Thr Ala Ala Leu Ser Ile Leu Pro Gly Ile Gly Ser Val
            100                 105                 110

Met Gly Ile Ala Asp Gly Ala Val His His Asn Thr Glu Glu Ile Val
        115                 120                 125

Ala Gln Ser Ile Ala Leu Ser Ser Leu Met Val Ala Gln Ala Ile Pro
    130                 135                 140

Leu Val Gly Glu Leu Val Asp Ile Gly Phe Ala Ala Tyr Asn Phe Val
145                 150                 155                 160

Glu Ser Ile Ile Asn Leu Phe Gln Val Val His Asn Ser Tyr Asn Arg
                165                 170                 175

Pro Ala Pro Tyr Asn Lys Ile Asn Gln Arg Ile Leu Val Val Asp Pro
            180                 185                 190

Val Thr Ser Glu His Glu Leu Thr Cys Gln Ala Glu Gly Tyr Pro Lys
        195                 200                 205

Ala Glu Val Ile Trp Thr Ser Ser Asp His Gln Val Leu Ser Gly Lys
    210                 215                 220

Thr Thr Thr Thr Asn Ser Lys Arg Glu Glu Lys Leu Phe Asn Val Thr
225                 230                 235                 240

Ser Thr Leu Arg Ile Asn Thr Thr Thr Asn Glu Ile Phe Tyr Cys Thr
                245                 250                 255

Phe Arg Arg Leu Asp Pro Glu Glu Asn His Thr Ala Glu Leu Val Ile
            260                 265                 270

Pro Glu Leu
        275

<210>  71
<211>  24
<212>  DNA
<213>  Artificial sequence

<220>
<223>  Polynucleotide

<400>  71
aggatatttg ctggcattat attc                                              24

<210>  72
<211>  21
<212>  DNA
<213>  Artificial sequence

<220>
<223>  Polynucleotide

<400>  72
ttacgtctcc tcgaattgtg t                                                 21

<210>  73
<211>  46
<212>  DNA
<213>  Artificial sequence

<220>
<223>  Polynucleotide

<400>  73
cggaattcga tgatgatgat aagataaatc ttgattggga tgtcat                      46

<210>  74
<211>  56
<212>  DNA
<213>  Artificial sequence

<220>
<223>  Polynucleotide

<400>  74
ctctggttga ttttgcggta tggggcggga cgattatacg aattatgaac tacttg          56

<210>  75
<211>  56
<212>  DNA
<213>  Artificial sequence

<220>
<223>  Polynucleotide

<400>  75
caagtagttc ataattcgta taatcgtccc gccccatacc gcaaaatcaa ccagag          56


<210>  76
<211>  30
<212>  DNA
<213>  Artificial sequence

<220>
<223>  Polynucleotide

<400>  76
cggctcgagc agttctggga tgatcagctc                                      30


<210>  77
<211>  46
<212>  DNA
<213>  Artificial sequence

<220>
<223>  Polynucleotide

<400>  77
cggaattcga tgatgatgat aagataaatc ttgattggga tgtcat                    46


<210>  78
<211>  43
<212>  DNA
<213>  Artificial sequence

<220>
<223>  Polynucleotide

<400>  78
cctttggagc cgtgatagta aagggacgat tatacgaatt atg                       43


<210>  79
<211>  43
<212>  DNA
<213>  Artificial sequence

<220>
<223>  Polynucleotide

<400>  79
cataattcgt ataatcgtcc ctttactatc acggctccaa agg                       43


<210>  80
<211>  28

<212>  DNA
<213>  Artificial sequence

<220>
<223>  Polynucleotide

<400>  80
ctcgagattg actttcagcg tgattcgc                                          28


<210>  81
<211>  28
<212>  DNA
<213>  Artificial sequence

<220>
<223>  Polynucleotide

<400>  81
ggaattcttt actatcacgg ctccaaag                                          28


<210>  82
<211>  26
<212>  DNA
<213>  Artificial sequence

<220>
<223>  Polynucleotide

<400>  82
gctcgagtca cagttctggg atgatc                                            26


<210>  83
<211>  31
<212>  PRT
<213>  Artificial sequence

<220>
<223>  Polypeptide

<400>  83

Asp Thr Cys Pro Pro Leu Met Leu Tyr Asn Pro Thr Thr Tyr Gln Met
1               5                   10                  15


Asp Val Asn Pro Glu Gly Lys Tyr Ser Phe Gly Ala Thr Cys Val
            20                  25                  30


<210>  84
<211>  349
<212>  PRT
<213>  Artificial sequence

<220>
<223>  Polypeptide

<400>  84

Ile Asn Leu Asp Trp Asp Val Ile Arg Asp Lys Thr Lys Thr Lys Ile

1               5                   10                  15

Glu Ser Leu Lys Glu His Gly Pro Ile Lys Asn Lys Met Ser Glu Ser
            20                  25                  30

Pro Asn Lys Thr Val Ser Glu Glu Lys Ala Lys Gln Tyr Leu Glu Glu
        35                  40                  45

Phe His Gln Thr Ala Leu Glu His Pro Glu Leu Ser Glu Leu Lys Thr
    50                  55                  60

Val Thr Gly Thr Asn Pro Val Phe Ala Gly Ala Asn Tyr Ala Ala Trp
65                  70                  75                  80

Ala Val Asn Val Ala Gln Val Ile Asp Ser Glu Thr Ala Asp Asn Leu
                85                  90                  95

Glu Lys Thr Thr Ala Ala Leu Ser Ile Leu Pro Gly Ile Gly Ser Val
            100                 105                 110

Met Gly Ile Ala Asp Gly Ala Val His His Asn Thr Glu Glu Ile Val
        115                 120                 125

Ala Gln Ser Ile Ala Leu Ser Ser Leu Met Val Ala Gln Ala Ile Pro
    130                 135                 140

Leu Val Gly Glu Leu Val Asp Ile Gly Phe Ala Ala Tyr Asn Phe Val
145                 150                 155                 160

Glu Ser Ile Ile Asn Leu Phe Gln Val Val His Asn Ser Tyr Asn Arg
                165                 170                 175

Pro Arg Lys Val Cys Asn Gly Ile Gly Ile Gly Glu Phe Lys Asp Thr
            180                 185                 190

Leu Ser Ile Asn Ala Thr Asn Ile Lys His Phe Lys Tyr Cys Thr Ala
        195                 200                 205

Ile Ser Gly Asp Leu His Ile Leu Pro Val Ala Phe Lys Gly Asp Ser
    210                 215                 220

Phe Thr Arg Thr Pro Pro Leu Asp Pro Arg Glu Leu Glu Ile Leu Lys
225                 230                 235                 240

Thr Val Lys Glu Ile Thr Gly Phe Leu Leu Ile Gln Ala Trp Pro Asp
                245                 250                 255

Asn Trp Thr Asp Leu His Ala Phe Glu Asn Leu Glu Ile Ile Arg Gly
            260                 265                 270

Arg Thr Lys Gln His Gly Gln Phe Ser Leu Ala Val Val Gly Leu Asn
        275                 280                 285

Ile Thr Ser Leu Gly Leu Arg Ser Leu Lys Glu Ile Ser Asp Gly Asp
    290                 295                 300

Val Ile Ile Ser Gly Asn Arg Asn Leu Cys Tyr Ala Asn Thr Ile Asn
305                 310                 315                 320

Trp Lys Lys Leu Phe Gly Thr Pro Asn Gln Lys Thr Lys Ile Met Asn
                325                 330                 335

Asn Arg Ala Glu Lys Asp Cys Lys Ala Val Asn His Val
            340                 345

<210>  85
<211>  349
<212>  PRT
<213>  Artificial sequence

<220>
<223>  Polypeptide

<400>  85

Ile Asn Leu Asp Trp Asp Val Ile Arg Asp Lys Thr Lys Thr Lys Ile
1               5                   10                  15

Glu Ser Leu Lys Glu His Gly Pro Ile Lys Asn Lys Met Ser Glu Ser
            20                  25                  30

Pro Asn Lys Thr Val Ser Glu Glu Lys Ala Lys Gln Tyr Leu Glu Glu
        35                  40                  45

Phe His Gln Thr Ala Leu Glu His Pro Glu Leu Ser Glu Leu Lys Thr
    50                  55                  60

Val Thr Gly Thr Asn Pro Val Phe Ala Gly Ala Asn Tyr Ala Ala Trp
65                  70                  75                  80

Ala Val Asn Val Ala Gln Val Ile Asp Ser Glu Thr Ala Asp Asn Leu
                85                  90                  95

Glu Lys Thr Thr Ala Ala Leu Ser Ile Leu Pro Gly Ile Gly Ser Val
            100                 105                 110

Met Gly Ile Ala Asp Gly Ala Val His His Asn Thr Glu Glu Ile Val

115                 120                 125

Ala Gln Ser Ile Ala Leu Ser Ser Leu Met Val Ala Gln Ala Ile Pro
    130                 135                 140

Leu Val Gly Glu Leu Val Asp Ile Gly Phe Ala Ala Tyr Asn Phe Val
145                 150                 155                 160

Glu Ser Ile Ile Asn Leu Phe Gln Val Val His Asn Ser Tyr Asn Arg
                165                 170                 175

Pro Arg Lys Val Cys Asn Gly Ile Gly Ile Gly Glu Phe Lys Asp Ser
            180                 185                 190

Leu Ser Ile Asn Ala Thr Asn Ile Lys His Phe Lys Asn Cys Thr Ser
        195                 200                 205

Ile Ser Gly Asp Leu His Ile Leu Pro Val Ala Phe Arg Gly Asp Ser
    210                 215                 220

Phe Thr His Thr Pro Pro Leu Asp Pro Gln Glu Leu Asp Ile Leu Lys
225                 230                 235                 240

Thr Val Lys Glu Ile Thr Gly Phe Leu Leu Ile Gln Ala Trp Pro Glu
                245                 250                 255

Asn Arg Thr Asp Leu His Ala Phe Glu Asn Leu Glu Ile Ile Arg Gly
            260                 265                 270

Arg Thr Lys Gln His Gly Gln Phe Ser Leu Ala Val Val Ser Leu Asn
        275                 280                 285

Ile Thr Ser Leu Gly Leu Arg Ser Leu Lys Glu Ile Ser Asp Gly Asp
    290                 295                 300

Val Ile Ile Ser Gly Asn Lys Asn Leu Cys Tyr Ala Asn Thr Ile Asn
305                 310                 315                 320

Trp Lys Lys Leu Phe Gly Thr Ser Gly Gln Lys Thr Lys Ile Ile Ser
                325                 330                 335

Asn Arg Gly Glu Asn Lys Cys Lys Ala Thr Gly Gln Val
            340                 345

<210>  86
<211>  385
<212>  PRT
<213>  Artificial sequence

<220>
<223>  Polypeptide

<400>  86

Ile Asn Leu Asp Trp Asp Val Ile Arg Asp Lys Thr Lys Thr Lys Ile
1               5                   10                  15

Glu Ser Leu Lys Glu His Gly Pro Ile Lys Asn Lys Met Ser Glu Ser
            20                  25                  30

Pro Asn Lys Thr Val Ser Glu Glu Lys Ala Lys Gln Tyr Leu Glu Glu
        35                  40                  45

Phe His Gln Thr Ala Leu Glu His Pro Glu Leu Ser Glu Leu Lys Thr
    50                  55                  60

Val Thr Gly Thr Asn Pro Val Phe Ala Gly Ala Asn Tyr Ala Ala Trp
65                  70                  75                  80

Ala Val Asn Val Ala Gln Val Ile Asp Ser Glu Thr Ala Asp Asn Leu
                85                  90                  95

Glu Lys Thr Thr Ala Ala Leu Ser Ile Leu Pro Gly Ile Gly Ser Val
            100                 105                 110

Met Gly Ile Ala Asp Gly Ala Val His His Asn Thr Glu Glu Ile Val
        115                 120                 125

Ala Gln Ser Ile Ala Leu Ser Ser Leu Met Val Ala Gln Ala Ile Pro
    130                 135                 140

Leu Val Gly Glu Leu Val Asp Ile Gly Phe Ala Ala Tyr Asn Phe Val
145                 150                 155                 160

Glu Ser Ile Ile Asn Leu Phe Gln Val Val His Asn Ser Tyr Asn Arg
                165                 170                 175

Pro Arg Lys Val Cys Asn Gly Ile Gly Ile Gly Glu Phe Lys Asp Thr
            180                 185                 190

Leu Ser Ile Asn Ala Thr Asn Ile Lys His Phe Lys Tyr Cys Thr Ala
        195                 200                 205

Ile Ser Gly Asp Leu His Ile Leu Pro Val Ala Phe Lys Gly Asp Ser
    210                 215                 220

Phe Thr Arg Thr Pro Pro Leu Asp Pro Arg Glu Leu Glu Ile Leu Lys

225                 230                 235                 240

Thr Val Lys Glu Ile Thr Gly Phe Leu Leu Ile Gln Ala Trp Pro Asp
                245                 250                 255

Asn Trp Thr Asp Leu His Ala Phe Glu Asn Leu Glu Ile Ile Arg Gly
            260                 265                 270

Arg Thr Lys Gln His Gly Gln Phe Ser Leu Ala Val Val Gly Leu Asn
        275                 280                 285

Ile Thr Ser Leu Gly Leu Arg Ser Leu Lys Glu Ile Ser Asp Gly Asp
    290                 295                 300

Val Ile Ile Ser Gly Asn Arg Asn Leu Cys Tyr Ala Asn Thr Ile Asn
305                 310                 315                 320

Trp Lys Lys Leu Phe Gly Thr Pro Asn Gln Lys Thr Lys Ile Met Asn
                325                 330                 335

Asn Arg Ala Glu Lys Asp Cys Lys Ala Val Asn His Val Gly Gly Gly
            340                 345                 350

Gly Ser Asp Thr Cys Pro Pro Leu Met Leu Tyr Asn Pro Thr Thr Tyr
        355                 360                 365

Gln Met Asp Val Asn Pro Glu Gly Lys Tyr Ser Phe Gly Ala Thr Cys
    370                 375                 380

Val
385

<210>  87
<211>  360
<212>  PRT
<213>  Artificial sequence

<220>
<223>  Polypeptide

<400>  87

Ile Asn Leu Asp Trp Asp Val Ile Arg Asp Lys Thr Lys Thr Lys Ile
1               5                   10                  15

Glu Ser Leu Lys Glu His Gly Pro Ile Lys Asn Lys Met Ser Glu Ser
            20                  25                  30

Pro Asn Lys Thr Val Ser Glu Glu Lys Ala Lys Gln Tyr Leu Glu Glu
        35                  40                  45

Phe His Gln Thr Ala Leu Glu His Pro Glu Leu Ser Glu Leu Lys Thr
    50                  55                  60

Val Thr Gly Thr Asn Pro Val Phe Ala Gly Ala Asn Tyr Ala Ala Trp
65                  70                  75                  80

Ala Val Asn Val Ala Gln Val Ile Pro Pro Leu Met Leu Tyr Asn Pro
                85                  90                  95

Thr Thr Tyr Gln Met Asp Val Asn Pro Glu Leu Glu Lys Thr Thr Ala
            100                 105                 110

Ala Leu Ser Ile Leu Pro Gly Ile Gly Ser Val Met Gly Ile Ala Asp
        115                 120                 125

Gly Ala Val His His Asn Thr Glu Glu Ile Val Ala Gln Ser Ile Ala
    130                 135                 140

Leu Ser Ser Leu Met Val Ala Gln Ala Ile Pro Leu Val Gly Glu Leu
145                 150                 155                 160

Val Asp Ile Gly Phe Ala Ala Tyr Asn Phe Val Glu Ser Ile Ile Asn
                165                 170                 175

Leu Phe Gln Val Val His Asn Ser Tyr Asn Arg Pro Arg Lys Val Cys
            180                 185                 190

Asn Gly Ile Gly Ile Gly Glu Phe Lys Asp Thr Leu Ser Ile Asn Ala
        195                 200                 205

Thr Asn Ile Lys His Phe Lys Tyr Cys Thr Ala Ile Ser Gly Asp Leu
    210                 215                 220

His Ile Leu Pro Val Ala Phe Lys Gly Asp Ser Phe Thr Arg Thr Pro
225                 230                 235                 240

Pro Leu Asp Pro Arg Glu Leu Glu Ile Leu Lys Thr Val Lys Glu Ile
                245                 250                 255

Thr Gly Phe Leu Leu Ile Gln Ala Trp Pro Asp Asn Trp Thr Asp Leu
            260                 265                 270

His Ala Phe Glu Asn Leu Glu Ile Ile Arg Gly Arg Thr Lys Gln His
        275                 280                 285

Gly Gln Phe Ser Leu Ala Val Val Gly Leu Asn Ile Thr Ser Leu Gly

290                 295                 300

Leu Arg Ser Leu Lys Glu Ile Ser Asp Gly Asp Val Ile Ile Ser Gly
305                 310                 315                 320

Asn Arg Asn Leu Cys Tyr Ala Asn Thr Ile Asn Trp Lys Lys Leu Phe
                325                 330                 335

Gly Thr Pro Asn Gln Lys Thr Lys Ile Met Asn Asn Arg Ala Glu Lys
            340                 345                 350

Asp Cys Lys Ala Val Asn His Val
        355                 360

<210>  88
<211>  57
<212>  DNA
<213>  Artificial sequence

<220>
<223>  Polynucleotide

<400>  88
cgcggatccc tggaagttct gttccagggg ccccgcaaag tttgtaatgg cataggc          57

<210>  89
<211>  51
<212>  DNA
<213>  Artificial sequence

<220>
<223>  Polynucleotide

<400>  89
gtagttcata attcgtataa tcgtccccgc aaagtttgta atggcatagg c                51

<210>  90
<211>  51
<212>  DNA
<213>  Artificial sequence

<220>
<223>  Polynucleotide

<400>  90
gcctatgcca ttacaaactt tgcggggacg attatacgaa ttatgaacta c                51

<210>  91
<211>  40
<212>  DNA
<213>  Artificial sequence

<220>
<223>  Polynucleotide

<400>  91
ccgctcgagc tagacgtggt tcacggcctt gcagtctttc                              40


<210>  92
<211>  57
<212>  DNA
<213>  Artificial sequence

<220>
<223>  Polynucleotide

<400>  92
cgcggatccc tggaagttct gttccagggg ccccgcaaag tttgtaatgg cataggc           57


<210>  93
<211>  56
<212>  DNA
<213>  Artificial sequence

<220>
<223>  Polynucleotide

<400>  93
gtagttcata attcgtataa tcgtccccgc aaagtgtgta acggaatagg tattgg            56


<210>  94
<211>  56
<212>  DNA
<213>  Artificial sequence

<220>
<223>  Polynucleotide

<400>  94
ccaataccta ttccgttaca cactttgcgg ggacgattat acgaattatg aactac            56


<210>  95
<211>  56
<212>  DNA
<213>  Artificial sequence

<220>
<223>  Polynucleotide

<400>  95
ccaataccta ttccgttaca cactttgcgg ggacgattat acgaattatg aactac            56


<210>  96
<211>  57
<212>  DNA
<213>  Artificial sequence

<220>
<223>  Polynucleotide

<400>  96
cgcggatccc tggaagttct gttccagggg ccccgcaaag tttgtaatgg cataggc           57

<210>  97
<211>  59
<212>  DNA
<213>  Artificial sequence

<220>
<223>  Polynucleotide

<400>  97
caaccccacc acctatcaga tggatgtcaa ccctgaattg gaaaagacaa ctgctgctc          59


<210>  98
<211>  71
<212>  DNA
<213>  Artificial sequence

<220>
<223>  Polynucleotide

<400>  98
gttgacatcc atctgatagg tggtggggtt gtacagcatg agtggtggga taacttgcgc          60

aacgtttact g                                                               71


<210>  99
<211>  40
<212>  DNA
<213>  Artificial sequence

<220>
<223>  Polynucleotide

<400>  99
ccgctcgagc tagacgtggt tcacggcctt gcagtctttc                                40


<210>  100
<211>  57
<212>  DNA
<213>  Artificial sequence

<220>
<223>  Polynucleotide

<400>  100
cgcggatccc tggaagttct gttccagggg ccccgcaaag tttgtaatgg cataggc             57


<210>  101
<211>  51
<212>  DNA
<213>  Artificial sequence

<220>
<223>  Polynucleotide

<400>  101
gtagttcata attcgtataa tcgtccccgc aaagtttgta atggcatagg c                   51

<210> 102
<211> 51
<212> DNA
<213> Artificial sequence

<220>
<223> Polynucleotide

<400> 102
gcctatgcca ttacaaactt tgcggggacg attatacgaa ttatgaacta c 51


<210> 103
<211> 40
<212> DNA
<213> Artificial sequence

<220>
<223> Polynucleotide

<400> 103
ccgctcgagc tacacacagg tggcaccaaa gctgtacttc 40


<210> 104
<211> 1047
<212> DNA
<213> Artificial sequence

<220>
<223> Polynucleotide

<400> 104
ataaatcttg attgggatgt cataagggat aaaactaaga caaagataga gtctttgaaa 60

gagcatggcc ctatcaaaaa taaaatgagc gaaagtccca ataaaacagt atctgaggaa 120

aaagctaaac aatacctaga agaatttcat caaacggcat tagagcatcc tgaattgtca 180

gaacttaaaa ccgttactgg gaccaatcct gtattcgctg gggctaacta tgcggcgtgg 240

gcagtaaacg ttgcgcaagt tatcgatagc gaaacagctg ataatttgga aaagacaact 300

gctgctcttt cgatacttcc tggtatcggt agcgtaatgg gcattgcaga cggtgccgtt 360

caccacaata cagaagagat agtggcacaa tcaatagctt tatcatcttt aatggttgct 420

caagctattc cattggtagg agagctagtt gatattggtt tcgctgcata taattttgta 480

gagagtatta tcaatttatt tcaagtagtt cataattcgt ataatcgtcc ccgcaaagtt 540

tgtaatggca taggcattgg tgaatttaaa gacacactct ccataaatgc tacaaacatc 600

aaacacttca aatactgcac tgccatcagc ggggaccttc acatcctgcc agtggccttt 660

aagggggatt ctttcacgcg cactcctcct ctagacccac gagaactaga aattctaaaa 720

accgtaaagg aaataacagg ctttttgctg attcaggctt ggcctgataa ctggactgac 780

ctccatgctt tcgagaacct agaaataata cgtggcagaa caaagcaaca tggtcagttt 840

tctttggcgg tcgttggcct gaacatcaca tcactggggc tgcgttccct caaggagatc 900

agtgatgggg atgtgatcat ttctggaaac cgaaatttgt gctacgcaaa cacaataaac 960

tggaaaaaac tcttcgggac acccaatcag aaaaccaaaa tcatgaacaa cagagctgag 1020

aaagactgca aggccgtgaa ccacgtc 1047

<210> 105
<211> 1047
<212> DNA
<213> Artificial sequence

<220>
<223> Polynucleotide

<400> 105
ataaatcttg attgggatgt cataagggat aaaactaaga caaagataga gtctttgaaa 60

gagcatggcc ctatcaaaaa taaaatgagc gaaagtccca ataaaacagt atctgaggaa 120

aaagctaaac aatacctaga agaatttcat caaacggcat tagagcatcc tgaattgtca 180

gaacttaaaa ccgttactgg gaccaatcct gtattcgctg gggctaacta tgcggcgtgg 240

gcagtaaacg ttgcgcaagt tatcgatagc gaaacagctg ataatttgga aaagacaact 300

gctgctcttt cgatacttcc tggtatcggt agcgtaatgg gcattgcaga cggtgccgtt 360

caccacaata cagaagagat agtggcacaa tcaatagctt tatcatcttt aatggttgct 420

caagctattc cattggtagg agagctagtt gatattggtt tcgctgcata taattttgta 480

gagagtatta tcaatttatt tcaagtagtt cataattcgt ataatcgtcc ccgcaaagtg 540

tgtaacggaa taggtattgg tgaatttaaa gactcactct ccataaatgc tacgaatatt 600

aaacacttca aaaactgcac ctccatcagt ggcgatctcc acatcctgcc ggtggcattt 660

aggggtgact ccttcacaca tactcctcct ctggatccac aggaactgga tattctgaaa 720

accgtaaagg aaatcacagg gtttttgctg attcaggctt ggcctgaaaa caggacggac 780

ctccatgcct ttgagaacct agaaatcata cgcggcagga ccaagcaaca tggtcagttt 840

tctcttgcag tcgtcagcct gaacataaca tccttgggat tacgctccct caaggagata 900

agtgatggag atgtgataat ttcaggaaac aaaaatttgt gctatgcaaa tacaataaac 960

tggaaaaaac tgtttgggac ctccggtcag aaaaccaaaa ttataagcaa cagaggtgaa 1020

aacagctgca aggccacagg ccaggtc 1047

<210> 106
<211> 1080
<212> DNA
<213> Artificial sequence

<220>
<223> Polynucleotide

<400> 106
ataaatcttg attgggatgt cataagggat aaaactaaga caaagataga gtctttgaaa 60

gagcatggcc ctatcaaaaa taaaatgagc gaaagtccca ataaaacagt atctgaggaa 120

aaagctaaac aatacctaga agaatttcat caaacggcat tagagcatcc tgaattgtca 180

gaacttaaaa ccgttactgg gaccaatcct gtattcgctg gggctaacta tgcggcgtgg 240

gcagtaaacg ttgcgcaagt tatcccacca ctcatgctgt acaaccccac cacctatcag 300

atggatgtca accctgaatt ggaaaagaca actgctgctc tttcgatact tcctggtatc 360

ggtagcgtaa tgggcattgc agacggtgcc gttcaccaca atacagaaga gatagtggca 420

caatcaatag ctttatcatc tttaatggtt gctcaagcta ttccattggt aggagagcta 480

gttgatattg gtttcgctgc atataatttt gtagagagta ttatcaattt atttcaagta 540

gttcataatt cgtataatcg tccccgcaaa gtttgtaatg gcataggcat tggtgaattt 600

aaagacacac tctccataaa tgctacaaac atcaaacact tcaaatactg cactgccatc 660

agcggggacc ttcacatcct gccagtggcc tttaaggggg attctttcac gcgcactcct 720

cctctagacc cacgagaact agaaattcta aaaaccgtaa aggaaataac aggctttttg 780

ctgattcagg cttggcctga taactggact gacctccatg ctttcgagaa cctagaaata 840

atacgtggca gaacaaagca acatggtcag ttttctttgg cggtcgttgg cctgaacatc 900

acatcactgg ggctgcgttc cctcaaggag atcagtgatg gggatgtgat catttctgga 960

aaccgaaatt tgtgctacgc aaacacaata aactggaaaa aactcttcgg gacacccaat 1020

cagaaaacca aaatcatgaa caacagagct gagaaagact gcaaggccgt gaaccacgtc 1080

<210> 107
<211> 1155
<212> DNA
<213> Artificial sequence

<220>
<223> Polynucleotide

<400> 107
ataaatcttg attgggatgt cataagggat aaaactaaga caaagataga gtctttgaaa 60

gagcatggcc ctatcaaaaa taaaatgagc gaaagtccca ataaaacagt atctgaggaa 120

aaagctaaac aatacctaga agaatttcat caaacggcat tagagcatcc tgaattgtca 180

gaacttaaaa ccgttactgg gaccaatcct gtattcgctg gggctaacta tgcggcgtgg 240

gcagtaaacg ttgcgcaagt tatcgatagc gaaacagctg ataatttgga aaagacaact 300

gctgctcttt cgatacttcc tggtatcggt agcgtaatgg gcattgcaga cggtgccgtt 360

caccacaata cagaagagat agtggcacaa tcaatagctt tatcatcttt aatggttgct 420

caagctattc cattggtagg agagctagtt gatattggtt tcgctgcata taattttgta 480

gagagtatta tcaatttatt tcaagtagtt cataattcgt ataatcgtcc ccgcaaagtt 540

tgtaatggca taggcattgg tgaatttaaa gacacactct ccataaatgc tacaaacatc 600

aaacacttca aatactgcac tgccatcagc ggggaccttc acatcctgcc agtggccttt 660

aagggggatt ctttcacgcg cactcctcct ctagacccac gagaactaga aattctaaaa 720

accgtaaagg aaataacagg ctttttgctg attcaggctt ggcctgataa ctggactgac 780

ctccatgctt tcgagaacct agaaataata cgtggcagaa caaagcaaca tggtcagttt 840

tctttggcgg tcgttggcct gaacatcaca tcactggggc tgcgttccct caaggagatc 900

agtgatgggg atgtgatcat ttctggaaac cgaaatttgt gctacgcaaa cacaataaac 960

tggaaaaaac tcttcgggac acccaatcag aaaaccaaaa tcatgaacaa cagagctgag 1020

aaagactgca aggccgtgaa ccacgtcggc ggcggcggca gcgacacctg cccaccactc 1080

atgctgtaca accccaccac ctatcagatg gatgtcaacc ctgaagggaa gtacagcttt 1140

ggtgccacct gtgtg 1155

<210> 108
<211> 261
<212> PRT
<213> Homo sapiens

<400> 108

Asn Ile Gln Leu Pro Lys Glu Glu Ile Lys Lys Leu Glu Val Asp Glu
1               5                   10                  15

Ile Thr Leu Trp Tyr Lys Met Ile Leu Pro Pro Gln Phe Asp Arg Ser
            20                  25                  30

Lys Lys Tyr Pro Leu Leu Ile Gln Val Tyr Gly Gly Pro Cys Ser Gln
        35                  40                  45

Ser Val Arg Ser Val Phe Ala Val Asn Trp Ile Ser Tyr Leu Ala Ser
    50                  55                  60

Lys Glu Gly Met Val Ile Ala Leu Val Asp Gly Arg Gly Thr Ala Phe
65                  70                  75                  80

Gln Gly Asp Lys Leu Leu Tyr Ala Val Tyr Arg Lys Leu Gly Val Tyr
                85                  90                  95

Glu Val Glu Asp Gln Ile Thr Ala Val Arg Lys Phe Ile Glu Met Gly
            100                 105                 110

Phe Ile Asp Glu Lys Arg Ile Ala Ile Trp Gly Trp Ser Tyr Gly Gly
        115                 120                 125

Tyr Val Ser Ser Leu Ala Leu Ala Ser Gly Thr Gly Leu Phe Lys Cys

130                 135                 140

Gly Ile Ala Val Ala Pro Val Ser Ser Trp Glu Tyr Tyr Ala Ser Val
145                 150                 155                 160

Tyr Thr Glu Arg Phe Met Gly Leu Pro Thr Lys Asp Asp Asn Leu Glu
                165                 170                 175

His Tyr Lys Asn Ser Thr Val Met Ala Arg Ala Glu Tyr Phe Arg Asn
            180                 185                 190

Val Asp Tyr Leu Leu Ile His Gly Thr Ala Asp Asp Asn Val His Phe
        195                 200                 205

Gln Asn Ser Ala Gln Ile Ala Lys Ala Leu Val Asn Ala Gln Val Asp
    210                 215                 220

Phe Gln Ala Met Trp Tyr Ser Asp Gln Asn His Gly Leu Ser Gly Leu
225                 230                 235                 240

Ser Thr Asn His Leu Tyr Thr His Met Thr His Phe Leu Lys Gln Cys
                245                 250                 255

Phe Ser Leu Ser Asp
            260

<210>  109
<211>  18
<212>  PRT
<213>  Homo sapiens

<400>  109

Pro Pro Leu Met Leu Tyr Asn Pro Thr Thr Tyr Gln Met Asp Val Asn
1               5                   10                  15

Pro Glu

<210>  110
<211>  172
<212>  PRT
<213>  Mus musculus

<400>  110

Arg Lys Val Cys Asn Gly Ile Gly Ile Gly Glu Phe Lys Asp Thr Leu
1               5                   10                  15

Ser Ile Asn Ala Thr Asn Ile Lys His Phe Lys Tyr Cys Thr Ala Ile
            20                  25                  30

Ser Gly Asp Leu His Ile Leu Pro Val Ala Phe Lys Gly Asp Ser Phe
        35                  40                  45

Thr Arg Thr Pro Pro Leu Asp Pro Arg Glu Leu Glu Ile Leu Lys Thr
    50                  55                  60

Val Lys Glu Ile Thr Gly Phe Leu Leu Ile Gln Ala Trp Pro Asp Asn
65                  70                  75                  80

Trp Thr Asp Leu His Ala Phe Glu Asn Leu Glu Ile Ile Arg Gly Arg
                85                  90                  95

Thr Lys Gln His Gly Gln Phe Ser Leu Ala Val Val Gly Leu Asn Ile
            100                 105                 110

Thr Ser Leu Gly Leu Arg Ser Leu Lys Glu Ile Ser Asp Gly Asp Val
        115                 120                 125

Ile Ile Ser Gly Asn Arg Asn Leu Cys Tyr Ala Asn Thr Ile Asn Trp
    130                 135                 140

Lys Lys Leu Phe Gly Thr Pro Asn Gln Lys Thr Lys Ile Met Asn Asn
145                 150                 155                 160

Arg Ala Glu Lys Asp Cys Lys Ala Val Asn His Val
                165                 170

<210>  111
<211>  172
<212>  PRT
<213>  Homo sapiens

<400>  111

Arg Lys Val Cys Asn Gly Ile Gly Ile Gly Glu Phe Lys Asp Ser Leu
1               5                   10                  15

Ser Ile Asn Ala Thr Asn Ile Lys His Phe Lys Asn Cys Thr Ser Ile
            20                  25                  30

Ser Gly Asp Leu His Ile Leu Pro Val Ala Phe Arg Gly Asp Ser Phe
        35                  40                  45

Thr His Thr Pro Pro Leu Asp Pro Gln Glu Leu Asp Ile Leu Lys Thr
    50                  55                  60

Val Lys Glu Ile Thr Gly Phe Leu Leu Ile Gln Ala Trp Pro Glu Asn
65                  70                  75                  80

Arg Thr Asp Leu His Ala Phe Glu Asn Leu Glu Ile Ile Arg Gly Arg
                85                  90                  95

Thr Lys Gln His Gly Gln Phe Ser Leu Ala Val Val Ser Leu Asn Ile
            100                 105                 110

Thr Ser Leu Gly Leu Arg Ser Leu Lys Glu Ile Ser Asp Gly Asp Val
        115                 120                 125

Ile Ile Ser Gly Asn Lys Asn Leu Cys Tyr Ala Asn Thr Ile Asn Trp
    130                 135                 140

Lys Lys Leu Phe Gly Thr Ser Gly Gln Lys Thr Lys Ile Ile Ser Asn
145                 150                 155                 160

Arg Gly Glu Asn Lys Cys Lys Ala Thr Gly Gln Val
                165                 170

<210>  112
<211>  191
<212>  PRT
<213>  Artificial sequence

<220>
<223>  Polypeptide

<400>  112

Ile Asn Leu Asp Trp Asp Val Ile Arg Asp Lys Thr Lys Thr Lys Ile
1               5                   10                  15

Glu Ser Leu Lys Glu His Gly Pro Ile Lys Asn Lys Met Ser Glu Ser
            20                  25                  30

Pro Asn Lys Thr Val Ser Glu Glu Lys Ala Lys Gln Tyr Leu Glu Glu
        35                  40                  45

Phe His Gln Thr Ala Leu Glu His Pro Glu Leu Ser Glu Leu Lys Thr
    50                  55                  60

Val Thr Gly Thr Asn Pro Val Phe Ala Gly Ala Asn Tyr Ala Ala Trp
65                  70                  75                  80

Ala Val Asn Val Ala Gln Val Ile Asp Ser Glu Thr Ala Val Ser Arg
                85                  90                  95

Phe Ala Ile Ser Tyr Gln Glu Lys Val Asn Leu Leu Ser Ala Glu Lys
            100                 105                 110

Thr Thr Ala Ala Leu Ser Ile Leu Pro Gly Ile Gly Ser Val Met Gly
        115                 120                 125

Ile Ala Asp Gly Ala Val His His Asn Thr Glu Glu Ile Val Ala Gln
    130                 135                 140

Ser Ile Ala Leu Ser Ser Leu Met Val Ala Gln Ala Ile Pro Leu Val
145                 150                 155                 160

Gly Glu Leu Val Asp Ile Gly Phe Ala Ala Tyr Asn Phe Val Glu Ser
                165                 170                 175

Ile Ile Asn Leu Phe Gln Val Val His Asn Ser Tyr Asn Arg Pro
            180                 185                 190

<210>  113
<211>  187
<212>  PRT
<213>  Artificial sequence

<220>
<223>  Polypeptide

<400>  113

Ile Asn Leu Asp Trp Asp Val Ile Arg Asp Lys Thr Lys Thr Lys Ile
1               5                   10                  15

Glu Ser Leu Lys Glu His Gly Pro Ile Lys Asn Lys Met Ser Glu Ser
            20                  25                  30

Pro Asn Lys Thr Val Ser Glu Glu Lys Ala Lys Gln Tyr Leu Glu Glu
        35                  40                  45

Phe His Gln Thr Ala Leu Glu His Pro Glu Leu Ser Glu Leu Lys Thr
    50                  55                  60

Val Thr Gly Thr Asn Pro Val Phe Ala Gly Ala Asn Tyr Ala Ala Trp
65                  70                  75                  80

Ala Val Asn Val Ala Gln Val Ile Asp Ser Glu Thr Ala Glu Ala Phe
                85                  90                  95

Ser Pro Val Ser Tyr Gln His Asp Leu Ala Glu Lys Thr Thr Ala Ala
            100                 105                 110

Leu Ser Ile Leu Pro Gly Ile Gly Ser Val Met Gly Ile Ala Asp Gly
        115                 120                 125

Ala Val His His Asn Thr Glu Glu Ile Val Ala Gln Ser Ile Ala Leu
    130                 135                 140

Ser Ser Leu Met Val Ala Gln Ala Ile Pro Leu Val Gly Glu Leu Val
145                 150                 155                 160

Asp Ile Gly Phe Ala Ala Tyr Asn Phe Val Glu Ser Ile Ile Asn Leu
                165                 170                 175

Phe Gln Val Val His Asn Ser Tyr Asn Arg Pro
            180                 185

<210>  114
<211>  429
<212>  PRT
<213>  Artificial sequence

<220>
<223>  Polypeptide

<400>  114

Ile Asn Leu Asp Trp Asp Val Ile Arg Asp Lys Thr Lys Thr Lys Ile
1               5                   10                  15

Glu Ser Leu Lys Glu His Gly Pro Ile Lys Asn Lys Met Ser Glu Ser
            20                  25                  30

Pro Asn Lys Thr Val Ser Glu Glu Lys Ala Lys Gln Tyr Leu Glu Glu
        35                  40                  45

Phe His Gln Thr Ala Leu Glu His Pro Glu Leu Ser Glu Leu Lys Thr
    50                  55                  60

Val Thr Gly Thr Asn Pro Val Phe Ala Gly Ala Asn Tyr Ala Ala Trp
65                  70                  75                  80

Ala Val Asn Val Ala Gln Val Ile Asp Ser Glu Thr Ala Asp Asn Leu
                85                  90                  95

Glu Lys Thr Thr Ala Ala Leu Ser Ile Leu Pro Gly Ile Gly Ser Val
            100                 105                 110

Met Gly Ile Ala Asp Gly Ala Val His His Asn Thr Glu Glu Ile Val
        115                 120                 125

Ala Gln Ser Ile Ala Leu Ser Ser Leu Met Val Ala Gln Ala Ile Pro
    130                 135                 140

Leu Val Gly Glu Leu Val Asp Ile Gly Phe Ala Ala Tyr Asn Phe Val

145                 150                 155                 160

Glu Ser Ile Ile Asn Leu Phe Gln Val Val His Asn Ser Tyr Asn Arg
                165                 170                 175

Pro Arg Lys Ser Leu Ser Ser Met Thr Arg Val Val Gly Gly Leu Val
            180                 185                 190

Ala Leu Arg Gly Ala His Pro Tyr Ile Ala Ala Leu Tyr Trp Gly His
        195                 200                 205

Ser Phe Cys Ala Gly Ser Leu Ile Ala Pro Cys Trp Val Leu Thr Ala
    210                 215                 220

Ala His Cys Leu Gln Asp Arg Pro Ala Pro Glu Asp Leu Thr Val Val
225                 230                 235                 240

Leu Gly Gln Glu Arg Arg Asn His Ser Cys Glu Pro Cys Gln Thr Leu
                245                 250                 255

Ala Val Arg Ser Tyr Arg Leu His Glu Ala Phe Ser Pro Val Ser Tyr
            260                 265                 270

Gln His Asp Leu Ala Leu Leu Arg Leu Gln Glu Asp Ala Asp Gly Ser
        275                 280                 285

Cys Ala Leu Leu Ser Pro Tyr Val Gln Pro Val Cys Leu Pro Ser Gly
    290                 295                 300

Ala Ala Arg Pro Ser Glu Thr Thr Leu Cys Gln Val Ala Gly Trp Gly
305                 310                 315                 320

His Gln Phe Glu Gly Ala Glu Glu Tyr Ala Ser Phe Leu Gln Glu Ala
                325                 330                 335

Gln Val Pro Phe Leu Ser Leu Glu Arg Cys Ser Ala Pro Asp Val His
            340                 345                 350

Gly Ser Ser Ile Leu Pro Gly Met Leu Cys Ala Gly Phe Leu Glu Gly
        355                 360                 365

Gly Thr Asp Ala Cys Gln Gly Asp Ser Gly Gly Pro Leu Val Cys Glu
    370                 375                 380

Asp Gln Ala Ala Glu Arg Arg Leu Thr Leu Gln Gly Ile Ile Ser Trp
385                 390                 395                 400

Gly Ser Gly Cys Gly Asp Arg Asn Lys Pro Gly Val Tyr Thr Asp Val
                405                 410                 415

Ala Tyr Tyr Leu Ala Trp Ile Arg Glu His Thr Val Ser
            420                 425

<210>  115
<211>  561
<212>  DNA
<213>  Artificial sequence

<220>
<223>  Polynucleotide

<400>  115
ataaatcttg attgggatgt cataagggat aaaactaaga caaagataga gtctttgaaa      60

gagcatggcc ctatcaaaaa taaaatgagc gaaagtccca ataaaacagt atctgaggaa     120

aaagctaaac aatacctaga agaatttcat caaacggcat tagagcatcc tgaattgtca     180

gaacttaaaa ccgttactgg gaccaatcct gtattcgctg gggctaacta tgcggcgtgg     240

gcagtaaacg ttgcgcaagt tatcgatagc gaaacagctg aggccttctc gcccgtcagc     300

taccagcacg acctggctga aaagacaact gctgctcttt cgatacttcc tggtatcggt     360

agcgtaatgg gcattgcaga cggtgccgtt caccacaata cagaagagat agtggcacaa     420

tcaatagctt tatcatcttt aatggttgct caagctattc cattggtagg agagctagtt     480

gatattggtt tcgctgcata taattttgta gagagtatta tcaatttatt tcaagtagtt     540

cataattcgt ataatcgtcc c                                               561

<210>  116
<211>  1287
<212>  DNA
<213>  Artificial sequence

<220>
<223>  Polynucleotide

<400>  116
ataaatcttg attgggatgt cataagggat aaaactaaga caaagataga gtctttgaaa      60

gagcatggcc ctatcaaaaa taaaatgagc gaaagtccca ataaaacagt atctgaggaa     120

aaagctaaac aatacctaga agaatttcat caaacggcat tagagcatcc tgaattgtca     180

gaacttaaaa ccgttactgg gaccaatcct gtattcgctg gggctaacta tgcggcgtgg     240

gcagtaaacg ttgcgcaagt tatcgatagc gaaacagctg ataatttgga aaagacaact     300

gctgctcttt cgatacttcc tggtatcggt agcgtaatgg gcattgcaga cggtgccgtt     360

caccacaata cagaagagat agtggcacaa tcaatagctt tatcatcttt aatggttgct     420

caagctattc cattggtagg agagctagtt gatattggtt tcgctgcata taattttgta     480

gagagtatta tcaatttatt tcaagtagtt cataattcgt ataatcgtcc ccgcaagagt 540

ctgtcttcga tgacccgcgt cgttggcggg ctggtggcgc tacgcggggc gcacccctac 600

atcgccgcgc tgtactgggg ccacagtttc tgcgccggca gcctcatcgc cccctgctgg 660

gtgctgacgg ccgctcactg cctgcaggac cggcccgcac ccgaggatct gacggtggtg 720

ctcggccagg aacgccgtaa ccacagctgt gagccgtgcc agacgttggc cgtgcgctcc 780

taccgcttgc acgaggcctt ctcgcccgtc agctaccagc acgacctggc tctgttgcgc 840

cttcaggagg atgcggacgg cagctgcgcg ctcctgtcgc cttacgttca gccggtgtgc 900

ctgccaagcg gcgccgcgcg accctccgag accacgctct gccaggtggc cggctggggc 960

caccagttcg agggggcgga ggaatatgcc agcttcctgc aggaggcgca ggtaccgttc 1020

ctctccctgg agcgctgctc agccccggac gtgcacggat cctccatcct ccccggcatg 1080

ctctgcgcag ggttcctcga gggcggcacc gatgcgtgcc agggtgattc cggaggcccg 1140

ctggtgtgtg aggaccaagc tgcagagcgc cggctcaccc tgcaaggcat catcagctgg 1200

ggatcgggct gtggtgaccg caacaagcca ggcgtctaca ccgatgtggc ctactacctg 1260

gcctggatcc gggagcacac cgtttcc 1287

<210> 117
<211> 17
<212> PRT
<213> Homo sapiens

<400> 117

Ile Ser Arg Ile Ala Val Ser Tyr Gln Thr Lys Val Asn Leu Leu Ser
1               5                   10                  15

Ala

<210> 118
<211> 18
<212> PRT
<213> Homo sapiens

<400> 118

Ile Ser Arg Ile Ala Val Ser Tyr Gln Thr Lys Val Asn Leu Leu Ser
1               5                   10                  15

Ala Ile

<210> 119
<211> 385
<212> PRT
<213> Artificial sequence

<220>
<223>  Polypeptide

<400>  119

Ile Asn Leu Asp Trp Asp Val Ile Arg Asp Lys Thr Lys Thr Lys Ile
1               5                   10                  15

Glu Ser Leu Lys Glu His Gly Pro Ile Lys Asn Lys Met Ser Glu Ser
            20                  25                  30

Pro Asn Lys Thr Val Ser Glu Glu Lys Ala Lys Gln Tyr Leu Glu Glu
        35                  40                  45

Phe His Gln Thr Ala Leu Glu His Pro Glu Leu Ser Glu Leu Lys Thr
    50                  55                  60

Val Thr Gly Thr Asn Pro Val Phe Ala Gly Ala Asn Tyr Ala Ala Trp
65                  70                  75                  80

Ala Val Asn Val Ala Gln Val Ile Asp Ser Glu Thr Ala Asp Asn Leu
                85                  90                  95

Glu Lys Thr Thr Ala Ala Leu Ser Ile Leu Pro Gly Ile Gly Ser Val
            100                 105                 110

Met Gly Ile Ala Asp Gly Ala Val His His Asn Thr Glu Glu Ile Val
        115                 120                 125

Ala Gln Ser Ile Ala Leu Ser Ser Leu Met Val Ala Gln Ala Ile Pro
    130                 135                 140

Leu Val Gly Glu Leu Val Asp Ile Gly Phe Ala Ala Tyr Asn Phe Val
145                 150                 155                 160

Glu Ser Ile Ile Asn Leu Phe Gln Val Val His Asn Ser Tyr Asn Arg
                165                 170                 175

Pro Arg Lys Val Cys Asn Gly Ile Gly Ile Gly Glu Phe Lys Asp Ser
            180                 185                 190

Leu Ser Ile Asn Ala Thr Asn Ile Lys His Phe Lys Asn Cys Thr Ser
        195                 200                 205

Ile Ser Gly Asp Leu His Ile Leu Pro Val Ala Phe Arg Gly Asp Ser
    210                 215                 220

Phe Thr His Thr Pro Pro Leu Asp Pro Gln Glu Leu Asp Ile Leu Lys

225                 230                 235                 240

Thr Val Lys Glu Ile Thr Gly Phe Leu Leu Ile Gln Ala Trp Pro Glu
                245                 250                 255

Asn Arg Thr Asp Leu His Ala Phe Glu Asn Leu Glu Ile Ile Arg Gly
            260                 265                 270

Arg Thr Lys Gln His Gly Gln Phe Ser Leu Ala Val Val Ser Leu Asn
        275                 280                 285

Ile Thr Ser Leu Gly Leu Arg Ser Leu Lys Glu Ile Ser Asp Gly Asp
    290                 295                 300

Val Ile Ile Ser Gly Asn Lys Asn Leu Cys Tyr Ala Asn Thr Ile Asn
305                 310                 315                 320

Trp Lys Lys Leu Phe Gly Thr Ser Gly Gln Lys Thr Lys Ile Ile Ser
                325                 330                 335

Asn Arg Gly Glu Asn Lys Cys Lys Ala Thr Gly Gln Val Gly Gly Gly
            340                 345                 350

Gly Ser Asp Thr Cys Pro Pro Leu Met Leu Tyr Asn Pro Thr Thr Tyr
        355                 360                 365

Gln Met Asp Val Asn Pro Glu Gly Lys Tyr Ser Phe Gly Ala Thr Cys
    370                 375                 380

Val
385

<210>  120
<211>  360
<212>  PRT
<213>  Artificial sequence

<220>
<223>  Polypeptide

<400>  120

Ile Asn Leu Asp Trp Asp Val Ile Arg Asp Lys Thr Lys Thr Lys Ile
1               5                   10                  15

Glu Ser Leu Lys Glu His Gly Pro Ile Lys Asn Lys Met Ser Glu Ser
            20                  25                  30

Pro Asn Lys Thr Val Ser Glu Glu Lys Ala Lys Gln Tyr Leu Glu Glu
        35                  40                  45

Phe His Gln Thr Ala Leu Glu His Pro Glu Leu Ser Glu Leu Lys Thr
    50                  55                  60

Val Thr Gly Thr Asn Pro Val Phe Ala Gly Ala Asn Tyr Ala Ala Trp
65                  70                  75                  80

Ala Val Asn Val Ala Gln Val Ile Pro Pro Leu Met Leu Tyr Asn Pro
                85                  90                  95

Thr Thr Tyr Gln Met Asp Val Asn Pro Glu Leu Glu Lys Thr Thr Ala
            100                 105                 110

Ala Leu Ser Ile Leu Pro Gly Ile Gly Ser Val Met Gly Ile Ala Asp
        115                 120                 125

Gly Ala Val His His Asn Thr Glu Glu Ile Val Ala Gln Ser Ile Ala
    130                 135                 140

Leu Ser Ser Leu Met Val Ala Gln Ala Ile Pro Leu Val Gly Glu Leu
145                 150                 155                 160

Val Asp Ile Gly Phe Ala Ala Tyr Asn Phe Val Glu Ser Ile Ile Asn
                165                 170                 175

Leu Phe Gln Val Val His Asn Ser Tyr Asn Arg Pro Arg Lys Val Cys
            180                 185                 190

Asn Gly Ile Gly Ile Gly Glu Phe Lys Asp Ser Leu Ser Ile Asn Ala
        195                 200                 205

Thr Asn Ile Lys His Phe Lys Asn Cys Thr Ser Ile Ser Gly Asp Leu
    210                 215                 220

His Ile Leu Pro Val Ala Phe Arg Gly Asp Ser Phe Thr His Thr Pro
225                 230                 235                 240

Pro Leu Asp Pro Gln Glu Leu Asp Ile Leu Lys Thr Val Lys Glu Ile
                245                 250                 255

Thr Gly Phe Leu Leu Ile Gln Ala Trp Pro Glu Asn Arg Thr Asp Leu
            260                 265                 270

His Ala Phe Glu Asn Leu Glu Ile Ile Arg Gly Arg Thr Lys Gln His
        275                 280                 285

Gly Gln Phe Ser Leu Ala Val Val Ser Leu Asn Ile Thr Ser Leu Gly

290                 295                 300

Leu Arg Ser Leu Lys Glu Ile Ser Asp Gly Asp Val Ile Ile Ser Gly
305                 310                 315                 320

Asn Lys Asn Leu Cys Tyr Ala Asn Thr Ile Asn Trp Lys Lys Leu Phe
                325                 330                 335

Gly Thr Ser Gly Gln Lys Thr Lys Ile Ile Ser Asn Arg Gly Glu Asn
            340                 345                 350

Lys Cys Lys Ala Thr Gly Gln Val
        355                 360

<210>  121
<211>  573
<212>  DNA
<213>  Artificial sequence

<220>
<223>  Polynucleotide

<400>  121
ataaatcttg attgggatgt cataagggat aaaactaaga caaagataga gtctttgaaa      60

gagcatggcc ctatcaaaaa taaaatgagc gaaagtccca ataaaacagt atctgaggaa     120

aaagctaaac aatacctaga agaatttcat caaacggcat tagagcatcc tgaattgtca     180

gaacttaaaa ccgttactgg gaccaatcct gtattcgctg gggctaacta tgcggcgtgg     240

gcagtaaacg ttgcgcaagt tatcgatagc gaaacagctg tcagccgatt tgctatctca     300

taccaggaga aagtcaacct cctctctgcc gaaaagacaa ctgctgctct ttcgatactt     360

cctggtatcg gtagcgtaat gggcattgca gacggtgccg ttcaccacaa tacagaagag     420

atagtggcac aatcaatagc tttatcatct ttaatggttg ctcaagctat tccattggta     480

ggagagctag ttgatattgg tttcgctgca tataattttg tagagagtat tatcaattta     540

tttcaagtag ttcataattc gtataatcgt ccc                                  573

<210>  122
<211>  290
<212>  PRT
<213>  Mus musculus

<400>  122

Met Arg Ile Phe Ala Gly Ile Ile Phe Thr Ala Cys Cys His Leu Leu
1               5                   10                  15

Arg Ala Phe Thr Ile Thr Ala Pro Lys Asp Leu Tyr Val Val Glu Tyr
            20                  25                  30

Gly Ser Asn Val Thr Met Glu Cys Arg Phe Pro Val Glu Arg Glu Leu
        35                  40                  45

Asp Leu Leu Ala Leu Val Val Tyr Trp Glu Lys Glu Asp Glu Gln Val
    50                  55                  60

Ile Gln Phe Val Ala Gly Glu Glu Asp Leu Lys Pro Gln His Ser Asn
65                  70                  75                  80

Phe Arg Gly Arg Ala Ser Leu Pro Lys Asp Gln Leu Leu Lys Gly Asn
                85                  90                  95

Ala Ala Leu Gln Ile Thr Asp Val Lys Leu Gln Asp Ala Gly Val Tyr
            100                 105                 110

Cys Cys Ile Ile Ser Tyr Gly Gly Ala Asp Tyr Lys Arg Ile Thr Leu
        115                 120                 125

Lys Val Asn Ala Pro Tyr Arg Lys Ile Asn Gln Arg Ile Ser Val Asp
    130                 135                 140

Pro Ala Thr Ser Glu His Glu Leu Ile Cys Gln Ala Glu Gly Tyr Pro
145                 150                 155                 160

Glu Ala Glu Val Ile Trp Thr Asn Ser Asp His Gln Pro Val Ser Gly
                165                 170                 175

Lys Arg Ser Val Thr Thr Ser Arg Thr Glu Gly Met Leu Leu Asn Val
            180                 185                 190

Thr Ser Ser Leu Arg Val Asn Ala Thr Ala Asn Asp Val Phe Tyr Cys
        195                 200                 205

Thr Phe Trp Arg Ser Gln Pro Gly Gln Asn His Thr Ala Glu Leu Ile
    210                 215                 220

Ile Pro Glu Leu Pro Ala Thr His Pro Pro Gln Asn Arg Thr His Trp
225                 230                 235                 240

Val Leu Leu Gly Ser Ile Leu Leu Phe Leu Ile Val Val Ser Thr Val
                245                 250                 255

Leu Leu Phe Leu Arg Lys Gln Val Arg Met Leu Asp Val Glu Lys Cys
            260                 265                 270

Gly Val Glu Asp Thr Ser Ser Lys Asn Arg Asn Asp Thr Gln Phe Glu
        275                 280                 285

Glu Thr
    290

<210>  123
<211>  290
<212>  PRT
<213>  Homo sapiens

<400>  123

Met Arg Ile Phe Ala Val Phe Ile Phe Met Thr Tyr Trp His Leu Leu
1               5                   10                  15

Asn Ala Phe Thr Val Thr Val Pro Lys Asp Leu Tyr Val Val Glu Tyr
            20                  25                  30

Gly Ser Asn Met Thr Ile Glu Cys Lys Phe Pro Val Glu Lys Gln Leu
        35                  40                  45

Asp Leu Ala Ala Leu Ile Val Tyr Trp Glu Met Glu Asp Lys Asn Ile
    50                  55                  60

Ile Gln Phe Val His Gly Glu Glu Asp Leu Lys Val Gln His Ser Ser
65                  70                  75                  80

Tyr Arg Gln Arg Ala Arg Leu Leu Lys Asp Gln Leu Ser Leu Gly Asn
                85                  90                  95

Ala Ala Leu Gln Ile Thr Asp Val Lys Leu Gln Asp Ala Gly Val Tyr
            100                 105                 110

Arg Cys Met Ile Ser Tyr Gly Gly Ala Asp Tyr Lys Arg Ile Thr Val
        115                 120                 125

Lys Val Asn Ala Pro Tyr Asn Lys Ile Asn Gln Arg Ile Leu Val Val
    130                 135                 140

Asp Pro Val Thr Ser Glu His Glu Leu Thr Cys Gln Ala Glu Gly Tyr
145                 150                 155                 160

Pro Lys Ala Glu Val Ile Trp Thr Ser Ser Asp His Gln Val Leu Ser
                165                 170                 175

Gly Lys Thr Thr Thr Thr Asn Ser Lys Arg Glu Glu Lys Leu Phe Asn
            180                 185                 190

Val Thr Ser Thr Leu Arg Ile Asn Thr Thr Thr Asn Glu Ile Phe Tyr
        195                 200                 205

Cys Thr Phe Arg Arg Leu Asp Pro Glu Glu Asn His Thr Ala Glu Leu
    210                 215                 220

Val Ile Pro Glu Leu Pro Leu Ala His Pro Pro Asn Glu Arg Thr His
225                 230                 235                 240

Leu Val Ile Leu Gly Ala Ile Leu Leu Cys Leu Gly Val Ala Leu Thr
                245                 250                 255

Phe Ile Phe Arg Leu Arg Lys Gly Arg Met Met Asp Val Lys Lys Cys
            260                 265                 270

Gly Ile Gln Asp Thr Asn Ser Lys Lys Gln Ser Asp Thr His Leu Glu
        275                 280                 285

Glu Thr
    290

<210>  124
<211>  211
<212>  PRT
<213>  Homo sapiens

<400>  124

Phe Thr Val Thr Val Pro Lys Asp Leu Tyr Val Val Glu Tyr Gly Ser
1               5                   10                  15

Asn Met Thr Ile Glu Cys Lys Phe Pro Val Glu Lys Gln Leu Asp Leu
            20                  25                  30

Ala Ala Leu Ile Val Tyr Trp Glu Met Glu Asp Lys Asn Ile Ile Gln
        35                  40                  45

Phe Val His Gly Glu Glu Asp Leu Lys Val Gln His Ser Ser Tyr Arg
    50                  55                  60

Gln Arg Ala Arg Leu Leu Lys Asp Gln Leu Ser Leu Gly Asn Ala Ala
65                  70                  75                  80

Leu Gln Ile Thr Asp Val Lys Leu Gln Asp Ala Gly Val Tyr Arg Cys
                85                  90                  95

Met Ile Ser Tyr Gly Gly Ala Asp Tyr Lys Arg Ile Thr Val Lys Val
            100                 105                 110

Asn Ala Pro Tyr Asn Lys Ile Asn Gln Arg Ile Leu Val Val Asp Pro
        115                 120                 125

Val Thr Ser Glu His Glu Leu Thr Cys Gln Ala Glu Gly Tyr Pro Lys
    130                 135                 140

Ala Glu Val Ile Trp Thr Ser Ser Asp His Gln Val Leu Ser Gly Lys
145                 150                 155                 160

Thr Thr Thr Thr Asn Ser Lys Arg Glu Glu Lys Leu Phe Asn Val Thr
                165                 170                 175

Ser Thr Leu Arg Ile Asn Thr Thr Thr Asn Glu Ile Phe Tyr Cys Thr
            180                 185                 190

Phe Arg Arg Leu Asp Pro Glu Glu Asn His Thr Ala Glu Leu Val Ile
        195                 200                 205

Pro Glu Leu
    210

<210>  125
<211>  98
<212>  PRT
<213>  Homo sapiens

<400>  125

Ala Pro Tyr Asn Lys Ile Asn Gln Arg Ile Leu Val Val Asp Pro Val
1               5                   10                  15

Thr Ser Glu His Glu Leu Thr Cys Gln Ala Glu Gly Tyr Pro Lys Ala
            20                  25                  30

Glu Val Ile Trp Thr Ser Ser Asp His Gln Val Leu Ser Gly Lys Thr
        35                  40                  45

Thr Thr Thr Asn Ser Lys Arg Glu Glu Lys Leu Phe Asn Val Thr Ser
    50                  55                  60

Thr Leu Arg Ile Asn Thr Thr Thr Asn Glu Ile Phe Tyr Cys Thr Phe
65                  70                  75                  80

Arg Arg Leu Asp Pro Glu Glu Asn His Thr Ala Glu Leu Val Ile Pro
                85                  90                  95

Glu Leu

<210>  126
<211>  388

<212>  PRT
<213>  Artificial sequence

<220>
<223>  Polypeptide

<400>  126

Ile Asn Leu Asp Trp Asp Val Ile Arg Asp Lys Thr Lys Thr Lys Ile
1               5                   10                  15

Glu Ser Leu Lys Glu His Gly Pro Ile Lys Asn Lys Met Ser Glu Ser
            20                  25                  30

Pro Asn Lys Thr Val Ser Glu Glu Lys Ala Lys Gln Tyr Leu Glu Glu
        35                  40                  45

Phe His Gln Thr Ala Leu Glu His Pro Glu Leu Ser Glu Leu Lys Thr
    50                  55                  60

Val Thr Gly Thr Asn Pro Val Phe Ala Gly Ala Asn Tyr Ala Ala Trp
65                  70                  75                  80

Ala Val Asn Val Ala Gln Val Ile Asp Ser Glu Thr Ala Asp Asn Leu
                85                  90                  95

Glu Lys Thr Thr Ala Ala Leu Ser Ile Leu Pro Gly Ile Gly Ser Val
            100                 105                 110

Met Gly Ile Ala Asp Gly Ala Val His His Asn Thr Glu Glu Ile Val
        115                 120                 125

Ala Gln Ser Ile Ala Leu Ser Ser Leu Met Val Ala Gln Ala Ile Pro
    130                 135                 140

Leu Val Gly Glu Leu Val Asp Ile Gly Phe Ala Ala Tyr Asn Phe Val
145                 150                 155                 160

Glu Ser Ile Ile Asn Leu Phe Gln Val Val His Asn Ser Tyr Asn Arg
                165                 170                 175

Pro Phe Thr Val Thr Val Pro Lys Asp Leu Tyr Val Val Glu Tyr Gly
            180                 185                 190

Ser Asn Met Thr Ile Glu Cys Lys Phe Pro Val Glu Lys Gln Leu Asp
        195                 200                 205

Leu Ala Ala Leu Ile Val Tyr Trp Glu Met Glu Asp Lys Asn Ile Ile
    210                 215                 220

Gln Phe Val His Gly Glu Glu Asp Leu Lys Val Gln His Ser Ser Tyr
225                 230                 235                 240

Arg Gln Arg Ala Arg Leu Leu Lys Asp Gln Leu Ser Leu Gly Asn Ala
                245                 250                 255

Ala Leu Gln Ile Thr Asp Val Lys Leu Gln Asp Ala Gly Val Tyr Arg
            260                 265                 270

Cys Met Ile Ser Tyr Gly Gly Ala Asp Tyr Lys Arg Ile Thr Val Lys
        275                 280                 285

Val Asn Ala Pro Tyr Asn Lys Ile Asn Gln Arg Ile Leu Val Val Asp
    290                 295                 300

Pro Val Thr Ser Glu His Glu Leu Thr Cys Gln Ala Glu Gly Tyr Pro
305                 310                 315                 320

Lys Ala Glu Val Ile Trp Thr Ser Ser Asp His Gln Val Leu Ser Gly
                325                 330                 335

Lys Thr Thr Thr Thr Asn Ser Lys Arg Glu Glu Lys Leu Phe Asn Val
            340                 345                 350

Thr Ser Thr Leu Arg Ile Asn Thr Thr Thr Asn Glu Ile Phe Tyr Cys
        355                 360                 365

Thr Phe Arg Arg Leu Asp Pro Glu Glu Asn His Thr Ala Glu Leu Val
    370                 375                 380

Ile Pro Glu Leu
385

<210>  127
<211>  275
<212>  PRT
<213>  Artificial sequence

<220>
<223>  Polypeptide

<400>  127

Ile Asn Leu Asp Trp Asp Val Ile Arg Asp Lys Thr Lys Thr Lys Ile
1               5                   10                  15

Glu Ser Leu Lys Glu His Gly Pro Ile Lys Asn Lys Met Ser Glu Ser
            20                  25                  30

Pro Asn Lys Thr Val Ser Glu Glu Lys Ala Lys Gln Tyr Leu Glu Glu
        35                  40                  45

Phe His Gln Thr Ala Leu Glu His Pro Glu Leu Ser Glu Leu Lys Thr
    50                  55                  60

Val Thr Gly Thr Asn Pro Val Phe Ala Gly Ala Asn Tyr Ala Ala Trp
65                  70                  75                  80

Ala Val Asn Val Ala Gln Val Ile Asp Ser Glu Thr Ala Asp Asn Leu
                85                  90                  95

Glu Lys Thr Thr Ala Ala Leu Ser Ile Leu Pro Gly Ile Gly Ser Val
            100                 105                 110

Met Gly Ile Ala Asp Gly Ala Val His His Asn Thr Glu Glu Ile Val
        115                 120                 125

Ala Gln Ser Ile Ala Leu Ser Ser Leu Met Val Ala Gln Ala Ile Pro
    130                 135                 140

Leu Val Gly Glu Leu Val Asp Ile Gly Phe Ala Ala Tyr Asn Phe Val
145                 150                 155                 160

Glu Ser Ile Ile Asn Leu Phe Gln Val Val His Asn Ser Tyr Asn Arg
                165                 170                 175

Pro Ala Pro Tyr Asn Lys Ile Asn Gln Arg Ile Leu Val Val Asp Pro
            180                 185                 190

Val Thr Ser Glu His Glu Leu Thr Cys Gln Ala Glu Gly Tyr Pro Lys
        195                 200                 205

Ala Glu Val Ile Trp Thr Ser Ser Asp His Gln Val Leu Ser Gly Lys
    210                 215                 220

Thr Thr Thr Thr Asn Ser Lys Arg Glu Glu Lys Leu Phe Asn Val Thr
225                 230                 235                 240

Ser Thr Leu Arg Ile Asn Thr Thr Thr Asn Glu Ile Phe Tyr Cys Thr
                245                 250                 255

Phe Arg Arg Leu Asp Pro Glu Glu Asn His Thr Ala Glu Leu Val Ile
            260                 265                 270

Pro Glu Leu
        275

<210>  128
<211>  13
<212>  PRT
<213>  Homo sapiens

<400>  128

Glu Ala Phe Ser Pro Val Ser Tyr Gln His Asp Leu Ala
1               5                   10


<210>  129
<211>  252
<212>  PRT
<213>  Homo sapiens

<400>  129

Arg Lys Ser Leu Ser Ser Met Thr Arg Val Val Gly Gly Leu Val Ala
1               5                   10                  15

Leu Arg Gly Ala His Pro Tyr Ile Ala Ala Leu Tyr Trp Gly His Ser
            20                  25                  30

Phe Cys Ala Gly Ser Leu Ile Ala Pro Cys Trp Val Leu Thr Ala Ala
        35                  40                  45

His Cys Leu Gln Asp Arg Pro Ala Pro Glu Asp Leu Thr Val Val Leu
    50                  55                  60

Gly Gln Glu Arg Arg Asn His Ser Cys Glu Pro Cys Gln Thr Leu Ala
65                  70                  75                  80

Val Arg Ser Tyr Arg Leu His Glu Ala Phe Ser Pro Val Ser Tyr Gln
                85                  90                  95

His Asp Leu Ala Leu Leu Arg Leu Gln Glu Asp Ala Asp Gly Ser Cys
            100                 105                 110

Ala Leu Leu Ser Pro Tyr Val Gln Pro Val Cys Leu Pro Ser Gly Ala
        115                 120                 125

Ala Arg Pro Ser Glu Thr Thr Leu Cys Gln Val Ala Gly Trp Gly His
    130                 135                 140

Gln Phe Glu Gly Ala Glu Glu Tyr Ala Ser Phe Leu Gln Glu Ala Gln
145                 150                 155                 160

Val Pro Phe Leu Ser Leu Glu Arg Cys Ser Ala Pro Asp Val His Gly
                165                 170                 175

Ser Ser Ile Leu Pro Gly Met Leu Cys Ala Gly Phe Leu Glu Gly Gly
                180                 185                 190

Thr Asp Ala Cys Gln Gly Asp Ser Gly Gly Pro Leu Val Cys Glu Asp
            195                 200                 205

Gln Ala Ala Glu Arg Arg Leu Thr Leu Gln Gly Ile Ile Ser Trp Gly
    210                 215                 220

Ser Gly Cys Gly Asp Arg Asn Lys Pro Gly Val Tyr Thr Asp Val Ala
225                 230                 235                 240

Tyr Tyr Leu Ala Trp Ile Arg Glu His Thr Val Ser
                245                 250

<210>  130
<211>  252
<212>  PRT
<213>  Mus musculus

<400>  130

Arg Lys Gly Leu Ser Ser Phe Met Arg Val Val Gly Gly Leu Val Ala
1               5                   10                  15

Leu Pro Gly Ser His Pro Tyr Ile Ala Ala Leu Tyr Trp Gly Asn Asn
            20                  25                  30

Phe Cys Ala Gly Ser Leu Ile Ala Pro Cys Trp Val Leu Thr Ala Ala
        35                  40                  45

His Cys Leu Gln Asn Arg Pro Ala Pro Glu Glu Leu Thr Val Val Leu
    50                  55                  60

Gly Gln Asp Arg His Asn Gln Ser Cys Glu Trp Cys Gln Thr Leu Ala
65                  70                  75                  80

Val Arg Ser Tyr Arg Leu His Glu Gly Phe Ser Ser Ile Thr Tyr Gln
                85                  90                  95

His Asp Leu Ala Leu Leu Arg Leu Gln Glu Ser Lys Thr Asn Ser Cys
            100                 105                 110

Ala Ile Leu Ser Pro His Val Gln Pro Val Cys Leu Pro Ser Gly Ala
        115                 120                 125

Ala Pro Pro Ser Glu Thr Val Leu Cys Glu Val Ala Gly Trp Gly His
    130                 135                 140

Gln Leu Glu Gly Ala Glu Glu Tyr Ser Thr Phe Leu Gln Glu Ala Gln
145                 150                 155                 160

Val Pro Phe Ile Ala Leu Asp Arg Cys Ser Asn Ser Asn Val His Gly
                165                 170                 175

Asp Ala Ile Leu Pro Gly Met Leu Cys Ala Gly Phe Leu Glu Gly Gly
            180                 185                 190

Thr Asp Ala Cys Gln Gly Asp Ser Gly Gly Pro Leu Val Cys Glu Glu
        195                 200                 205

Gly Thr Ala Glu His Gln Leu Thr Leu Arg Gly Val Ile Ser Trp Gly
    210                 215                 220

Ser Gly Cys Gly Asp Arg Asn Lys Pro Gly Val Tyr Thr Asp Val Ala
225                 230                 235                 240

Asn Tyr Leu Ala Trp Ile Gln Lys His Ile Ala Ser
                245                 250


<210>  131
<211>  13
<212>  PRT
<213>  Mus musculus

<400>  131

Glu Gly Phe Ser Ser Ile Thr Tyr Gln His Asp Leu Ala
1               5                   10